# EUROPEAN PATENT SPECIFICATION

(11) **EP 3 916 108 B1**
(45) Date of publication and mention of the grant of the patent: **29.03.2023**
(21) Application number: 21177621.6
(22) Date of filing: 17.11.2017
(51) Int. Cl.: C12Q 1/6834, C12Q 1/6841, C12Q 1/6874

(54) **METHOD FOR SPATIAL TAGGING AND ANALYSING NUCLEIC ACIDS IN A BIOLOGICAL SPECIMEN**
VERFAHREN ZUR RÄUMLICHEN MARKIERUNG UND ANALYSE VON NUKLEINSÄUREN IN EINER BIOLOGISCHEN PROBE
PROCÉDÉ DE MARQUAGE SPATIAL ET D'ANALYSE D'ACIDES NUCLÉIQUES DANS UN ÉCHANTILLON BIOLOGIQUE

(30) Priority: 17.11.2016 GB 201619458
(43) Date of publication of application: 01.12.2021
(62) Divisional of application: 17809218.5
(73) Proprietor: Spatial Transcriptomics AB, 113 63 Stockholm (SE)
(72) Inventor: FRISÉN, Jonas, 11140 Stockholm (SE); STÅHL, Patrik, SE-118 26 Stockholm (SE); LUNDEBERG, Joakim, 181 34 Stockholm (SE); SALMÉN, Fredrik, 3521 XV Utrecht (NL); VICKOVIC, Sanja, 118 61 Stockholm (SE)
(74) Representative: D Young & Co LLP

(56) References cited:
- WO-A1-2012/140224
- WO-A1-2016/162309
- WO-A1-2016/168825
- WO-A2-2017/019456

## Description

The present description relates generally to the spatial tagging of nucleic acid molecules in a biological specimen, e.g. the localised or spatial detection of nucleic acids in a biological specimen, such as a tissue sample (so-called "spatial genomics" and "spatial transcriptomics"). In particular, the present description relates to an improvement in said methods to increase the number and diversity of unique nucleic acids captured and tagged from each biological specimen.

Spatial genomic and transcriptomic methods utilise nucleic acid probes immobilized to a solid substrate (e.g. an array) to capture nucleic acids (DNA or RNA) from a biological specimen and subsequently tag said nucleic acids (e.g. with a nucleic acid "barcode" sequence) based on their location within the biological specimen (see e.g. WO 2012/140224). The "tagged" nucleic acids are subsequently released from the solid substrate and analysed to generate information about the localisation, distribution and/or expression of genes, or indeed about the localisation or distribution of any genomic variation (not necessarily in a gene) in a biological specimen, such as a tissue sample.

By way of example, spatial transcriptomics utilises reverse transcription (RT) primers comprising unique positional tags (domains, such as barcode sequences), which are immobilized on a solid substrate, e.g. a glass slide, to generate an "array". The unique positional tags correspond to the location of the RT primers on the array (the features of the array). Biological specimens, such as tissue sections, are placed onto the array and a reverse transcription reaction is performed in the tissue section on the array. The RT primers, to which the RNA in the tissue sample binds (or hybridizes), are extended using the bound RNA as a template to obtain cDNA, which is therefore bound to the surface of the array. As consequence of the unique positional tags in the RT primers, each cDNA strand carries information about the position of the template RNA in the tissue section. The cDNA is then released from the surface of the array and analysed, e.g. sequenced, which results in a transcriptome with exact positional information. The sequence data can then be matched to a position in the tissue sample. For instance, the tissue section may be visualised or imaged, e.g. stained and photographed, before or after the cDNA synthesis step to facilitate the correlation of the positional tag in the cDNA molecule with a position within the tissue sample and the sequence data may be overlaid on an image of the tissue specimen, e.g. using a computer, to display the expression pattern of any gene of interest across the tissue. However, the visualisation step is not essential as the tagged cDNA molecule may be correlated with a position in the tissue sample using other means, e.g. the unique profile of the molecules captured with the same positional tag, i.e. at the same location (feature) on the array, may enable the molecule to be correlated to a position within the tissue sample based on the known expression characteristics of cells or areas of cells within the tissue sample.

The sensitivity of spatial transcriptomics and genomics is limited by various factors including: (i) the efficiency of the hybridisation of the nucleic acid molecules to the capture probes; (ii) the efficiency of the subsequent extension reaction; (iii) the density of the capture probes on the array, i.e. the resolution of the array; and (iv) the non-specific extension of nucleic acid molecules that are not bound to capture probes, e.g. extension reactions primed by nucleic acids within the biological specimen, such as fragmented DNA and RNA caused by the preparation of the biological specimen, e.g. fixation of a tissue section. These limitations mean that some transcripts may be under-represented in the subsequent analysis. For instance, some transcripts may not be captured on low density arrays because the tissue in which they are expressed is does not make contact with a feature of the array. The limitations above can be particularly problematic for low abundance transcripts.

Methods for improving the density of capture probes on the array have been proposed in WO 2016/162309, which utilises flow cell and bead array technology to generate "random" arrays with a high density of features, i.e. arrays on which the location of the capture probes is not pre-determined.

WO 2016/1668825 describes methods for performing spatial profiling of biological molecules. WO 2012/140224 describes a method and product for localised or spatial detection of nucleic acid in a tissue sample.

However, there is room to improve the sensitivity of spatial transcriptomic and genomic methods in order to overcome the limitations of the methods known in the art. In this respect, the inventors have surprisingly found that it is possible to increase the number and diversity of unique nucleic acid molecules captured by the capture probes by combining the step of releasing the probes from surface of the solid substrate, e.g. array, with the step of extending the probes using the captured nucleic acids as templates for extension, e.g. the release and extension steps are performed simultaneously.

In particular, the inventors have found that it is possible to combine the enzyme used to release the capture probes from the surface of the solid substrate (i.e. the cleavage enzyme) with the reaction mixture used in the nucleic acid extension step without a substantial loss of cleavage enzyme activity. Furthermore, and contrary to expectations, the combined (e.g. simultaneous) release and extension of the capture probes does not eliminate the localised capture of nucleic acid molecules, i.e. the spatial tagging of the nucleic acids in the biological specimen is retained in the methods of the invention. Moreover, as discussed in detail in the Examples below, the inventors have advantageously determined that the simultaneous release and extension of the capture probes increases the number and diversity of unique nucleic acid molecules captured by the methods of the invention, thereby improving the sensitivity of the spatial transcriptomic and genomic methods.

Whilst not wishing to be bound by theory it is hypothesised that the combination of the release and nucleic acid synthesis steps may result in the localised release of some or all of the capture probes prior to their extension, i.e. the probes may be released into the local vicinity of their original position on the array. It is thought that this may enhance the sensitivity of the methods by enabling capture probes (that have not hybridised to a nucleic acid from the biological specimen on contact between the specimen and the array) to diffuse into the specimen, thereby increasing the probability that the capture probe hybridises to a target sequence. Additionally or alternatively, the simultaneous release and extension of the capture probes may improve extension reaction, e.g. DNA synthesis reaction, because the capture probes are no longer physically constrained, i.e. immobilized on solid substrate, and/or because the microreactor environment formed between the biological specimen and the surface of the array is particularly suited for a templated nucleic acid extension reaction. Thus, the extension reaction may proceed more efficiently once capture probes, that hybridised to target sequences when they were still immobilised on the solid substrate, are released from the surface of the array.

The present invention provides a method for spatially tagging nucleic acids of a biological specimen comprising:
(a) providing a solid substrate on which multiple species of capture probes are immobilized such that each species occupies a distinct position on the solid substrate, wherein each capture probe of the multiple species is a primer for an extension reaction and wherein each capture probe of the multiple species is immobilized at its 3' end and comprises a nucleic acid molecule comprising in a 5' to 3' direction:
   (i) a positional domain that corresponds to a position of the capture probe on the solid substrate;
   (ii) a capture domain; and
   (iii) a cleavage domain for releasing the capture probe from the solid substrate;
(b) contacting the solid substrate with the biological specimen; and
(c) simultaneously releasing the multiple species of capture probes from the solid substrate under conditions that allow a nucleic acid of the biological specimen to hybridise to a capture domain of the capture probe and extending the capture probe using the nucleic acid hybridised to the capture probe as an extension template to produce an extended capture probe, wherein step (c) comprises contacting the solid substrate with an aqueous reaction mixture comprising:
   (i) a polymerase capable of extending the capture probe using the nucleic acid hybridised to the capture probe as an extension template to produce the extended capture probe; and
   (ii) a means for releasing the multiple species of capture probes from the solid substrate.

Accordingly, in one embodiment the present description provides a method for spatially tagging nucleic acids of a biological specimen comprising:
(a) providing a solid substrate on which multiple species of capture probes are immobilized such that each species occupies a distinct position on the solid substrate, wherein said probes are for a primer extension reaction and wherein each species of said capture probe comprises a nucleic acid molecule comprising:
   (i) a cleavage domain for releasing the capture probe from the surface of the solid substrate,
   (ii) a positional domain that corresponds to the position of the capture probe on the solid substrate, and
   (iii) a capture domain;
(b) contacting said solid substrate with a biological specimen; and
(c) releasing said capture probes from the surface of the solid substrate under conditions that allow nucleic acids of the biological specimen to hybridise to the capture domain in said capture probes and simultaneously and/or subsequently extending said capture probes using the nucleic acid molecules hybridised to the capture probes as extension templates to produce extended probes thereby spatially tagging the nucleic acids of the biological specimen.

The methods of the invention represent a significant improvement over other methods for spatial transcriptomics and genomics known in the art, e.g. WO 2012/140224, WO 2014/060483 and WO 2016/162309. The methods described herein result in an increase in the number and diversity of unique transcripts captured from the biological specimen, thereby providing a spatially tagged transcriptome that is more representative of the expression profile of the biological specimen. Moreover, as the capture probes may be released from the surface of the array prior to their extension the probes do not need to be attached to the array via their 5' ends in the methods of the present invention. In this respect, the capture probes may be attached to the array via their 3' ends and the release of the capture probes from the surface of the array will expose the 3' ends of the probes, enabling the probes to function as primers for nucleic acid extension reactions.

As discussed in more detail below, the method of the invention may comprise an additional step of analysing the extended probes. In this respect, it is evident that the combination of spatial tagging of the nucleic acids from a biological specimen and subsequent analysis of said tagged nucleic acids facilitates the localised detection of a nucleic acid in a biological specimen, e.g. tissue sample. Thus, in one embodiment, the method of the invention may be used for determining and/or analysing all of the transcriptome or genome of a biological specimen, e.g. the global transcriptome or genome of a biological specimen. However, the method is not limited to this and encompasses determining and/or analysing all or part of the transcriptome or genome. Thus, the method may involve determining and/or analysing a part or subset of the transcriptome or genome, e.g. a transcriptome corresponding to a subset of genes, e.g. a set of particular genes, for example related to a particular disease or condition, tissue type etc.

Viewed from another aspect, the method steps set out above can be seen as providing a method of obtaining a spatially defined transcriptome or genome, and in particular the spatially defined global transcriptome or genome, of a biological specimen, e.g. tissue sample.

Alternatively viewed, the method of the invention may be seen as a method for localised or spatial detection of nucleic acid, whether DNA or RNA, in a biological specimen, e.g. tissue sample, or for localised or spatial determination and/or analysis of nucleic acid (DNA or RNA) in a tissue sample. In particular, the method may be used for the localised or spatial detection or determination and/or analysis of gene expression or genomic variation in a tissue sample. The localised/spatial detection/determination/analysis means that the RNA or DNA may be localised to its native position or location within a cell or tissue in the tissue sample. Thus for example, the RNA or DNA may be localised to a cell or group of cells, or type of cells in the sample, or to particular regions of areas within a tissue sample. The native location or position of the RNA or DNA (or in other words, the location or position of the RNA or DNA in the tissue sample), e.g. an expressed gene or genomic locus, may be determined.

Thus, in some embodiments, the description provides a method for localised detection of nucleic acid in a biological specimen comprising:
(a) providing a solid substrate on which multiple species of capture probes are immobilized such that each species occupies a distinct position on the solid substrate, wherein said probes are for a primer extension reaction and wherein each species of said capture probe comprises a nucleic acid molecule comprising:
   (i) a cleavage domain for releasing the capture probe from the surface of the solid substrate,
   (ii) a positional domain that corresponds to the position of the capture probe on the solid substrate, and
   (iii) a capture domain;
(b) contacting said solid substrate with a biological specimen;
(c) releasing said capture probes from the surface of the solid substrate under conditions that allow nucleic acids of the biological specimen to hybridise to the capture domain in said capture probes and simultaneously and/or subsequently extending said capture probes using the nucleic acid molecules hybridised to the capture probes as extension templates to produce extended probes thereby spatially tagging the nucleic acids of the biological specimen; and
(d) analysing the extended probes of (c), i.e. analysing the spatially tagged nucleic acids of the biological specimen.

As described in more detail below, any method of nucleic acid analysis may be used in the analysis step (step (d)). Typically this may involve sequencing, i.e. analysing the sequence of the extended probes, but it is not necessary to perform an actual sequence determination. For example sequence-specific methods of analysis may be used. For example a sequence-specific amplification reaction may be performed, for example using primers which are specific for the positional domain and/or for a specific target sequence, e.g. a particular target DNA to be detected (i.e. corresponding to a particular cDNA/RNA or gene etc.). An exemplary analysis method is a sequence-specific PCR reaction.

The sequence analysis information obtained in step (d) may be used to obtain spatial information as to the RNA or DNA in the biological specimen, e.g. tissue sample. In other words the sequence analysis information may provide information as to the location of the RNA or DNA in the biological specimen, e.g. tissue sample. This spatial information may be derived from the nature of the sequence analysis information determined, for example it may reveal the presence of a particular RNA or DNA which may itself be spatially informative in the context of the biological specimen, e.g. tissue sample, used, and/or the spatial information (e.g. spatial localisation) may be derived from the position of the biological specimen, e.g. tissue sample, on the solid substrate, e.g. array, coupled with the sequencing information. Thus, the method may involve simply correlating the sequence analysis information to a position in the biological specimen, e.g. tissue sample, e.g. by virtue of the positional tag and its correlation to a position in the biological specimen, e.g. tissue sample. However, in some embodiments, spatial information may conveniently be obtained by correlating the sequence analysis data to an image of the biological specimen, e.g. tissue sample. Accordingly, in a preferred embodiment the method also includes a step of:
(e) correlating said sequence analysis information with an image of said biological specimen, wherein the biological specimen is imaged before or after step (c), i.e. after step (b).

It will be seen therefore that the array of the present description may be used to capture RNA, e.g. mRNA, or DNA of a biological specimen, e.g. tissue sample, that is contacted with said solid substrate, e.g. array. The methods of the invention may thus be considered as methods of quantifying the spatial expression of one or more genes in a tissue sample. Expressed another way, the methods of the present invention may be used to detect the spatial expression of one or more genes in a biological specimen, e.g. tissue sample. In yet another way, the methods of the present invention may be used to determine simultaneously the expression of one or more genes at one or more positions within a biological specimen, e.g. tissue sample. Still further, the methods may be seen as methods for partial or global transcriptome or genome analysis of a biological specimen, e.g. tissue sample, with two-dimensional spatial resolution.

Step (c) in the method above of releasing said capture probes from the surface of the solid substrate and simultaneously and/or subsequently extending said capture probes will be seen as relating to combining the release and extension reactions, i.e. combining the means for achieving capture probe release and target nucleic acid templated extension of said capture probes. As discussed above, the improvement in the sensitivity of the method may be a consequence of the release of capture probes that have not bound to a target sequence in step (b), which may facilitate the binding of other target nucleic acid molecules in the biological specimen. Alternatively or additionally, the improvement in the sensitivity of the method may result from the reduction of steric hindrance on the capture probe-target nucleic acid complex or the formation of microenvironment that is particularly conducive to nucleic acid extension reactions, thereby enhancing the efficiency of the extension reaction.

Accordingly, the simultaneous release and extension of the capture probes does not mean that all capture probes will be released and extended at the same time, but rather that the means for releasing and extending the capture probes are applied to the solid substrate, e.g. array, simultaneously, i.e. at substantially the same time.

Notably, as mentioned above, the capture probes of the presented description are not restricted to a particular orientation on the array, unlike the methods in WO 2012/140224, WO 2014/060483 and WO 2016/162309, which require the capture probes to be immobilized on the solid substrate such that they have a free 3' end capable of functioning as an extension primer. In this respect, the combination of the release and extension steps eliminates the requirement for a particular orientation of the capture probes on the solid substrate. However, in some embodiments, it is preferred that the capture probes are immobilized on the solid substrate such that they have a free 3' end capable of functioning as an extension primer.

Furthermore, as the capture probes may be oriented on the solid substrate such that the capture domain is not free or available to interact with (i.e. bind or hybridise to) the nucleic acid molecules in the biological specimen (i.e. the capture probes may be immobilized via their 3' ends), it is not necessary to contact the biological specimen with the solid substrate under conditions that allow (i.e. are suitable for or facilitate) the nucleic acids of the biological specimen to hybridise to the capture domain in said capture probes. However, in some embodiments (e.g. where the capture probes are immobilized on the solid substrate such that they have a free 3' end capable of functioning as an extension primer, e.g. via their 5' ends) step (b) may be performed under conditions that allow the nucleic acids of the biological specimen to hybridise to the capture domain in said capture probes.

As described herein, where the capture probes are immobilized on the solid substrate such that they have a free 3' end capable of functioning as an extension primer (e.g. by their 5' end), some capture probes may be released from the solid substrate prior to their extension, i.e. some capture probes are released and subsequently extended. In methods of the invention, some capture probes may be extended at the same time as they are released from the solid substrate, i.e. some capture probes are extended and released from the solid substrate simultaneously. Accordingly, in embodiments where the capture probes are immobilized on the solid substrate such that they have a free 3' end capable of functioning as an extension primer (e.g. by their 5' end), step (c) may comprise simultaneous release and extension reactions and sequential release and extension reactions, i.e. release and subsequent extension of the capture probes. The simultaneous reactions may occur particularly when step (b) is performed under conditions that allow the nucleic acids of the biological specimen to hybridise to the capture domain in said capture probes, i.e. such that some nucleic acids may hybridise to the capture domains of the capture probes prior to the combined release and extension reactions, i.e. prior to step (c).

As described herein, where the capture probes are immobilized on the solid substrate such that they do not have a free 3' end capable of functioning as an extension primer (e.g. by their 3' end), all capture probes may be released from the solid substrate prior to their extension, i.e. all of capture probes are released and subsequently extended. Accordingly, where the capture probes are immobilized on the solid substrate such that they do not have a free 3' end capable of functioning as an extension primer (e.g. by their 3' end), step (c) may comprise sequential release and extension reactions, i.e. release and subsequent extension of the capture probes, despite the presence of both components required for the release and extension reactions.

Thus, in some embodiments step (c) may be seen to comprise contacting said solid substrate, e.g. array, with:
(i) a polymerase enzyme capable of extending said capture probes using the nucleic acid molecules hybridised to the capture probes as extension templates; and
(ii) means for releasing said capture probes from the surface of the solid substrate,
at the same time.

The phrase "at the same time" means substantially the same time, i.e. one component may be contacted with the solid substrate before the other component, e.g. within seconds, (e.g. within 15, 30, 45, 60, 90, 120 or 180 seconds) or minutes (e.g. within 1, 2, 3, 4, 5, 6, 7, 8, 9, 10, 12 or 15 minutes), but such that the reactions are allowed to proceed together. If one component is contacted with the solid substrate before the other component it is preferred that the means for releasing the capture probes from the surface of the solid substrate is contacted first and the polymerase enzyme is contacted within seconds or minutes as defined above. However, in some embodiments, it may be desirable to contact the polymerase enzyme first and contact the means for releasing the capture probes from the surface of the solid substrate within seconds or minutes as defined above.

In view of the fact that step (c) may comprise sequential release and extension reactions, i.e. release and subsequent extension of the capture probes, it will be evident that the sequential release and extension reactions may be achieved by contacting solid substrate with the means for releasing said capture probes from the surface of the solid substrate and the polymerase enzyme separately.

Thus, in some embodiments step (c) may be seen to comprise contacting said solid substrate, e.g. array, with means for releasing said capture probes from the surface of the solid substrate and subsequently contacting said solid substrate, e.g. array, with a polymerase enzyme capable of extending said capture probes using the nucleic acid molecules hybridised to the capture probes as extension templates.

The term "subsequently" means that the polymerase enzyme is contacted with the solid substrate after the means for releasing said capture probes from the surface of the solid substrate is contacted with the solid substrate, such that the extension reaction does not occur immediately after the capture probes have hybridised to their target molecules. There is no particular limit on the amount of time that may be allowed to lapse between contacting the solid substrate with the means for releasing the capture probes and the polymerase enzyme. However, it is preferred that the polymerase enzyme is contacted with the solid substrate before the target nucleic acid molecules (e.g. RNA) have substantially degraded. Thus, in some embodiments, subsequently contacting the polymerase enzyme means that the polymerase enzyme is contacted with the solid substrate minutes or hours after the step of contacting the means for releasing said capture probes with the solid substrate. For instance, the polymerase enzyme may be contacted with the solid substrate at least 1, 2, 3, 4, 5, 6, 7, 8, 9, 10, 15, 20, 25, 30, 40, 50 or 60 minutes after the means for releasing said capture probes from the surface of the solid substrate, e.g. within 120, 90 or 60 minutes, i.e. between 1-120, 5-90, 10-60 minutes after the means for releasing said capture probes from the surface of the solid substrate. In some embodiments, the polymerase enzyme may be contacted with the solid substrate at least 1, 2, 3, 4, 5, 6, 7, 8, 9, 10, 12, 18, 24, 36 or 48 hours after the means for releasing said capture probes from the surface of the solid substrate, e.g. within 72, 48 or 24 hours, i.e. between 1-72, 6-48, 12-24 hours after the means for releasing said capture probes from the surface of the solid substrate.

In a particularly preferred embodiment, the polymerase enzyme and means for releasing said capture probes from the surface of the solid substrate, e.g. cleavage enzyme, are combined in a single reaction mixture, which is contacted with the array. Thus, in some embodiments, step (c) comprises contacting said array with a reaction mixture (i.e. an aqueous reaction mixture) comprising:
(i) a polymerase enzyme capable of extending said capture probes using the nucleic acid molecules hybridised to the capture probes as extension templates; and
(ii) means for releasing said capture probes from the surface of the solid substrate.

Accordingly, the invention can be seen to provide the use of a reaction mixture (i.e. an aqueous reaction mixture) comprising:
(i) a polymerase enzyme capable of extending capture probes (as defined above) using the nucleic acid molecules hybridised to the capture probes as extension templates; and
(ii) means for releasing said capture probes from the surface of a solid substrate,
in a method for spatially tagging nucleic acids of a biological specimen, such as the methods defined herein.

The step of releasing the capture probes from the surface of the solid substrate may be achieved in a number of ways. In this respect, as mentioned above, the capture probes comprise a cleavage domain to facilitate the release of the capture probes. Accordingly, "means for releasing the capture probes from the surface of the solid substrate" includes any components that are suitable for this purpose, e.g. chemical or enzymatic means. As described below, the preferred method for releasing the capture probes from the surface of the solid substrate comprises enzymatic cleavage of the capture probe in the cleavage domain. In this respect, releasing the probe via the cleavage domain ensures that the released capture probes (including any extended or partially extended capture probes) comprise the positional domain, which contains information relating to the location of the capture probes on the array.

The term "cleavage domain" refers to a domain within the capture probe that can be cleaved specifically to release the capture probe (or part of the capture probe comprising the positional domain and the sequence 3' to the positional domain (if present), i.e. the capture domain and extension product (if present)) from the surface of the solid substrate.

As discussed below, the capture probes may be attached to the solid substrate by any suitable means, such as by chemical immobilization, e.g. by a chemical cross-linker. Thus, in some embodiments, the cleavage domain of the capture probe refers to a cleavable chemical cross-linker attaching the capture probe to the surface of the solid substrate. Thus, in some embodiments, the capture domain does not form part of the nucleotide sequence of the capture probe. For instance, the capture probe may comprise a nucleic acid positional domain and capture domain and a non-nucleic acid cleavage domain (e.g. a cleavable chemical cross-linker) conjugated to the 5' or 3' end of the capture probe (depending on the orientation of the probe attached to the solid substrate). Thus, cleavage of said domain results in the release of the whole of the nucleic acid capture probe, i.e. comprising the positional domain and the sequence 3' to the positional domain from the surface of the solid substrate. Cleavable chemical cross-linkers capable of immobilizing nucleic acid molecules to a solid substrate, as defined herein, are known in the art.

In preferred embodiments, the cleavage domain of the capture probe refers to a nucleotide sequence within the capture probe that can be cleaved specifically, either chemically or preferably enzymatically. The location of the cleavage domain within the capture probe will depend on whether or not the capture probes are immobilized on the solid substrate such that they have a free 3' end capable of functioning as an extension primer (e.g. by their 5' or 3' end). For instance, if the capture probes are immobilized by their 5' end, the cleavage domain will be located 5' to the positional domain and cleavage of said domain results in the release of part of the capture probe comprising the positional domain and the sequence 3' to the positional domain, and optionally part of the cleavage domain, from the surface of the solid substrate. Alternatively, if the capture probes are immobilized by their 3' end, the cleavage domain will be located 3' to the capture domain (and positional domain) and cleavage of said domain results in the release of part of the capture probe comprising the positional domain and the sequence 3' to the positional domain from the surface of the solid substrate. Preferably, cleavage results in complete removal of the cleavage domain, particularly when the capture probes are immobilized via their 3' end as the presence of a part of the cleavage domain may interfere with the hybridisation of the capture domain and the target nucleic acid and/or its subsequent extension.

"Cleavage" is defined broadly herein to include any means of breaking a covalent bond. In some embodiments, cleavage involves cleavage of a covalent bond in a nucleotide chain (i.e. strand cleavage or strand scission), for example by cleavage of a phosphodiester bond.

Thus, the cleavage domain may comprise a sequence that is recognised by one or more enzymes capable of cleaving a nucleic acid molecule, i.e. capable of breaking the phosphodiester linkage between two or more nucleotides. For instance, the cleavage domain may comprise a restriction endonuclease (restriction enzyme) recognition sequence. Restriction enzymes cut double-stranded or single stranded DNA at specific recognition nucleotide sequences known as restriction sites and suitable enzymes are well known in the art. For example, it is particularly advantageous to use rare-cutting restriction enzymes, i.e. enzymes with a long recognition site (at least 8 base pairs in length), to reduce the possibility of cleaving elsewhere in the nucleic acid, e.g. cDNA molecule. As mentioned above, it will be seen that releasing at least part of the capture probes from the surface of the solid substrate requires releasing a part comprising the positional domain and capture domain of the capture probe and, in some embodiments, any sequence produced by the templated extension of the capture domain. Thus, if the capture probe is extended partially or fully whilst still attached to (i.e. immobilized on) the solid substrate, cleavage of the cleavage domain will release the extended capture probe, comprising the positional domain, capture domain and extension product. Hence, in some preferred embodiments, i.e. when the capture probes are immobilized via their 5' end, cleavage of the capture probe should take place 5' to the positional domain.

By way of example, the cleavage domain may comprise a poly-U sequence which may be cleaved by a mixture of Uracil DNA glycosylase (UDG) and the DNA glycosylase-lyase Endonuclease VIII, commercially known as the USER^{™} enzyme.

A further example of a cleavage domain can be utilised in embodiments where the capture probe is immobilized to the solid substrate indirectly, i.e. via a surface probe defined below. The cleavage domain may comprise one or more mismatch nucleotides, i.e. when the complementary parts of the surface probe and the capture probe are not 100% complementary. Such a mismatch is recognised, e.g. by the MutY and T7 endonuclease I enzymes, which results in cleavage of the nucleic acid molecule at the position of the mismatch.

Another example of a cleavage domain that can be utilised in embodiments where the capture probe is immobilized to the solid substrate indirectly, i.e. via a surface probe, comprises a nickase recognition site or sequence. In this respect, nickase enzymes cleave only one strand in a nucleic acid duplex. Nickases are endonucleases which cleave only a single strand of a DNA duplex. Thus, the cleavage domain may comprise a nickase recognition site close to the 5' end of the surface probe (and/or the 5' end of the capture probe) such that cleavage of the surface probe or capture probe destabilises the duplex between the surface probe and capture probe thereby releasing the capture probe from the solid substrate.

Nickase enzymes could also be used in embodiments where the capture probe is immobilized to the solid substrate directly. For instance, the solid substrate may be contacted with a nucleic acid molecule that hybridises to the cleavage domain of the capture probe to provide or reconstitute a nickase recognition site, i.e. a cleavage helper probe. Thus, contact with a nickase enzyme will result in cleavage of the cleavage domain thereby releasing the capture probe from the solid substrate. It will be evident that such cleavage helper probes could be used to provide or reconstitute cleavage recognition sites for other cleavage enzymes, e.g. restriction enzymes, as discussed above.

Thus, in some embodiments, the method may also comprise a step of contacting the solid substrate with a cleavage helper probe, i.e. under conditions that allow the cleavage helper probe to hybridise to the cleavage domain of the capture probe to provide a cleavage recognition site, e.g. a nickase enzyme recognition site or a restriction enzyme recognition site.

Some nickases introduce single-stranded nicks only at particular sites on a DNA molecule, by binding to and recognizing a particular nucleotide recognition sequence. A number of naturally-occurring nickases have been discovered, of which at present the sequence recognition properties have been determined for at least four. Nickases are described in U.S. Patent No. 6,867,028 and any suitable nickase may be used in the methods of the invention.

In embodiments that utilise a nickase enzyme, the nickase enzyme may be removed from the assay or inactivated prior to step (d) to prevent unwanted cleavage of the extended probes or amplicons thereof.

Thus, in a preferred embodiment, "means for releasing said capture probes from the surface of a solid substrate" comprises a cleavage enzyme, such as an enzyme as defined above. It will be evident from the description above that a cleavage enzyme may refer to a combination or mixture of enzymes that have cleavage activity. For instance, in a preferred embodiment, the cleavage enzyme comprises a mixture of Uracil DNA glycosylase (UDG) and a DNA glycosylase-lyase, such as Endonuclease VIII. A commercially available mixture of these enzymes, known as the USER^{™} enzyme, is particularly preferred. Thus, in some embodiments, the cleavage domain comprises a poly-U sequence.

As mentioned above, the cleavage enzyme is contacted with the array at the same time as, or before, the polymerase enzyme, preferably in a single reaction mixture. Accordingly, in some embodiments the cleavage enzyme must be functional in the same conditions as the polymerase enzyme, e.g. functional in the same buffer, salt, temperature conditions used for the polymerase enzyme.

By "functional" is meant that the cleavage enzyme may show some reduced activity in cleaving the cleavage domain of the capture probe relative to the activity of the cleavage enzyme on the cleavage domain in conditions that are optimum for the enzyme, e.g. in the buffer, salt and temperature conditions recommended by the manufacturer. Thus, the cleavage enzyme may be considered to be functional if it has at least 50%, e.g. at least 60, 70, 80, 85, 90, 95, 96, 97, 98, 99 or 100%, activity relative to the activity of the cleavage enzyme in conditions that are optimum for the enzyme.

Additionally or alternatively, in some embodiments the polymerase enzyme must be functional in the same conditions as the cleavage enzyme, e.g. functional in the same buffer, salt, temperature conditions used for the cleavage enzyme.

By "functional" is meant that the polymerase enzyme may show some reduced activity in target templated extension of the capture probe relative to the activity of the polymerase enzyme on the same target templated extension in conditions that are optimum for the enzyme, e.g. in the buffer, salt and temperature conditions recommended by the manufacturer. Thus, the polymerase enzyme may be considered to be functional if it has at least 50%, e.g. at least 60, 70, 80, 85, 90, 95, 96, 97, 98, 99 or 100%, activity relative to the activity of the polymerase enzyme in conditions that are optimum for the enzyme.

Whilst it will be evident from the discussion herein that the method of the invention may be used to capture DNA (e.g. genomic DNA) and RNA, for the sake of simplicity, the invention will be described in detail in the context of capturing RNA, which forms a preferred aspect of the invention. However, particular embodiments in relation to the capture of DNA are presented below.

The RNA may be any RNA molecule which may occur in a cell. Thus it may be mRNA, tRNA, rRNA, viral RNA, small nuclear RNA (snRNA), small nucleolar RNA (snoRNA), microRNA (miRNA), small interfering RNA (siRNA), piwi-interacting RNA (piRNA), ribozymal RNA, antisense RNA or non-coding RNA. Preferably however it is mRNA.

Step (c) in the method above involves extending the capture probes using the nucleic acid molecules hybridised to the capture probes (i.e. "captured" by the capture probes) as extension templates to produce extended probes thereby spatially tagging the nucleic acids of the biological specimen. Thus, in the context of RNA, this aspect of step (c) may be viewed as generating cDNA (particularly tagged cDNA) from the captured RNA, i.e. relating to the synthesis of the cDNA. This will involve a step of reverse transcription (RT) of the captured RNA, extending the capture probe, which functions as the RT primer, using the captured RNA as template. Such a step generates so-called first strand cDNA, i.e. an extended probe. As will be described in more detail below, second strand cDNA synthesis may take place in a separate step, prior to the step of analyzing the extended probes (e.g. the sequence of the extended probes) or may take place as part of the analysis step. Thus, for instance, second strand synthesis may occur in the first step of amplification of a released first strand cDNA molecule. In some embodiments, second strand synthesis may occur contemporaneously with the first strand synthesis or may be performed immediately following the first strand synthesis reaction. For instance, second strand synthesis may occur contemporaneously with the first strand synthesis reaction when a template switching reaction is used for second strand synthesis. Template switching reactions are described in detail below.

Thus, in some embodiments (i.e. when the method is used to capture RNA), the extension reaction is performed using a reverse transcriptase enzyme, i.e. the polymerase enzyme is a reverse transcriptase enzyme. The desired reverse transcriptase activity may be provided by one or more distinct reverse transcriptase enzymes, wherein suitable examples are: M-MLV, MuLV, AMV, HIV, ArrayScript^{™}, MultiScribe^{™}, ThermoScript^{™}, and SuperScript^{®} I, II, and III enzymes. As used herein, the term "reverse transcriptase" includes not only naturally occurring enzymes but also all such modified derivatives, including also derivatives of naturally occurring reverse transcriptase enzymes.

Particularly preferred reverse transcriptase enzymes for use in the methods of the present application include M-MLV, MuLV, AMV and HIV reverse transcriptase enzymes and derivatives, e.g. sequence-modified derivatives, or mutants thereof.

Sequence-modified derivatives or mutants of M-MLV, MuLV, AMV and HIV reverse transcriptase enzymes include mutants that retain at least some of the functional, e.g. reverse transcriptase, activity of the wild-type sequence. Mutations may affect the activity profile of the enzymes, e.g. enhance or reduce the rate of polymerisation, under different reaction conditions, e.g. temperature, template concentration, primer concentration etc. Mutations or sequence-modifications may also affect the RNase activity and/or thermostability of the enzyme. The reverse transcriptase enzyme may be provided as part of a composition which comprises other components, e.g. stabilizing components, that enhance or improve the activity of the reverse transcriptase enzyme, such as RNase inhibitor(s), inhibitors of DNA-dependent DNA synthesis, e.g. actinomycin D. Many sequence-modified derivatives or mutants of reverse transcriptase enzymes, e.g. M-MLV, and compositions comprising unmodified and modified enzymes are known in the art and are commercially available, e.g. ArrayScript^{™}, MultiScribe^{™}, ThermoScript^{™}, and SuperScript^{®} I, II, III and IV enzymes, and all such enzymes are considered to be useful in the methods of the invention.

Hence, in one embodiment the preferred reverse transcriptase enzyme of the description is encoded by a nucleotide sequence comprising SEQ ID NO: 1 (M-MLV reverse transcriptase) or a nucleotide sequence that has at least 80, 85 or 90% sequence identity thereto. Alternatively or additionally, the preferred reverse transcriptase enzyme of the description comprises the polypeptide sequence of SEQ ID NO: 2 or a polypeptide sequence that has at least 80, 85 or 90% sequence identity thereto. Thus, the reverse transcriptase enzyme of the description may be a natural variant or derivative of the M-MLV reverse transcriptase enzyme, e.g. from another strain of M-MLV.

Preferably said nucleotide or polypeptide sequence is at least 90, 91, 92, 93, 94, 95, 96, 97, 98 or 99% identical to the sequence to which it is compared.

Sequence identity of nucleotide molecules may be determined by, e.g. FASTA Search using GCG packages, with default values and a variable pamfactor, and gap creation penalty set at 12.0 and gap extension penalty set at 4.0 with a window of 6 nucleotides.

Preferably such sequence identity related nucleic acid molecules are functionally equivalent to the nucleic acid molecule which is set forth in SEQ ID NO: 1. Such functionally equivalent nucleic acid molecules may take the form of derivatives and are considered functionally equivalent if they encode polypeptides which would be considered functional equivalents according to the reverse transcriptase activity tests known in the art. Preferred functional equivalents are those which encode the preferred polypeptides as set out above.

Sequence identity of polypeptide molecules may be determined by, e.g. using the SWISS-PROT protein sequence databank using FASTA pep-cmp with a variable pamfactor, and gap creation penalty set at 12.0 and gap extension penalty set at 4.0, and a window of 2 amino acids. Preferably said comparison is made over the full length of the sequence, but may be made over a smaller window of comparison, e.g. less than 600, 500, 400, 300, 200, 100 or 50 contiguous amino acids.

Preferably such sequence identity related polypeptides are functionally equivalent to the polypeptide which is set forth in SEQ ID NO: 2. As such, the polypeptide with a sequence as set forth in SEQ ID NO: 2 may be modified without affecting the sequence of the polypeptide.

Modifications that do not affect the sequence of the polypeptide include, e.g. chemical modification, including by deglycosylation or glycosylation. Such polypeptides may be prepared by post-synthesis/isolation modification of the polypeptide without affecting functionality, e.g. certain glycosylation, methylation etc. of particular residues.

As referred to herein, to achieve "functional equivalence" the polypeptide may show some increased or reduced efficacy in reverse transcriptase activity relative to the parent molecule (i.e. the molecule from which it was derived, e.g. by amino acid substitution), but preferably is as efficient or is more efficient. In some embodiments, part of the native enzyme activity may be reduced or inhibited in the functionally equivalent polypeptide, e.g. RNase activity. Thus, functional equivalence relates to a polypeptide which has reverse transcriptase activity capable of extending the capture domains of the capture probes in a RNA templated extension reaction. This may be tested by comparison of the reverse transcriptase activity of the derivative polypeptide relative to the polypeptide from which it is derived in a quantitative manner, as described above. The derivative is preferably at least 30, 50, 70 or 90% as effective as the parent polypeptide in the methods of the invention.

Functionally-equivalent proteins which are related to or derived from the naturally-occurring protein, may be obtained by modifying the native amino acid sequence by single or multiple amino acid substitution, addition and/or deletion (providing they satisfy the above-mentioned sequence identity requirements), but without destroying the molecule's function. Preferably the native sequence has less than 20 substitutions, additions or deletions, e.g. less than 10, 5, 4, 3, 2, or 1 such modifications. Such proteins are encoded by "functionally-equivalent nucleic acid molecules" which are generated by appropriate substitution, addition and/or deletion of one or more bases.

As used herein the term "multiple" means two or more, or at least two, e.g. 3, 5, 10, 15, 20, 30, 40, 50, 60, 70, 80, 90, 100, 150, 200, 400, 500, 1000, 2000, 5000, 10,000, 100,000, 1000,000, 10,000,000, 15,000,000 or more etc. Thus for example, the number of capture probes may be any integer in any range between any two of the aforementioned numbers.

As discussed above, the capture probes are immobilised on a solid substrate and thus the solid substrate may therefore be viewed as an array or comprising one or more arrays, e.g. 2, 3, 4, 5, 6, 7, 8, 9, 10, 11, 12, 13, 14, 15, 16, 17, 18, 19, 20 or more arrays. For instance, a solid substrate may comprise 20, 25, 30, 35, 40 or more arrays. Arrays for use in the context of nucleic acid analysis in general, and DNA analysis in particular, are discussed and described below. Specific details and embodiments described herein in relation to arrays and capture probes for use in the context of RNA, apply equally (where appropriate) to all such arrays, including those for use with DNA. In this respect, it will be appreciated however that it is envisaged that conventional-type arrays comprising capture probes as defined herein may be used in the methods of the invention.

Thus, the methods outlined herein may utilise high density nucleic acid arrays comprising "capture probes" as defined herein for capturing and tagging transcripts from all of the single cells within a biological specimen, e.g. tissue sample, such as a thin tissue sample slice, or "section". Tissue samples or sections for analysis may be produced in a highly parallelized fashion, such that the spatial information in the section is retained. The captured RNA (preferably mRNA) molecules for each cell, or "transcriptomes", are transcribed into cDNA and the resultant cDNA molecules are analysed, for example by high throughput sequencing. The resultant data may be correlated to images of the biological specimen, e.g. tissue samples through so-called positional domains, e.g. unique nucleotide sequences within the capture probes, e.g. barcode sequences or ID tags).

High density nucleic acid arrays or microarrays are a core component of the spatial transcriptome and genome labelling and tagging methods described herein. A microarray is a multiplex technology used in molecular biology. A typical microarray consists of an arrayed series of microscopic spots (features or sites) of oligonucleotides (millions of spots can be incorporated on a single array and several arrays can be presented on a single solid substrate). The distinct (discrete) position of each nucleic acid (oligonucleotide) spot (each species of oligonucleotide/nucleic acid molecule) is known as a "feature" or "site" (and hence in the methods set out above each species of capture probe may be viewed as a specific feature of the array; each feature occupies a distinct (discrete) position on the array), and typically each separate feature contains in the region of picomoles (10⁻¹² moles) of a specific DNA sequence (a "species"), which are known as "probes" (or "reporters"). Typically, these can be a short section of a gene or other nucleic acid element to which a cDNA or cRNA sample (or "target") can hybridize under high-stringency hybridization conditions. However, as described below, the probes of the present description differ from the probes of standard microarrays.

Thus, the term "array" refers to a population of nucleic acid features or sites on a solid substrate that can be differentiated from each other according to relative location. Different nucleic acid molecules that are at different sites or features of an array can be differentiated from each other according to the locations of the sites or features in the array. An individual site or feature of an array can include one or more molecules of a particular type (e.g. species of capture probe). For example, a site or feature can include a single nucleic acid molecule having a particular sequence or a site can include several nucleic acid molecules having the same sequence.

The capture probes may be attached to the solid substrate, e.g. array, of the description by any suitable means. As used herein, the term "attached" refers to the state of two things being joined, fastened, adhered, connected or bound to each other. For example, a nucleic acid can be attached to a material, such as a gel or solid support, by a covalent or non-covalent bond. A covalent bond is characterized by the sharing of pairs of electrons between atoms. A non-covalent bond is a chemical bond that does not involve the sharing of pairs of electrons and can include, for example, hydrogen bonds, ionic bonds, van der Waals forces, hydrophilic interactions and hydrophobic interactions.

In a preferred embodiment the probes are immobilized to the solid substrate, e.g. array, by chemical immobilization, i.e. by a covalent bond. This may be an interaction between the solid substrate and the probe based on a chemical reaction. Such a chemical reaction typically does not rely on the input of energy via heat or light, but can be enhanced by either applying heat, e.g. a certain optimal temperature for a chemical reaction, or light of certain wavelength. For example, a chemical immobilization may take place between functional groups on the substrate and corresponding functional elements on the probes. Such corresponding functional elements in the probes may either be an inherent chemical group of the probe, e.g. a hydroxyl group or be additionally introduced. An example of a functional group introduced to the capture probe is an amine group. Typically, the probe to be immobilized comprises a functional amine group or is chemically modified in order to comprise a functional amine group. As discussed above, in some embodiments the probe may comprise a cleavable chemical linker which attaches the probe to the solid substrate, such that it forms a cleavage domain of the capture probe. Means and methods for such a chemical modification are well known.

The localization of said functional group within the probe to be immobilized may be used in order to control and shape the binding behaviour and/or orientation of the probe, e.g. the functional group may be placed at the 5' or 3' end of the probe or within sequence of the probe. A typical substrate on which a probe may be immobilized comprises moieties which are capable of binding to such probes, e.g. to amine-functionalized nucleic acids. Examples of such substrates are carboxy, aldehyde or epoxy substrates. Such materials are known to the person skilled in the art. Functional groups, which impart a connecting reaction between probes which are chemically reactive by the introduction of an amine group, and array substrates are known to the person skilled in the art.

Alternative substrates on which probes may be immobilized may have to be chemically activated, e.g. by the activation of functional groups, available on the solid substrate. The term "activated substrate" relates to a material in which interacting or reactive chemical functional groups were established or enabled by chemical modification procedures as known to the person skilled in the art. For example, a substrate comprising carboxyl groups has to be activated before use. Furthermore, there are substrates available that contain functional groups that can react with specific moieties already present in the nucleic acid probes.

In some embodiments, the probes may be synthesized directly on the solid substrate. Suitable methods for such an approach are known to the person skilled in the art. For instance, features (i.e. clusters of probes) may be made by a solid-phase amplification method known as bridge amplification, as discussed in more detail below. Other examples including manufacture techniques developed by Agilent Inc., Affymetrix Inc., Roche Nimblegen Inc. or Flexgen BV. Typically, lasers and a set of mirrors that specifically activate the spots where nucleotide additions are to take place are used. Such an approach may provide, for example, spot sizes (i.e. features) of around 30 µm or larger.

The solid substrate therefore may be any suitable substrate known to the person skilled in the art, i.e. suitable for use as an array. The substrate may have any suitable form or format, e.g. it may be flat, curved, e.g. convexly or concavely curved towards the area where the interaction between the biological specimen, e.g. tissue sample, and the substrate takes place. Particularly preferred is the where the substrate is a flat, i.e. planar, chip or slide. In some embodiments, the substrate may comprise various structures such as wells, beads (or other particles) in or on a substrate, projections from the substrate, ridges on the substrate or channels in a substrate. In some embodiments, the structures of a substrate, e.g. wells or beads, can each bear a different capture probe. Different capture probes attached to each structure can be identified according to the locations of the structures in or on the surface of the substrate. Exemplary arrays in which separate structures are located on the substrate include, without limitation, those having beads in wells.

The term "solid substrate" typically refers to a rigid support that is insoluble in aqueous liquid and which allows for an accurate and traceable positioning of the probes on the substrate. An example of a substrate is a solid material or a substrate comprising functional chemical groups, e.g. amine groups or amine-functionalized groups. The substrate can be non-porous or porous. Thus, the substrate can optionally be capable of taking up a liquid (e.g. due to porosity) but will typically be sufficiently rigid that the substrate does not swell substantially when taking up the liquid and does not contract substantially when the liquid is removed by drying. A non-porous solid support is generally impermeable to liquids or gases. Exemplary solid supports include, but are not limited to, glass and modified or functionalized glass, plastics (including acrylics, polystyrene and copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, metals, inorganic glasses, optical fiber bundles, and polymers, such as polystyrene, cyclic olefin copolymers (COCs), cyclic olefin polymers (COPs), polypropylene, polyethylene and polycarbonate. A preferred substrate envisaged by the present invention is a non-porous substrate. In some embodiments, the solid substrate is located within a flow cell apparatus.

Any suitable material known to the person skilled in the art may be used. Typically, glass or polystyrene is used. Polystyrene is a hydrophobic material suitable for binding negatively charged macromolecules because it normally contains few hydrophilic groups. For nucleic acids immobilized on glass slides, it is furthermore known that by increasing the hydrophobicity of the glass surface the nucleic acid immobilization may be increased. Such an enhancement may permit a relatively more densely packed formation. In addition to a coating or surface treatment with poly-L-lysine, the substrate, in particular glass, may be treated by silanation, e.g. with epoxy-silane or amino-silane or by silynation or by a treatment with polyacrylamide.

A number of standard arrays are commercially available and both the number and size of the features may be varied. In the present invention, the arrangement of the features may be altered to correspond to the size and/or density of the cells present in different biological specimens, e.g. tissues or organisms. For instance, animal cells typically have a cross-section in the region of 1-100µm, whereas the cross-section of plant cells typically may range from 1-10000µm. Hence, Nimblegen^{®} arrays, which are available with up to 2.1 million features, or 4.2 million features, and feature sizes of 13 micrometers, may be preferred for tissue samples from an animal or fungus, whereas other formats, e.g. with 8x130k features, may be sufficient for plant tissue samples. Commercial arrays are also available or known for use in the context of sequence analysis and in particular in the context of NGS technologies. Such arrays may also be used as the array surface in the context of the present invention e.g. an Illumina bead array or flow cell, on which the probes are produced using bridge amplification. In addition to commercially available arrays, which can themselves be customized, it is possible to make custom or non-standard "in-house" arrays and methods for generating arrays are well-established. The methods of the invention may utilise both standard and non-standard arrays that comprise probes as defined below.

The term "flow cell" is intended to mean a vessel having a chamber where a reaction can be carried out, an inlet for delivering reagents to the chamber and an outlet for removing reagents from the chamber. In some embodiments the chamber is configured for detection of the reaction that occurs in the chamber. For example, the chamber can include one or more transparent surfaces allowing optical detection of biological specimens, optically labelled molecules, or the like in the chamber. Exemplary flow cells include, but are not limited to those used in a nucleic acid sequencing apparatus such as flow cells for the Genome Analyzer^{®}, MiSeq^{®}, NextSeq^{®} or HiSeq^{®} platforms commercialized by Illumina, Inc. (San Diego, CA); or for the SOLiD^{™} or Ion Torrent^{™} sequencing platform commercialized by Life Technologies (Carlsbad, CA). Exemplary flow cells and methods for their manufacture and use are also described, for example, in WO 2016/162309, WO 2014/142841, U.S. Pat. App. Pub. No. 2010/0111768 and U.S. Pat. No. 8,951,781.

The term "bead array" refers to a solid support comprising a collection of beads or other particles. The particles can be suspended in a solution or they can be located on the surface of a solid substrate. Examples of arrays having beads located on a surface include those wherein beads are located in wells such as a BeadChip array (Illumina Inc., San Diego CA), substrates used in sequencing platforms from 454 LifeSciences (a subsidiary of Roche, Basel Switzerland) or substrates used in sequencing platforms from Ion Torrent (a subsidiary of Life Technologies, Carlsbad California). Other solid supports having beads located on a surface are described in US Pat. Nos. 6,266,459; 6,355,431; 6,770,441; 6,859,570; 6,210,891; 6,258,568; or 6,274,320; US Pat. App. Publ. Nos. 2009/0026082 A1; 2009/0127589 A1; 2010/0137143 A1; or 2010/0282617 A1 or PCT Publication No. WO 00/63437 and WO 2016/162309. Several of the above references describe methods for attaching nucleic acid probes to beads prior to loading the beads in or on a solid support. As such, the collection of beads can include different beads each having a unique capture probe attached. It will however, be understood that the beads can be made to include universal primers, and the beads can then be loaded onto an array, thereby enabling the synthesis of capture probes on the array, e.g. using bridge amplification methods. As set forth previously herein, the solid supports typically used for bead arrays can be used without beads. For example, nucleic acids, such as capture probes, can be attached directly to the wells or to gel material in wells. Thus, the above references are illustrative of materials, compositions or apparatus that can be modified for use in the methods and compositions set forth herein.

Accordingly, a solid substrate for use in the method of the invention can include an array of beads, wherein different capture probes are attached to different beads in the array. In this embodiment, each bead can be attached to a different capture probe and the beads can be randomly distributed on the solid support in order to effectively immobilize the different capture probes to the solid support. Optionally, the solid support can include wells having dimensions that accommodate no more than a single bead. In such a configuration, the beads may be attached to the wells due to forces resulting from the fit of the beads in the wells. It is also possible to use attachment chemistries or adhesives to hold the beads in the wells.

The probes on a solid substrate, e.g. array, may be immobilized, i.e. attached or bound, to the array preferably via the 5' or 3' end, depending on the chemical matrix of the array. Typically, for commercially available arrays, the probes are attached via a 3' linkage, thereby leaving a free 5' end. However, arrays comprising probes attached to the substrate via a 5' linkage, thereby leaving a free 3' end, are available and may be synthesized using standard techniques that are well known in the art and are described elsewhere herein.

The covalent linkage used to couple a nucleic acid probe to a solid substrate, e.g. array, may be viewed as both a direct and indirect linkage, in that the although the probe is attached by a "direct" covalent bond, there may be a chemical moiety or linker separating the "first" nucleotide of the nucleic acid probe from the, e.g. glass or silicon, substrate, i.e. an indirect linkage. For the purposes of the present invention probes that are immobilized to the substrate by a covalent bond and/or chemical linker are generally seen to be immobilized or attached directly to the substrate. As mentioned above, in some embodiments the chemical linker is cleavable, e.g. enzymatically and/or chemically, such that it forms the cleavage domain of the capture probe.

As will be described in more detail below, the capture probes of the description may be immobilized on, or interact with, the solid substrate, e.g. array, directly or indirectly. Thus the capture probes need not bind directly to the array, but may interact indirectly, for example by binding to a molecule or entity (e.g. bead) which itself binds directly or indirectly to the solid substrate, e.g. array (e.g. the capture probe may interact with (e.g. bind or hybridize to) a binding partner for the capture probe, i.e. a surface probe, which is itself bound to the array directly or indirectly). Generally speaking, however, the capture probe will be, directly or indirectly (by one or more intermediaries), bound to, or immobilized on, the solid substrate, e.g. array.

The method of the description may comprise probes that are immobilized via their 5' or 3' end. However, when the capture probe is immobilized directly to the solid substrate, e.g. it may be preferred that it is immobilized such that the 3' end of the capture probe is free to be extended, e.g. it is immobilized by its 5' end. In some embodiments, the capture probe may be immobilized indirectly, such that it has a free, i.e. extendible, 3' end.

By extended or extendible 3' end, it is meant that further nucleotides may be added to the most 3' nucleotide of the nucleic acid molecule, e.g. capture probe, to extend the length of the nucleic acid molecule, i.e. the standard polymerization reaction utilized to extend nucleic acid molecules, e.g. templated polymerization catalyzed by a polymerase, e.g. reverse transcriptase.

Thus, in one embodiment, the array comprises probes that are immobilized directly via their 3' end, so-called surface probes, which are defined below. Each species of surface probe comprises a region of complementarity to each species of capture probe, such that the capture probe may hybridize to the surface probe, resulting in the capture probe comprising a free extendible 3' end. However, it will be evident that, in some embodiments, the surface probes may be immobilized directly via their 5' end, such that cleavage of the cleavage domain is required to release the 3' end of the capture probe, i.e. to expose the capture domain for extension. In a preferred aspect of the invention, when the solid substrate, e.g. array, comprises surface probes, the capture probes are synthesized *in situ* on the array.

The probes may be made up of ribonucleotides and/or deoxyribonucleotides as well as synthetic nucleotide residues that are capable of participating in Watson-Crick type or analogous base pair interactions. Thus, the nucleic acid domain may be DNA or RNA or any modification thereof e.g. PNA or other derivatives containing non-nucleotide backbones. However, in the context of transcriptome analysis the capture domain of the capture probe must capable of priming a reverse transcription reaction to generate cDNA that is complementary to the captured RNA molecules. As described below in more detail, in the context of genome analysis, the capture domain of the capture probe must be capable of binding to the DNA fragments, which may comprise binding to a binding domain that has been added to the fragmented DNA. In some embodiments, the capture domain of the capture probe may prime a DNA extension (polymerase) reaction to generate DNA that is complementary to the captured DNA molecules.

In a preferred embodiment of the invention at least the capture domain of the capture probe comprises or consists of deoxyribonucleotides (dNTPs). In a particularly preferred embodiment the whole of the capture probe comprises or consists of deoxyribonucleotides.

In a preferred embodiment of the description the capture probes are immobilized on the substrate of the array directly, i.e. by their 5' end, resulting in a free extendible 3' end.

The capture probes of the description comprise at least three domains, a capture domain and a positional domain (or a feature identification tag or domain; the positional domain may alternatively be defined as an identification (ID) domain or tag, or as a positional tag) and a cleavage domain, as defined above. The capture probe may further comprise an amplification domain as defined further below. Where the capture probe is indirectly attached to the array surface via hybridization to a surface probe, the surface probe requires a sequence (e.g. a portion or domain) which is complementary to the capture probe. For instance, when the surface probe is immobilized on the solid substrate via its 3' end, such a complementary sequence may be complementary to a positional/identification domain and/or an amplification domain and/or cleavage domain on the capture probe. In other words the positional domain and/or amplification domain and/or cleavage domain may constitute the region or portion of the capture probe which is complementary to the surface probe. However, the capture probe may also comprise an additional domain (or region, portion or sequence) which is complementary to the surface probe.

Alternatively, when the surface probe is immobilized on the solid substrate via its 5' end, such a complementary sequence may be complementary to a cleavage domain and/or capture domain and/or positional/identification domain and/or an amplification domain the capture probe, preferably the cleavage domain. In other words the cleavage domain and/or capture domain and/or positional/identification domain and/or an amplification domain may constitute the region or portion of the capture probe which is complementary to the surface probe. However, the capture probe may also comprise an additional domain (or region, portion or sequence) which is complementary to the surface probe.

The capture domain is typically located at the 3' end of the capture probe and comprises a free 3' end that can be extended by template dependent polymerization. However, it will be evident that in some embodiments, e.g. when the capture probe is immobilized directly or indirectly by its 3' end, the cleavage domain may be located at the 3' end and the capture domain is located directly or indirectly upstream of (i.e. at the 5' end of) the cleavage domain. The capture domain comprises a nucleotide sequence that is capable of hybridizing to nucleic acid, e.g. RNA (preferably mRNA), present in the cells of the biological specimen, e.g. tissue sample, contacted with the solid substrate, e.g. array.

Advantageously, the capture domain may be selected or designed to bind (or put more generally may be capable of binding) selectively or specifically to the particular nucleic acid, e.g. RNA, it is desired to detect or analyse. For example the capture domain may be selected or designed for the selective capture of mRNA. As is well known in the art, this may be on the basis of hybridisation to the poly-A tail of mRNA. Thus, in a preferred embodiment the capture domain comprises a poly-T oligonucleotide, i.e. a series of consecutive deoxythymidine residues linked by phosphodiester bonds, which is capable of hybridizing to the poly-A tail of mRNA. Alternatively, the capture domain may comprise nucleotides which are functionally or structurally analogous to poly-T, i.e. are capable of binding selectively to poly-A, for example a poly-U oligonucleotide or an oligonucleotide comprised of deoxythymidine analogues or combinations thereof, wherein said oligonucleotide retains the functional property of binding to poly-A. In a particularly preferred embodiment the capture domain, or more particularly the poly-T and/or -U element of the capture domain, comprises at least 10 nucleotides, preferably at least 11, 12, 13, 14, 15, 16, 17, 18, 19 or 20 nucleotides. In a further embodiment, the capture domain, or more particularly the poly-T and/or -U element of the capture domain comprises at least 25, 30 or 35 nucleotides.

Random sequences may also be used in the capture of nucleic acid, as is known in the art, e.g. random hexamers or similar sequences, and hence such random sequences may be used to form all or a part of the capture domain. For example, random sequences may be used in conjunction with poly-T (or poly-T analogue etc.) sequences. Thus, where a capture domain comprises a poly-T (or a "poly-T-like") oligonucleotide, it may also comprise a random oligonucleotide sequence. This may for example be located 5' or 3' of the poly-T sequence, e.g. at the 3' end of the capture probe, but the positioning of such a random sequence is not critical. Such a construct may facilitate the capturing of the initial part of the poly-A of mRNA. Alternatively, the capture domain may be an entirely random sequence. Degenerate capture domains may also be used, according to principles known in the art.

The capture domain may be capable of binding selectively to a desired subtype or subset of nucleic acid, e.g. RNA, for example a particular type of RNA such mRNA or rRNA etc. as listed above, or to a particular subset of a given type of RNA, for example, a particular mRNA species, e.g. corresponding to a particular gene or group of genes. Such a capture probe may be selected or designed based on sequence of the RNA it is desired to capture. Thus it may be a sequence-specific capture probe, specific for a particular RNA target or group of targets (target group etc). Thus, it may be based on a particular gene sequence or particular motif sequence or common/conserved sequence etc., according to principles well known in the art.

In embodiments where the capture domain comprises a sequence specific for a particular target gene or group of genes it may be desirable to synthesise the capture probes on the solid substrate directly, e.g. using ligation reactions. In particular, it may be useful to synthesise the capture domain of the capture probe on the solid substrate directly.

For instance, a solid substrate may be provided comprising capture probes immobilized by their 5' end in which the capture domain is replaced with a universal domain that is common to all of the probes on the solid substrate (such probes may be viewed as a positional domain oligonucleotide). The substrate is contacted with one or more ligation helper probes that comprise 5' to 3':
(i) a domain that is complementary to a sequence at the 5' of the capture domain; and
(ii) a domain that is complementary to a universal domain in the probes immobilized on the solid substrate,

under conditions that allow the ligation helper probe to hybridise to the probes immobilized on the solid substrate. The substrate is also contacted with one or more species of probe containing a capture domain (such probes may be viewed as a capture domain oligonucleotide), wherein the probes comprise 5' to 3':
   (i) a domain that is complementary to a sequence at the 5' of the helper probe; and
   (ii) a capture domain comprising a sequence specific for a particular target gene,
under conditions that allow the probes comprising the capture domain to hybridise to the helper probes.

Finally the substrate is contacted with components (e.g. a ligase enzyme and appropriate buffers, salts etc.) that facilitate the ligation of the probes containing the capture domain to the probes immobilized on the solid substrate using the ligation helper probes as a ligation template to form capture probes. Thus, if one species of probe containing the capture domain is ligated to the probes immobilized on the solid substrate, each feature of the array will be capable of capturing a single target nucleic acid from the biological specimen, e.g. to analyse the expression of a single gene. However, if more than one species of probe containing the capture domain is ligated to the probes immobilized on the solid substrate (i.e. each species of probe contains a capture domain comprising a sequence specific for a different target gene), each feature of the array will be capable of capturing multiple target nucleic acids from the biological specimen, e.g. to analyse the expression of a subset of genes. It will be evident that the ligation helper probes, probes containing the capture domain and components that facilitate the ligation of the probes may be contacted with the solid substrate separately (e.g. sequentially) or simultaneously.

Whilst a solid substrate comprising capture probes that are specific for a single gene or groups of genes may be produced by any suitable means, it will be evident that the method described above provides a convenient method of generating multiple gene specific substrates based on a single array platform. In this respect, a common or universal substrate comprising capture probes in which the capture domain is replaced with a universal domain that is common to all of the probes on the solid substrate may be manufactured and used to generate multiple substrates each specific for a particular target gene or group of target genes. Such substrates may find particular utility in diagnostic assays, e.g. for analysing the expression of genes or groups of genes that are associated with one or more diseases, e.g. cancer.

Thus, step (a) of the method described above may comprise:
(1) providing a solid substrate on which multiple species of probes are immobilized such that each species occupies a distinct position on the solid substrate and is oriented to have a free 3' end, wherein said probe is for a templated ligation reaction, wherein each species of said probe comprises a nucleic acid molecule with 5' to 3':
   (i) a cleavage domain for releasing the capture probe from the surface of the solid substrate,
   (ii) a positional domain that corresponds to the position of the capture probe on the solid substrate, and optionally
   (iii) a universal domain;
(2) contacting said solid substrate with:
   (i) a ligation helper probe as defined above;
   (ii) one or more species of probe containing a capture domain as defined above; and
   (iii) components that facilitate the ligation of the probes in (ii) to the probes in (1) using the helper probe in (2)(i) as a ligation template,
under suitable conditions, e.g. conditions that suitable form hybridisation of the complementary domains of the probes and the ligation of the probes in (ii) to the probes in (1) to form capture probes, which are for a primer extension reaction.

Suitable enzymes and conditions for performing ligation reactions are discussed in detail below and may be applied to the method described above.

The positional domain (feature identification domain or tag) of the capture probe is located directly or indirectly upstream, i.e. closer to the 5' end of the capture probe nucleic acid molecule, of the capture domain. Preferably the positional domain is directly adjacent to the capture domain, i.e. there is no intermediate sequence between the capture domain and the positional domain. In some embodiments the positional domain forms the 5' end of the capture probe. For instance, the positional domain may form the 5' end of the capture probe when the cleavage domain is a cleavable cross-linker attaching the capture probe to the solid substrate. Additionally or alternatively, the positional domain may form the 5' end of the capture probe when the capture probe is immobilized to the substrate via its 3' end.

As discussed above, each feature (distinct position) of the solid substrate, e.g. array, comprises a spot of a species of nucleic acid probe, wherein the positional domain at each feature (or groups of adjacent features) is unique. Thus, a "species" of capture probe is defined with reference to its positional domain; a single species of capture probe will have the same positional domain. However, it is not required that each member of a species of capture probe has the same sequence in its entirety. In particular, since the capture domain may be or may comprise a random or degenerate sequence, the capture domains of individual probes within a species may vary. Accordingly, in some embodiments where the capture domains of the capture probes are the same, each feature comprises a single probe sequence. However, in other embodiments where the capture probe varies, members of a species of probe will not have the exact same sequence, although the sequence of the positional domain of each member in the species will be the same. What is required is that each feature or site (or groups of adjacent features or sites) of the solid substrate, e.g. array, carries a capture probe of a single species (specifically each feature or position carries a capture probe which has an identical positional tag, i.e. there is a single positional domain at each feature or position or groups of adjacent features or positions). Each species has a different positional domain which identifies the species. However, each member of a species, may in some cases, as described in more detail herein, have a different capture domain, as the capture domain may be random or degenerate or may have a random or degenerate component or, as described above, capture domains specific for subsets of genes may be produced at each feature. This means that within a given feature, or position, the capture domain of the probes may differ.

Thus in some, but not necessarily in all embodiments, the nucleotide sequence of any one probe molecule immobilized at a particular feature is the same as the other probe molecules immobilized at the same feature, but the nucleotide sequence of the probes at each feature (or groups of adjacent features) is different, distinct or distinguishable from the probes immobilized at every other feature. Preferably each feature comprises a different species of probe. However, in some embodiments it may be advantageous for a group of adjacent features to comprise the same species of probe, i.e. effectively to produce a feature covering an area of the array that is greater than a single feature, e.g. to lower the resolution of the array. In other embodiments, the nucleotide sequence of the positional domain of any one probe molecule immobilized at a particular feature may be the same as the other probe molecules immobilized at the same feature but the capture domain may vary. The capture domain may nonetheless be designed to capture the same type of molecule, e.g. mRNA, in general. Thus, for instance, where the features comprise capture probes containing multiple capture domains, e.g. capture domains each specific for a particular gene, each feature is capable of capturing the same target nucleic acids.

The positional domain (or tag) of the capture probe comprises the sequence which is unique to each feature (or group of adjacent features) and acts as a positional or spatial marker (the identification tag). In this way each region or domain of the biological specimen, e.g. tissue sample, e.g. each cell in the tissue, will be identifiable by spatial resolution across the solid substrate, e.g. array, linking the nucleic acid, e.g. RNA (e.g. the transcripts) from a certain cell to a unique positional domain sequence in the capture probe. By virtue of the positional domain a capture probe in the array may be correlated to a position in the tissue sample, for example it may be correlated to a cell in the sample. Thus, the positional domain of the capture domain may be seen as a nucleic acid tag (identification tag).

Any suitable sequence may be used as the positional domain in the capture probes of the description. By a suitable sequence, it is meant that the positional domain should not interfere with (i.e. inhibit or distort) the interaction between the nucleic acid, e.g. RNA, of the biological specimen, e.g. tissue sample, and the capture domain of the capture probe. For example, the positional domain should be designed such that nucleic acid molecules in the tissue sample do not hybridize specifically to the positional domain. Preferably, the nucleic acid sequence of the positional domain of the capture probes has less than 80% sequence identity to the nucleic acid sequences in the biological specimen, e.g. tissue sample. Preferably, the positional domain of the capture probe has less than 70%, 60%, 50% or less than 40% sequence identity across a substantial part of the nucleic acids molecules in the biological specimen, e.g. tissue sample. Sequence identity may be determined by any appropriate method known in the art, e.g. the using BLAST alignment algorithm.

In a preferred embodiment the positional domain of each species of capture probe contains a barcode sequence. The barcode sequences may be generated using random sequence generation. The randomly generated sequences may be followed by stringent filtering by mapping to the genomes of all common reference species and with pre-set Tm intervals, GC content and a defined distance of difference to the other barcode sequences to ensure that the barcode sequences will not interfere with the capture of the nucleic acid, e.g. RNA, from the tissue sample and will be distinguishable from each other without difficulty.

In some embodiments of the invention, as described elsewhere herein, it may be advantageous to amplify the extended probes, e.g. tagged cDNA molecules, produced by the methods of the invention. Thus, in some embodiments, the capture probe comprises an amplification domain, i.e. a domain that is common to all of the capture probes and facilitates the amplification of all of the extended probes in a single reaction. The amplification domain of the capture probe is located directly or indirectly upstream, i.e. closer to the 5' end of the capture probe nucleic acid molecule, of the positional domain. In some embodiments, e.g. when the capture probe is immobilized on the array via its 5' end, the amplification domain may be located directly or indirectly downstream, i.e. closer to the 3' end of the capture probe nucleic acid molecule, of the cleavage domain, i.e. the amplification domain may be located between the cleavage domain and the positional domain. Thus, in some embodiments the amplification domain is directly adjacent to the positional domain, i.e. there is no intermediate sequence between the positional domain and the amplification domain.

However, in some embodiments, e.g. when the capture probe is immobilized on the array via its 3' end, the amplification domain may be located indirectly upstream, i.e. closer to the 5' end of the capture probe nucleic acid molecule, of the cleavage domain. Thus, in some embodiments where the capture probe comprises an amplification domain, the domain may form the 5' end of the capture probe. For instance, the amplification domain may form the 5' end of the capture probe when the cleavage domain is a cleavable cross-linker attaching the capture probe to the solid substrate, e.g. array. Alternatively, the amplification domain may form the 5' end of the capture probe when the capture probe is immobilized via its 5' end, e.g. when the cleavage domain is located at the 3' end of the capture probe.

It will be appreciated that amplification of the extended probes may take place on the solid substrate, e.g. array, i.e. the extended probes do not need to be removed or collected (e.g. pooled) from the substrate, e.g. transferred to a separate vessel or container, prior to performing the amplification step. Nevertheless, in some embodiments it may be convenient to remove or collect (e.g. pool) the extended probes prior to amplification (or second strand synthesis, as discussed below). In this respect, the method may comprise a step of pooling the extended probes from step (c) (e.g. to form a mixture of the extended probes). The step of pooling the extended probes may be viewed as removing, collecting and/or transferring the solution comprising the extended probes from the solid substrate, e.g. array, to a separate vessel or container, e.g. a PCR tube.

The amplification domain comprises a distinct sequence to which an amplification primer may hybridize. The amplification domain of the capture probe is preferably identical for each species of capture probe. Hence a single amplification reaction will be sufficient to amplify all of the extended probes, e.g. tagged cDNA molecules.

Any suitable sequence may be used as the amplification domain in the capture probes of the description. By a suitable sequence, it is meant that the amplification domain should not interfere with (i.e. inhibit or distort) the interaction between the nucleic acid, e.g. RNA, of the biological specimen, e.g. tissue sample, and the capture domain of the capture probe. Furthermore, the amplification domain should comprise a sequence that is not the same or substantially the same as any sequence in the nucleic acid, e.g. RNA, of the biological specimen, e.g. tissue sample, such that the primer used in the amplification reaction can hybridized only to the amplification domain under the amplification conditions of the reaction.

For example, the amplification domain should be designed such that nucleic acid molecules in the biological specimen, e.g. tissue sample, do not hybridize specifically to the amplification domain or the complementary sequence of the amplification domain. Preferably, the nucleic acid sequence of the amplification domain of the capture probes and the complement thereof has less than 80% sequence identity to the nucleic acid sequences in the tissue sample. Preferably, the positional domain of the capture probe has less than 70%, 60%, 50% or less than 40% sequence identity across a substantial part of the nucleic acid molecules in the tissue sample. Sequence identity may be determined by any appropriate method known in the art, e.g. the using BLAST alignment algorithm.

In one representative embodiment of the invention only the positional domain of each species of capture probe is unique. Hence, the capture domains, cleavage domains and amplification domains (if present) are in one embodiment the same for every species of capture probe for any particular array to ensure that the capture of the nucleic acid, e.g. RNA, from the biological specimen, e.g. tissue sample, release of the capture probes and amplification of the extended probes is uniform across the array. However, as discussed above, in some embodiments the capture domains may differ by virtue of including random or degenerate sequences or capture domains specific for subsets of specific genes.

In embodiments where the capture probe is immobilized on the substrate of the array indirectly, e.g. via hybridisation to a surface probe, the capture probe may be synthesised on the array as described below.

For example, the surface probes may be immobilized on the substrate of the array directly by or at, e.g. their 3' end. Each species of surface probe is unique to each feature (or group of adjacent features) (distinct position) of the array and is partly complementary to the capture probe, defined above.

Hence, in some embodiments, the surface probe comprises at its 5' end a domain (complementary capture domain) that is complementary to a part of the capture domain that does not bind to the nucleic acid, e.g. RNA, of the biological specimen, e.g. tissue sample. In other words, it comprises a domain that can hybridize to at least part of a capture domain oligonucleotide. The surface probe further comprises a domain (complementary positional domain or complementary feature identification domain) that is complementary to the positional domain of the capture probe and a domain (complementary cleavage domain) that is complementary to the cleavage domain of the capture probe. The complementary positional domain is located directly or indirectly downstream (i.e. at the 3' end) of the complementary capture domain, i.e. there may be an intermediary or linker sequence separating the complementary positional domain and the complementary capture domain. The complementary cleavage domain is located directly or indirectly downstream (i.e. at the 3' end) of the complementary positional domain, i.e. there may be an intermediary or linker sequence separating the complementary cleavage domain and the complementary positional domain. When the capture probe comprises an amplification domain, the surface probe may also contain a domain (complementary amplification domain) that is complementary to the amplification domain and located directly or indirectly downstream (i.e. at the 3' end) of the complementary positional domain, i.e. there may be an intermediary or linker sequence separating the complementary amplification domain and the complementary positional domain. However, the complementary amplification domain will be located upstream (i.e. at the 5' end) of the complementary cleavage domain, i.e. to ensure that the extended capture probe contains the amplification domain, the positional domain and the extended sequence, i.e. the sequence at the 3' end of the capture domain.

In embodiments where the capture probe is synthesized on the solid substrate surface, e.g. array, the surface probes of the substrate, e.g. array, may comprise a domain (e.g. complementary amplification domain) at the 3' end of the surface probe, i.e. directly or indirectly downstream of the complementary cleavage domain, which is complementary to a domain (e.g. amplification domain) of the capture probe.

In some embodiments of the invention the sequence of the surface probe shows 100% complementarity or sequence identity to the positional, cleavage and amplification (if present) domains and to the part of the capture domain that does not bind to the nucleic acid, e.g. RNA, of the biological specimen, e.g. tissue sample. In other embodiments the sequence of the surface probe may show less than 100% sequence identity to the domains of the capture probe, e.g. less than 99%, 98%, 97%, 96%, 95%, 94%, 93%, 92%, 91% or 90%. In a particularly preferred embodiment of the invention, the complementary cleavage domain shares less than 100% sequence identity to the cleavage domain of the capture probe.

As noted above, in some embodiments of the invention, the capture probe may be synthesized or generated on the substrate of the array.

In a representative embodiment, the array comprises surface probes as defined above. Oligonucleotides that correspond to the capture domain and at least the cleavage domain of the capture probe (optionally also including the amplification domain) are contacted with the solid substrate, e.g. array, and allowed to hybridize to the complementary domains of the surface probes. Excess oligonucleotides may be removed by washing the solid substrate, e.g. array, under standard hybridization conditions. The resultant substrate, e.g. array, comprises partially single stranded probes, wherein both the 5' and 3' ends of the surface probe are double stranded and the complementary positional domain (and optionally also the complementary amplification domain) is single stranded. The solid substrate, e.g. array, may be treated with a polymerase enzyme to extend the 3' end of the cleavage domain oligonucleotide, in a template dependent manner, so as to synthesize the positional domain (and optionally the amplification domain) of the capture probe. The 3' end of the synthesized positional domain is then ligated, e.g. using a ligase enzyme, to the 5' end of the capture domain oligonucleotide to generate the capture probe. It will be understood in this regard that the 5' end of the capture domain oligonucleotide is phosphorylated to enable ligation to take place. As each species of surface probe comprises a unique complementary positional domain, each species of capture probe will comprise a unique positional domain.

The term "hybridisation" or "hybridises" as used herein refers to the formation of a duplex between nucleotide sequences which are sufficiently complementary to form duplexes via Watson-Crick base pairing. Two nucleotide sequences are "complementary" to one another when those molecules share base pair organization homology. "Complementary" nucleotide sequences will combine with specificity to form a stable duplex under appropriate hybridization conditions. For instance, two sequences are complementary when a section of a first sequence can bind to a section of a second sequence in an anti-parallel sense wherein the 3'-end of each sequence binds to the 5'-end of the other sequence and each A, T(U), G and C of one sequence is then aligned with a T(U), A, C and G, respectively, of the other sequence. RNA sequences can also include complementary G=U or U=G base pairs. Thus, two sequences need not have perfect homology to be "complementary" under the invention. Usually two sequences are sufficiently complementary when at least about 90% (preferably at least about 95%) of the nucleotides share base pair organization over a defined length of the molecule. The domains of the capture and surface probes thus contain a region of complementarity. Furthermore the capture domain of the capture probe contains a region of complementarity for the nucleic acid, e.g. RNA (preferably mRNA), of the biological specimen, e.g. tissue sample.

In a further representative embodiment, the capture probe may be synthesised on the solid substrate, e.g. array, using polymerase extension (similarly to as described above) and a terminal transferase enzyme to add a "tail" which may constitute the capture domain. The use of terminal transferases to add nucleotide sequences to the end of an oligonucleotide is known in the art, e.g. to introduce a homopolymeric tail e.g. a poly-T tail. Accordingly, in such a synthesis an oligonucleotide that corresponds to the cleavage domain of the capture probe (a cleavage domain oligonucleotide) may be contacted with the array and allowed to hybridize to the complementary domain of the surface probes. Excess oligonucleotides may be removed by washing the array under standard hybridization conditions. The resultant array comprises partially single stranded probes, wherein the 3' ends of the surface probes are double stranded and the complementary positional domain is single stranded. The array may be treated with a polymerase enzyme to extend the 3' end of the cleavage domain oligonucleotide, in a template dependent manner, so as to synthesize the positional domain (and optionally the amplification domain) of the capture probe. The capture domain, e.g. comprising a poly-T sequence may then be introduced using a terminal transferase to add a poly-T tail to generate the capture probe.

In yet another representative example, the capture probes may be synthesised on the solid substrate, e.g. array, using amplification techniques, such as bridge amplification. For example, a first plurality of nucleic acid primers is attached to the solid support, wherein the nucleic acid primers include a first universal primer sequence that is common to said first nucleic acid primers and a second plurality of nucleic acid primers is attached to the solid support, wherein the nucleic acid primers comprise a second universal primer sequence that is common to said second nucleic acid primers. The solid support comprising said first and second nucleic acid primers is contacted with a plurality of different probes comprising sequences that are complementary to capture domain, positional domain, cleavage domain and amplification domain (if present), a first universal primer binding sequence that hybridizes to the first universal primers on the solid support and a sequence identical to the second universal primer sequence. The probes hybridise to the first universal primers, which are extended using the nucleic acid probes as templates thereby generating a first plurality of extended nucleic acid molecules.

The first plurality of extended nucleic acid molecules comprise second universal primer binding sequences that hybridize to the second universal primers on the solid support, which are extended using the first plurality of extended nucleic acid molecules as templates thereby generating a second plurality of extended nucleic acid molecules that are identical to the probes contacted with the solid substrate. The second plurality of extended nucleic acid molecules comprise first universal primer binding sequences that hybridize to the first universal primers on the solid support and thus are able to template the extension of said first universal primers. The continued extension of the primers results in the formation of clusters on the surface of the solid substrate comprising nucleic acid molecules with sequences that are identical or complementary to the plurality of different probes contacted with the solid support.

The nucleic acid probes contacted with the solid substrate comprise a cleavage site or domain between the sequence that is identical to the second universal primer sequence and the sequence that is complementary to the capture domain. Cleavage of this cleavage site or domain results in the release of the domain comprising the second universal primer sequence. It will be evident that, in some embodiments, release of the domain comprising the second universal primer sequence may reveal the capture domain of the capture probes, and release the second plurality of extended nucleic acid molecules from the surface of the array. In some embodiments, release of the domain comprising the second universal primer sequence may reveal the positional domain or amplification domain of the capture probes, and release the second plurality of extended nucleic acid molecules from the surface of the array.

Thus, method described above results in random clusters of capture probes attached to the solid support which can be used for capturing nucleic acids from a biological specimen using method set forth previously herein. Advantageously, the released second plurality of extended nucleic acid molecules may be removed, e.g. by washing the solid substrate, prior to performing the method of the invention.

In this respect, it will be evident that the method described above may be modified to use a plurality of probes that comprise the capture domain, positional domain and cleavage domain, rather than their complements, e.g. by including the cleavage site or domain between the first universal primer binding sequence and the capture domain. Indeed the order of the domains of the probes contacted with the solid substrate will depend on the whether the capture probe is immobilized via its 3' end or 5' end and the skilled person readily could modify the order of the domain of the probes in order to generate an solid substrate, e.g. array, comprising capture probes suitable for use in the methods of the invention. Notably, the method of synthesizing the capture probes on the arrays using bridge amplification may be particularly useful in generating solid substrates in the form of flow cells, as described above.

As used herein, the term "random" can be used to refer to the spatial arrangement of the capture probes on the solid substrate, e.g. array. In this respect, there are at least two parameters relating to the spatial arrangement of the capture probes (i.e. features) on the solid substrate, e.g. array, described herein. The first parameter relates to the spacing and relative location of features and the second relates to identity or predetermined knowledge of the particular species of molecule that is present at a particular feature. Accordingly, in some embodiments, the features of an array can be randomly spaced such that adjacent features have variable spacing between each other. Alternatively, in some embodiments, the spacing between features can be ordered, for example, forming a regular pattern such as a rectilinear grid or hexagonal grid. In another respect, the features of an array can be random with respect to the identity or predetermined knowledge of the species of capture probe that occupies each feature independent of whether spacing produces a random pattern or ordered pattern. For example, in some embodiments set forth herein a solid substrate may be contacted with a population of nucleic acids under conditions where the nucleic acids attach at sites that are ordered with respect to their relative locations but 'randomly located' with respect to knowledge of the sequence for the nucleic acid species present at any particular site. Reference to "randomly distributing" nucleic acids at locations on a surface is intended to refer to the absence of knowledge or absence of predetermination regarding which nucleic acid will be captured at which location (regardless of whether the locations are arranged in an ordered pattern or not).

It will be evident that random arrays may be produced using any convenient means. However, in some embodiments, the random arrays are produced by amplifying capture probes on the surface of the solid substrate using primers that are attached to the surface of the solid substrate such that the location of the capture probes is not predetermined. In some embodiments, random arrays are produced using so-called bead arrays, wherein different capture probes are attached to different beads in the array, which are randomly distributed on the surface of the array such that each bead, and thus each species of capture probe, is represented only once on the solid substrate. In this respect, it will be evident that the beads may be arranged in an ordered spatial arrangement, e.g. wherein the solid substrate comprises wells in an ordered spatial arrangement having dimensions that accommodate no more than a single bead. However, such bead arrays may be viewed as random arrays insofar as there is an absence of knowledge or absence of predetermination regarding where each capture probe will be located on the solid substrate.

As the methods of the invention rely on the ability to correlate the sequence of an extended probe comprising a positional domain to a particular location on the solid substrate, it is evident that random arrays must be analysed prior to their use in the methods of the invention to determine where each species of capture probe (i.e. where each positional domain) is located on the solid substrate. This may be achieved by any suitable means known in the art, such as sequencing or using hybridisation probes.

Thus, in some embodiments, the method of the invention may comprise a step of performing a nucleic acid detection reaction on the solid substrate to determine the positional domain sequences of the randomly located probes on the solid substrate. For instance, the nucleic acid detection reaction may be a sequencing reaction to determine the positional domain sequences of the capture probes located randomly on the solid substrate. In some embodiments, the nucleic acid detection reaction may be a decoder probe hybridization reaction on the solid substrate to determine the positional domain sequences of the capture probes located randomly on the solid substrate. These steps may be performed prior to step (b) of the method of the invention.

By way of example, combinatorial hybridization methods, such as those used for decoding of multiplex bead arrays (see e.g. US Pat. No. 8,460,865), can be used to decode a "random" array. Such methods utilize labelled nucleic acid decoder probes that are complementary to at least a portion of the positional domain sequence (e.g. barcode sequence). A hybridization reaction can be carried out using decoder probes having known labels such that the location where the labels end up on the solid substrate identifies the nucleic acid probes according to rules of nucleic acid complementarity. In some cases, pools of many different probes with distinguishable labels are used, thereby allowing a multiplex decoding operation. The number of different barcodes determined in a decoding operation can exceed the number of labels used for the decoding operation. For example, decoding can be carried out in several stages where each stage constitutes hybridization with a different pool of decoder probes. The same decoder probes can be present in different pools but the label that is present on each decoder probe can differ from pool to pool (i.e. each decoder probe is in a different "state" when in different pools). Various combinations of these states and stages can be used to expand the number of positional domains (e.g. barcodes) that can be decoded well beyond the number of distinct labels available for decoding.

Sequencing techniques, such as sequencing-by-synthesis (SBS) techniques, are a particularly useful method for determining positional domain (e.g. barcode) sequences on random arrays. By way of example, SBS can be carried out as follows. To initiate a first SBS cycle, one or more labelled nucleotides, DNA polymerase, SBS primers etc., can be contacted with one or more features on the solid substrate (e.g. feature(s) where capture probes are attached to the solid support). Those features where SBS primer extension causes a labelled nucleotide to be incorporated can be detected. Optionally, the nucleotides can include a reversible termination moiety that terminates further primer extension once a nucleotide has been added to the SBS primer. For example, a nucleotide analogue having a reversible terminator moiety can be added to a primer such that subsequent extension cannot occur until a de-blocking agent is delivered to remove the moiety. Thus, for embodiments that use reversible termination, a de-blocking reagent can be delivered to the solid substrate (before or after detection occurs). Washes can be carried out between the various delivery steps. The cycle can then be repeated n times to extend the primer by n nucleotides, thereby detecting a sequence of length n. Exemplary SBS procedures, fluidic systems and detection platforms that can be readily adapted for use with the method of the present invention are described, for example, in WO 91/06678, WO 04/018497, WO 07/123744; US Pat. Nos. 7,057,026, 7,329,492, 7,211,414, 7,315,019 or 7,405,281; and US Pat. App. Publ. No. 2008/0108082.

Thus, in some embodiments, the capture probes may also comprise a domain comprising a sequencing primer binding site. In particular, the sequencing primer binding site may be located between the capture domain and positional domain of the capture probe. A domain comprising a sequencing primer binding site located between the capture domain and positional domain of the capture probe may be particularly useful for sequencing the capture probes on a random array as described above.

Thus, it will be evident from the discussion above that the capture probe may comprise one or more universal domains, i.e. domains that are common to each species of capture probe. In particular, the universal domain(s) may be primer binding sites useful in synthesising the capture probes on the solid substrate, e.g. array, and/or sequencing the capture probe on random arrays.

It will also be evident that the capture probe of the description may comprise two different cleavage sites or domains. For instance, a first cleavage domain may be located upstream (i.e. at the 5' end) of the positional domain, e.g. when the capture probe is immobilized by its 5' end. However, in some embodiments, the first cleavage domain may be located downstream (i.e. at the 3' end) of the capture domain, e.g. when the capture probe is immobilized by its 3' end. A second cleavage domain may be located between a first universal primer binding site used in the synthesis of the capture probe on the solid substrate, e.g. array, and the capture domain or complement thereof. The cleavage sites or domains must be reactive to different cleavage reactions (e.g. contain different cleavage enzyme recognition sites) such that each one can be selectively cleaved without necessarily cleaving the other. For instance, the second cleavage site or domain can be cleaved prior to step (c) (i.e. prior to releasing and extended the capture probe), thereby separating or removing the first universal primer binding site from the other domains of the capture probe that remain attached to a solid substrate. The first cleavage site or domain is cleaved after contacting the solid substrate with the biological specimen, i.e. simultaneously with the extension reaction.

A typical solid substrate, e.g. array, for use in the methods of the invention may contain multiple spots or "features". A feature may be defined as an area or distinct position on the array substrate at which a single species of capture probe is immobilized. Hence each feature will comprise a multiplicity of probe molecules, of the same species. It will be understood in this context that whilst it is encompassed that each capture probe of the same species may have the same sequence, this need not necessarily be the case. Each species of capture probe will have the same positional domain (i.e. each member of a species and hence each probe in a feature will be identically "tagged"), but the sequence of each member of the feature (species) may differ, because the sequence of a capture domain may differ. As described above, random or degenerate capture domains may be used or capture domains specific for a subset of genes may be used. Thus the capture probes within a feature may comprise different random or degenerate sequences or different gene specific sequences. The number and density of the features on the solid substrate, e.g. array, will determine the resolution of the array, i.e. the level of detail at which the transcriptome or genome of the tissue sample can be analysed. Hence, a higher density of features will typically increase the resolution of the array.

As discussed above, the size and number of the features on the array of the description will depend on the nature of the tissue sample and required resolution. Thus, if it is desirable to determine a transcriptome or genome only for regions of cells within a biological specimen, e.g. tissue sample, (or the sample contains large cells) then the number and/or density of features on the array may be reduced (i.e. lower than the possible maximum number of features) and/or the size of the features may be increased (i.e. the area of each feature may be greater than the smallest possible feature), e.g. an array comprising few large features. As mentioned above, in some embodiments, adjacent features may comprise the same capture probes, i.e. capture probes containing the same positional domain, in order to effectively increase the size of a feature, i.e. to lower the resolution of the array. Alternatively, if it is desirable to determine a transcriptome or genome of individual cells within a sample, it may be necessary to use the maximum number of features possible, which would necessitate using the smallest possible feature size, e.g. an array comprising many small features.

Whilst single cell resolution may be a preferred and advantageous feature of the present invention, it is not essential to achieve this, and resolution at the cell group level is also of interest, for example to detect or distinguish a particular cell type or tissue region, e.g. normal vs. tumour cells.

In representative embodiments of the invention, an array may contain at least 2, 5, 10, 50, 100, 500, 750, 1000, 1500, 3000, 5000, 10000, 20000, 40000, 50000, 75000, 100000, 150000, 200000, 300000, 400000, 500000, 750000, 800000, 1000000, 1200000, 1500000, 1750000, 2000000, 2100000, 3000000, 3500000, 4000000, 4500000, 5000000, 6000000, 7000000, 8000000, 9000000, 10000000, 12000000 or 15000000 features. As noted above, feature size and/or average distance between features (i.e. the pitch of the features) may be decreased and this may allow greater numbers of features to be accommodated within the same or a similar area. By way of example, these features may be comprised in an area of less than about 20cm², 10cm², 5cm², 1cm², 1mm², or 100µm².

It is well-established in the art that a solid substrate may comprise more than one "array", thereby enabling multiplex analysis of biological specimens on a single substrate. Thus, in some embodiments, the solid substrate comprises 2 or more arrays, e.g. 3, 4, 5, 6, 7, 8, 9, 10 or more arrays, e.g. 12, 15, 20, 25 or more arrays.

Thus, in some embodiments of the invention the area of each feature may be from about 0.1 µm², 0.5 µm², 1µm², 2 µM², 3 µm², 4 µm², 5 µm², 10 µm², 12 µm², 15 µm², 20 µm², 50 µm², 75 µm², 100 µm², 150 µm², 200 µm², 250 µm², 300 µm², 400 µm², or 500 µm².

As used herein, the term "pitch," when used in reference to features of an array, is intended to refer to the centre-to-centre spacing for adjacent features. A pattern of features can be characterized in terms of average pitch. The pattern can be ordered such that the coefficient of variation around the average pitch is small or the pattern can be random in which case the coefficient of variation can be relatively large. In either case, the average pitch can be, for example, at least about 10 nm, 0.1 µm, 0.5 µm, 1 µm, 5 µm, 10 µm, 100 µm or more. Alternatively or additionally, the average pitch can be, for example, at most about 100 µm, 10 µm, 5 µm, 1 µm, 0.5 µm 0.1 µm or less. Of course, the average pitch for a particular pattern of features can be between one of the lower values and one of the upper values selected from the ranges above.

The term "biological specimen" is intended to mean one or more cell, tissue, organism or portion thereof. It will be evident that a biological specimen from any organism could be used in the methods of the invention, e.g. plant, animal or fungal. The method of the invention allows the capture of any nucleic acid, e.g. mRNA, molecules, which are present in cells that are capable of transcription and/or translation. The method of the invention is particularly suitable for isolating and analysing the transcriptome or genome of cells within a biological specimen, e.g. a tissue sample, wherein spatial resolution of the transcriptomes or genomes is desirable, e.g. where the cells are interconnected or in contact directly with adjacent cells. However, it will be apparent to a person of skill in the art that the methods of the invention may also be useful for the analysis of the transcriptome or genome of different cells or cell types within a sample even if said cells do not interact directly, e.g. a blood sample. In other words, the cells do not need to present in the context of a tissue and can be applied to the array as single cells (e.g. cells isolated from a non-fixed tissue, e.g. a blood sample). Such single cells, whilst not necessarily fixed to a certain position in a tissue, are nonetheless applied to a certain position on the array and can be individually identified. Thus, in the context of analysing cells that do not interact directly, or are not present in a tissue context, the spatial properties of the described methods may be applied to obtaining or retrieving unique or independent transcriptome or genome information from individual cells.

The biological specimen may thus be a harvested or biopsied tissue sample, or possibly a cultured sample. Representative samples include clinical samples e.g. whole blood or blood-derived products, blood cells, tissues, biopsies, or cultured tissues or cells etc. including cell suspensions. Artificial tissues may for example be prepared from cell suspension (including for example blood cells). Cells may be captured in a matrix (for example a gel matrix e.g. agar, agarose, etc) and may then be sectioned in a conventional way. Such procedures are known in the art in the context of immunohistochemistry (see e.g. Andersson et al 2006, J. Histochem. Cytochem. 54(12): 1413-23. Epub 2006 Sep 6).

The mode of preparation of the biological specimen, e.g. tissue sample, and how the resulting sample is handled may affect the transcriptomic or genomic analysis of the methods of the invention. Moreover, various biological specimens, e.g. tissue samples, will have different physical characteristics and it is well within the skill of a person in the art to perform the necessary manipulations to yield a biological specimen, e.g. tissue sample, for use with the methods of the invention. However, it is evident from the disclosures herein that any method of sample preparation may be used to obtain a biological specimen, e.g. tissue sample, that is suitable for use in the methods of the invention. For instance any layer of cells with a thickness of approximately 1 cell or less may be used in the methods of the invention. In one embodiment, the thickness of the biological specimen, e.g. tissue sample, may be less than 0.9, 0.8, 0.7, 0.6, 0.5, 0.4, 0.3, 0.2 or 0.1 of the cross-section of a cell. However, since as noted above, the present invention is not limited to single cell resolution and hence it is not a requirement that the biological specimen, e.g. tissue sample, has a thickness of one cell diameter or less; thicker biological specimens, e.g. tissue samples, may if desired be used. For example cryostat sections may be used, which may be e.g. 10-20 µm thick.

The biological specimen, e.g. tissue sample, may be prepared in any convenient or desired way and the invention is not restricted to any particular type of tissue preparation. Fresh, frozen, fixed or unfixed tissues may be used. Any desired convenient procedure may be used for fixing or embedding the biological specimen, e.g. tissue sample, as described and known in the art. Thus any known fixatives or embedding materials may be used.

As a first representative example of a biological specimen, e.g. tissue sample for use in the invention, a tissue may prepared by deep freezing at temperature suitable to maintain or preserve the integrity (i.e. the physical characteristics) of the tissue structure, e.g. less than -20°C and preferably less than -25, -30, -40, -50, -60, -70 or -80 °C. The frozen tissue sample may be sectioned, i.e. thinly sliced, onto the solid substrate, e.g. array, surface by any suitable means. For example, a tissue sample may be prepared using a chilled microtome, a cryostat, set at a temperature suitable to maintain both the structural integrity of the tissue sample and the chemical properties of the nucleic acids in the sample, e.g. to less than -15°C and preferably less than -20 or -25°C. Thus, the sample should be treated so as to minimize the degeneration or degradation of the nucleic acid, e.g. RNA, in the tissue. Such conditions are well-established in the art and the extent of any degradation may be monitored through nucleic acid extraction, e.g. total RNA extraction, and subsequent quality analysis at various stages of the preparation of the tissue sample.

In a second representative example, a tissue may be prepared using standard methods of formalin-fixation and paraffin-embedding (FFPE), which are well-established in the art. Following fixation of a tissue sample and embedding in a paraffin or resin block, the tissue sample may sectioned, i.e. thinly sliced, onto the solid substrate, e.g. array. As noted above, other fixatives and/or embedding materials can be used.

It will be apparent that the biological specimen, e.g. tissue sample section, will need to be treated to remove the embedding material e.g. to deparaffinize, i.e. to remove the paraffin or resin, from the sample prior to carrying out the methods of the invention. This may be achieved by any suitable method and the removal of paraffin or resin or other material from tissue samples is well established in the art, e.g. by incubating the sample (on the surface of the solid substrate, e.g. array) in an appropriate solvent e.g. xylene, e.g. twice for 10 minutes, followed by an ethanol rinse, e.g. 99.5% ethanol for 2 minutes, 96% ethanol for 2 minutes, and 70% ethanol for 2 minutes.

It will be evident to the skilled person that the RNA in tissue sections prepared using methods of FFPE or other methods of fixing and embedding is more likely to be partially degraded than in the case of frozen tissue. However, without wishing to be bound by any particular theory, it is believed that this may be advantageous in the methods of the invention. For instance, if the RNA in the sample is partially degraded the average length of the RNA polynucleotides will be less and more randomized than a non-degraded sample. It is postulated therefore that partially degraded RNA would result in less bias in the various processing steps, described elsewhere herein, e.g. ligation of adaptors (amplification domains), amplification of the cDNA molecules and sequencing thereof.

Hence, in one embodiment of the invention the biological specimen, e.g. tissue sample, i.e. the section of the tissue sample contacted with the solid substrate, e.g. array, is prepared using FFPE or other methods of fixing and embedding. In other words the sample may be fixed, e.g. fixed and embedded. In an alternative embodiment of the invention the tissue sample is prepared by deep-freezing. In another embodiment a touch imprint of a tissue may be used, according to procedures known in the art. In other embodiments an unfixed sample may be used.

Whilst the biological specimen may be prepared for use in the methods of the invention using any suitable techniques or preparation methods, in some particularly preferred embodiments, the nucleic acid molecules of the specimen (particularly RNA molecules) are not modified prior to contact with the solid substrate (e.g. array). For instance, in some preferred embodiments the nucleic acid molecules (e.g. RNA) of the biological specimen are not treated to enhance or facilitate their interaction with the capture probes prior to contact with the solid substrate. In particular, in some embodiments the nucleic acid molecules (e.g. RNA) of the biological specimen are not contacted with or hybridized to nucleic acids (e.g. aptamers, oligonucleotides or nucleic acid tags) that facilitate the interaction between the nucleic acid molecules of the biological sample and capture probes) prior to contacting the biological specimen with the solid substrate. For instance, in some embodiments, the biological specimen is not contacted with or hybridized to nucleic acids that function as an intermediary between the target nucleic acid of the biological specimen and the capture probes, i.e. that function to indirectly attach or link the target nucleic acid of the biological specimen to the capture probe, prior to contacting the biological specimen with the substrate (e.g. array).

The thickness of the biological specimen, e.g. tissue sample section, for use in the methods of the invention may be dependent on the method used to prepare the sample and the physical characteristics of the tissue. Thus, any suitable section thickness may be used in the methods of the invention. In representative embodiments of the invention the thickness of the tissue sample section will be at least 0.1µm, further preferably at least 0.2, 0.3, 0.4, 0.5, 0.7, 1.0, 1.5, 2, 3, 4, 5, 6, 7, 8, 9 or 10µm. In other embodiments the thickness of the tissue sample section is at least 10, 12, 13, 14, 15, 20, 30, 40 or 50µm. However, the thickness is not critical and these are representative values only. Thicker samples may be used if desired or convenient e.g. 70 or 100 µm or more. Typically, the thickness of the tissue sample section is between 1-100 µm, 1-50 µm , 1-30 µm, 1-25 µm, 1-20 µm, 1-15 µm, 1-10 µm, 2-8µm, 3-7µm or 4-6µm, but as mentioned above thicker samples may be used.

On contact of the biological specimen, e.g. tissue sample section, with the solid substrate, e.g. following removal of the embedding material, e.g. deparrafinization, the nucleic acid, e.g. RNA, molecules in the biological specimen, e.g. tissue sample, will bind to the immobilized capture probes on the array. In some embodiments it may be advantageous to facilitate the hybridization of the nucleic acid, e.g. RNA, molecules to the capture probes. Typically, facilitating the hybridization comprises modifying the conditions under which hybridization occurs. The primary conditions that can be modified are the time and temperature of the incubation of the biological specimen, e.g. tissue sample section, on the array prior to the reverse transcription step, which is described elsewhere herein.

It will be evident that biological specimens, e.g. tissue samples, from different sources may require different treatments to allow the nucleic acids to interact with, i.e. hybridize to, the capture probes. For instance, it may be useful to permeabilize the biological specimen, e.g. tissue sample, to facilitate the interaction between the nucleic acid and the capture probes. If the tissue sample is not permeabilized sufficiently the amount of nucleic acid captured by the capture probes may be too low to enable further analysis. Conversely, if the biological specimen, e.g. tissue sample, is too permeable, the nucleic acid may diffuse away from its origin in the biological specimen, e.g. tissue sample, i.e. the nucleic acid may be captured by the capture probes may not correlate accurately with its original spatial distribution in the biological specimen, e.g. tissue sample. Hence, there must be a balance between permeabilizing the biological specimen, e.g. tissue sample, enough to obtain enable efficient interaction between the nucleic acids and the capture probes whilst maintaining the spatial resolution of the nucleic acid distribution in the biological specimen, e.g. tissue sample. The methods used to fix the biological specimen, e.g. tissue sample, may also impact on the interaction between the nucleic acid of the biological specimen, e.g. tissue sample, and the capture probes.

Thus, in some embodiments, the method further comprises a step of permeabilizing the biological specimen. This step may be performed after the step of contacting the specimen with the solid substrate. In some embodiments, the step of permeabilizing the biological specimen may take place before or contemporaneously with the step of releasing and extending the capture probes.

Suitable methods and agents for permeabilizing and/or fixing biological specimens, e.g. cells and tissues, are well known in the art and any appropriate method may be selected for use in the methods of the invention. Some proteases are particularly useful in permeabilizing cells in a biological specimen, e.g. pepsin. Particularly useful fixatives include, e.g. methanol.

As conditions for spatial tagging, e.g. localised or spatial detection, of nucleic acid molecules from a biological specimen, e.g. tissue sample, on a solid substrate vary depending on the biological specimen, e.g. tissue sample, a typical range of parameters is discussed herein. For instance, on contacting a biological specimen, e.g. tissue sample section, with the solid substrate, e.g. array, the substrate may be incubated for at least 1 hour to allow the nucleic acid, e.g. RNA, to hybridize to the capture probes. Preferably the solid substrate, e.g. array, may be incubated for at least 2, 3, 5, 10, 12, 15, 20, 22 or 24 hours or until the tissue sample section has dried. The substrate incubation time is not critical and any convenient or desired time may be used. Typical substrate, e.g. array, incubations may be up to 72 hours. Thus, the incubation may occur at any suitable temperature, for instance at room temperature, although in a preferred embodiment the biological specimen, e.g. tissue sample section, is incubated on the substrate, e.g. array, at a temperature of at least 25, 26, 27, 28, 29, 30, 31, 32, 33, 34, 35, 36 or 37°C. Incubation temperatures of up to 55°C are commonplace in the art. In a particularly preferred embodiment the tissue sample section is allowed to dry on the array at 37°C for 24 hours. Once the biological specimen, e.g. tissue sample section, has dried the substrate, e.g. array, may be stored at room temperature before performing the combined cleavage and extension, e.g. reverse transcription, step. It will be understood that the if the biological specimen, e.g. tissue sample section, is allowed to dry on the surface of the substrate, e.g. array, it will need to be rehydrated before further manipulation of the captured nucleic acid can be achieved, e.g. the step of cleaving the capture probes from the solid substrate and simultaneously or subsequently reverse transcribing the captured RNA.

Hence, the method of the invention may comprise a further step of rehydrating the biological specimen, e.g. tissue sample, after contacting the specimen with the solid substrate, e.g. array.

In some embodiments it may be advantageous to block (e.g. mask or modify) the capture probes prior to contacting the biological specimen, e.g. tissue sample, with the substrate, e.g. array, particularly when the nucleic acid in the biological specimen, e.g. tissue sample, is subject to a process of modification prior to its capture on the array. Specifically, it may be advantageous to block or modify the free 3' end of the capture probe. In a particular embodiment, the nucleic acid in the biological specimen, e.g. tissue sample, e.g. fragmented genomic DNA, may be modified such that it can be captured by the capture probe. For instance, and as described in more detail below, an adaptor sequence (comprising a binding domain capable of binding to the capture domain of the capture probe) may be added to the end of the nucleic acid, e.g. fragmented genomic DNA. This may be achieved by, e.g. ligation of an adaptor or extension of the nucleic acid, e.g. using an enzyme to incorporate additional nucleotides at the end of the sequence, e.g. a poly-A tail. It is necessary to block or modify the capture probes, particularly the free 3' end of the capture probe, prior to contacting the biological specimen, e.g. tissue sample, with the array to avoid modification of the capture probes, e.g. to avoid the addition of a poly-A tail to the free 3' end of the capture probes. Preferably the incorporation of a blocking domain may be incorporated into the capture probe when it is synthesised. However, the blocking domain may be incorporated to the capture probe after its synthesis.

In some embodiments the capture probes may be blocked by any suitable and reversible means that would prevent modification of the capture domains during the process of modifying the nucleic acid of the biological specimen, e.g. tissue sample, which occurs after the biological specimen, e.g. tissue sample, has been contacted with the array. In other words, the capture probes may be reversibly masked or modified such that the capture domain of the capture probe does not comprise a free 3' end, i.e. such that the 3' end is removed or modified, or made inaccessible so that the capture probe is not susceptible to the process which is used to modify the nucleic acid of the biological specimen, e.g. ligation or extension, or the additional nucleotides may be removed to reveal and/or restore the 3' end of the capture domain of the capture probe.

For example, blocking probes may be hybridised to the capture probes to mask the free 3' end of the capture domain, e.g. hairpin probes or partially double stranded probes, suitable examples of which are known in the art. The free 3' end of the capture domain may be blocked by chemical modification, e.g. addition of an azidomethyl group as a chemically reversible capping moiety, such that the capture probes do not comprise a free 3' end. Suitable alternative capping moieties are well known in the art, e.g. the terminal nucleotide of the capture domain could be a reversible terminator nucleotide, which could be included in the capture probe during or after probe synthesis.

Alternatively or additionally, the capture domain of the capture probe could be modified so as to allow the removal of any modifications of the capture probe, e.g. additional nucleotides, that occur when the nucleic acid molecules of the biological specimen, e.g. tissue sample, are modified. For instance, the capture probes may comprise an additional sequence downstream of the capture domain, i.e. 3' to capture domain, namely a blocking domain. This could be in the form of a cleavage domain as defined above, e.g. a restriction endonuclease recognition sequence or a sequence of nucleotides cleavable by specific enzyme activities, e.g. uracil. Following the modification of the nucleic acid of the biological specimen, e.g. tissue sample, the capture probes could be subjected to an enzymatic cleavage, which would allow the removal of the blocking domain and any of the additional nucleotides that are added to the 3' end of the capture probe during the modification process. The removal of the blocking domain would reveal and/or restore the free 3' end of the capture domain of the capture probe. The blocking domain could be synthesised as part of the capture probe (e.g. as described above with respect to synthesising the probes on the solid substrate using bridge amplification) or could be added to the capture probe *in situ* (i.e. as a modification of an existing array), e.g. by ligation of the blocking domain.

The capture probes may be blocked using any combination of the blocking mechanisms described above. In this respect, it will be evident that the capture domain may be "blocked" by immobilizing the capture probes by their 3' end.

Once the nucleic acid of the biological specimen, e.g. tissue sample, e.g. fragmented genomic DNA, has been modified to enable it to hybridise to the capture domain of the capture probe, the capture probe must be unblocked, e.g. by dissociation of the blocking oligonucleotide, removal of the capping moiety and/or blocking domain.

In order to correlate the sequence analysis or transcriptome or genome information obtained from each feature of the array with the region (i.e. an area or cell) of the biological specimen, e.g. tissue sample, the biological specimen is oriented in relation to the features on the array. In other words, the tissue sample is placed on the array such that the position of a capture probe on the array may be correlated with a position in the biological specimen, e.g. tissue sample. Thus it may be identified where in the biological specimen, e.g. tissue sample, the position of each species of capture probe (or each feature of the array) corresponds. In other words, it may be identified to which location in the biological specimen, e.g. tissue sample, the position of each species of capture probe corresponds. For instance, this may be done by virtue of positional markers present on the solid substrate, e.g. array, as described below.

Conveniently, but not necessarily, the biological specimen, e.g. tissue sample, may be imaged following its contact with the array. This may be performed before or after the nucleic acid of the biological specimen, e.g. tissue sample, is processed, e.g. before or after the combined release and extension step. In some embodiments the biological specimen, e.g. tissue sample, is imaged prior to the combined release and extension of the capture probes from the solid substrate, e.g. array. In a particularly preferred embodiment the biological specimen, e.g. tissue sample, is imaged after the combined release and extension of the capture probes from the solid substrate, e.g. array. It is not necessary to remove any residual tissue from the solid substrate, e.g. array, prior to the pooling, i.e. collecting, the released molecules (extended probes), from the substrate as the extended probes are capable of diffusing through the residue tissue. Furthermore, the step of processing the captured nucleic acid, e.g. combined release and extension of the capture probes, may act to remove some tissue from the array surface, e.g. when using tissue preparing by deep-freezing. In such a case, it may be necessary to separate residual tissue collected when pooling the extended probes from the solid substrate, if performed, prior to the step of analysing the extended probes. This may be achieved by any suitable means, e.g. centrifugation.

Thus, in some embodiments, the method may comprise a step of separating tissue from the pooled extended probes.

When the step of processing the capture nucleic acid acts to remove some tissue from the array surface, imaging of the biological specimen, e.g. tissue sample, may take place prior to the processing step, e.g. the combined release and extension step. Generally speaking, imaging may take place at any time after contacting the biological specimen, e.g. tissue sample, with the solid substrate, but before any step which degrades or removes the biological specimen, e.g. tissue sample. As noted above, this may depend on the biological specimen, e.g. tissue sample.

Advantageously, the solid substrate, e.g. array, may comprise markers to facilitate the orientation of the biological specimen, e.g. tissue sample, or the image thereof in relation to the features of the solid substrate, e.g. array. Any suitable means for marking the solid substrate, e.g. array, may be used such that they are detectable when the biological specimen, e.g. tissue sample, is imaged. For instance, a molecule, e.g. a fluorescent molecule, that generates a signal, preferably a visible signal, may be immobilized directly or indirectly on the surface of the substrate, e.g. array. Preferably, the substrate, e.g. array, comprises at least two markers in distinct positions on the surface of the array, further preferably at least 3, 4, 5, 6, 7, 8, 9, 10, 12, 15, 20, 30, 40, 50, 60, 70, 80, 90 or 100 markers. Conveniently several hundred or even several thousand markers may be used. The markers may be provided in a pattern, for example make up an outer edge of the array, e.g. an entire outer row of the features of an array. Other informative patterns may be used, e.g. lines sectioning the array. This may facilitate aligning an image of the biological specimen, e.g. tissue sample, to an array, or indeed generally in correlating the features of the array to the biological specimen, e.g. tissue sample. Thus, the marker may be an immobilized molecule to which a signal giving molecule may interact to generate a signal.

In a representative example, the solid substrate, e.g. array, may comprise a marker feature, e.g. a nucleic acid probe immobilized on the substrate, e.g. array, to which a labelled nucleic acid may hybridize. For instance, the labelled nucleic acid molecule, or marker nucleic acid, may be linked or coupled to a chemical moiety capable of fluorescing when subjected to light of a specific wavelength (or range of wavelengths), i.e. excited. Such a marker nucleic acid molecule may be contacted with the substrate, e.g. array, before, contemporaneously with or after the biological specimen, e.g. tissue sample, is stained in order to visualize or image the biological specimen, e.g. tissue sample. However, the marker must be detectable when the biological specimen, e.g. tissue sample is imaged. Thus, in a preferred embodiment the marker may be detected using the same imaging conditions used to visualize the biological specimen, e.g. tissue sample.

In a particularly preferred embodiment of the invention, the substrate, e.g. array, comprises marker features to which a labelled, preferably fluorescently labelled, marker nucleic acid molecule, e.g. oligonucleotide, is hybridized.

It will be evident however, that the use of positional markers is not essential for correlating the sequence analysis or transcriptome or genome information obtained from each feature of the array with the region (i.e. an area or cell) of the biological specimen, i.e. for aligning the biological specimen with the features of the array. In this respect, it may be possible to align the topology of the biological specimen to the data generated from analysing the extended probes. For instance, a crack in a tissue sample section would correspond to the absence of extended probes from particular features on the array and this may be sufficient to align the biological specimen with the features on the array. Furthermore, in some embodiments, the features on the surface of the solid substrate are visible, particularly following staining of the biological specimen, using high resolution imaging, thereby enabling the biological specimen to be aligned with the features on the array. Thus, in some embodiments, the feature of the array may be viewed as positional markers.

The step of imaging the biological specimen, e.g. tissue sample, may use any convenient histological means known in the art, e.g. light, bright field, dark field, phase contrast, fluorescence, reflection, interference, confocal microscopy or a combination thereof. Typically the biological specimen, e.g. tissue sample, is stained prior to visualization to provide contrast between the different regions, e.g. cells, of the biological specimen, e.g. tissue sample. The type of stain used will be dependent on the type of biological specimen, e.g. tissue, and the region of the cells to be stained. Such staining protocols are known in the art. In some embodiments more than one stain may be used to visualize (image) different aspects of the biological specimen, e.g. tissue sample, e.g. different regions of the tissue sample, specific cell structures (e.g. organelles) or different cell types. In other embodiments, the biological specimen, e.g. tissue sample, may be visualized or imaged without staining the sample, e.g. if the tissue sample contains already pigments that provide sufficient contrast or if particular forms of microscopy are used.

In a preferred embodiment, the tissue sample is visualized or imaged using fluorescence microscopy.

In some embodiments, extending the capture probe comprises generating cDNA from the captured (hybridized) RNA. It will be understood that this refers to the synthesis of a complementary strand of the hybridized nucleic acid, e.g. generating cDNA based on the captured RNA template (the RNA hybridized to the capture domain of the capture probe). Thus, in an initial step of extending the capture probe, e.g. the cDNA generation, the captured (hybridized) nucleic acid, e.g. RNA, acts as a template for the extension, e.g. reverse transcription, step.

Reverse transcription concerns the step of synthesizing cDNA (complementary or copy DNA) from RNA, preferably mRNA (messenger RNA), by reverse transcriptase. Thus cDNA can be considered to be a copy of the RNA present in a cell at the time at which the biological specimen, e.g. tissue sample, was taken, i.e. it represents all or some of the genes that were expressed in said cell at the time of isolation.

The capture probe, specifically the capture domain of the capture probe, acts as a primer for producing the complementary strand of the nucleic acid hybridized to the capture probe, e.g. a primer for reverse transcription. Hence, the nucleic acid, e.g. cDNA, molecules generated by the extension reaction, e.g. reverse transcription reaction, incorporate the sequence of the capture probe, i.e. the extension reaction, e.g. reverse transcription reaction, may be seen as a way of labelling or tagging indirectly the nucleic acid, e.g. transcripts, of the biological specimen, e.g. tissue sample, that are in contact with each feature of the array or within the vicinity of the feature such that the released capture probes are able to bind to the transcripts in a localised manner. As mentioned above, each species of capture probe comprises a positional domain (feature identification tag) that represents a unique sequence for each feature (or group of adjacent features) of the array. Thus, all of the nucleic acid, e.g. cDNA, molecules synthesized at a specific feature (or in the vicinity of a specific feature) will comprise the same nucleic acid "tag".

The nucleic acid, e.g. cDNA, molecules synthesized at each feature (or in the vicinity of a specific feature) of the array may represent the genome of, or genes expressed from, the region or area of the biological specimen, e.g. tissue sample, in contact with that feature (or in the vicinity of that feature), e.g. a tissue or cell type or group or sub-group thereof, and may further represent genes expressed under specific conditions, e.g. at a particular time, in a specific environment, at a stage of development or in response to stimulus etc. Hence, the cDNA at any single feature (i.e. captured by a specific species of capture probe) may represent the genes expressed in a single cell, or if the feature is in contact with (or in the vicinity of) the sample at a cell junction, the cDNA may represent the genes expressed in more than one cell. Similarly, if a single cell is in contact with (or in the vicinity of) multiple features, then each feature (i.e. a specific species of extended capture probe) may represent a proportion of the genes expressed in said cell. Similarly, in embodiments in which the captured nucleic acid is DNA, any single feature (i.e. a specific species of extended capture probe) may be representative of the genome of a single cell or more than one cell. Alternatively, the genome of a single cell may be represented by multiple features (i.e. multiple species of extended capture probe).

The extension of the capture probe, e.g. reverse transcription reaction, may be performed using any suitable enzymes and protocol of which many exist in the art, as described herein. However, it will be evident that it is not necessary to provide a primer for the synthesis of the first nucleic acid, e.g. cDNA, strand because the capture domain of the capture probe acts as the primer, e.g. reverse transcription primer.

Preferably, in the context of the present invention the extended probes, e.g. cDNA, are treated to comprise double stranded DNA. Treatment of the extended probes to produce double stranded DNA may be achieved in a single reaction to generate only a second DNA, e.g. cDNA, strand, i.e. to produce double stranded DNA molecules without increasing the number of double stranded DNA molecules, or in an amplification reaction to generate multiple copies of the second strand, which may be in the form of single stranded DNA (e.g. linear amplification) or double stranded DNA, e.g. cDNA (e.g. exponential amplification).

The step of second strand DNA, e.g. cDNA, synthesis may take place on the solid substrate, e.g. array, (i.e. prior to pooling the extended molecules), either as a discrete step of second strand synthesis, for example using random primers as described in more detail below, or in the initial step of an amplification reaction. Alternatively, the first strand DNA (i.e. extended probes), e.g. cDNA (the strand comprising, i.e. incorporating, the capture probe) may be pooled (e.g. collected) and second strand synthesis, whether as a discrete step or in an amplification reaction may occur subsequently, e.g. in a reaction carried out in a separate vessel or container, e.g. PCR tube.

Where second strand synthesis takes place on the solid substrate, e.g. array, the method may include an optional step of removing the captured nucleic acid, e.g. RNA, before the second strand synthesis, for example using an RNA digesting enzyme (RNase), e.g. RNase H. Procedures for this are well known and described in the art. However, this is generally not necessary, and in most cases the RNA degrades naturally. RNase H can be used if desired to increase the robustness of RNA removal.

For instance, in biological specimens, e.g. tissue samples, that comprise large amounts of RNA, the step of generating the double stranded cDNA may yield a sufficient amount of cDNA that it may be sequenced directly (following the step of pooling the extended probes). In this case, second strand cDNA synthesis may be achieved by any means known in the art and as described below. The second strand synthesis reaction may be performed on the solid substrate, i.e. prior to pooling the extended probes (e.g. transferring the extended probes to a separate vessel or container, e.g. PCR tube), or after the extended probes, e.g. cDNA, have been pooled.

In other embodiments it may be necessary to enhance, i.e. amplify, the extended probes, e.g. synthesized cDNA, to yield quantities that are sufficient for analysis, e.g. DNA sequencing. In this embodiment, the first strand of the extended probes, e.g. cDNA molecules, which comprise also the capture probe of the features of the array, acts as a template for the amplification reaction, e.g. a polymerase chain reaction. The first reaction product of the amplification will be a second strand of DNA, e.g. cDNA, which itself will act as a template for further cycles of the amplification reaction.

In either of the above described embodiments, the second strand of DNA, e.g. cDNA, will comprise a complement of the capture probe. If the capture probe comprises an amplification domain, then this may be used for the subsequent amplification and sequence analysis of the DNA, e.g. cDNA, e.g. the amplification reaction may comprise a primer with the same sequence as the amplification domain, i.e. a primer that is complementary (i.e. hybridizes) to the complement of the amplification domain. In view of the fact that the amplification domain is upstream of the positional domain of the capture probe (in the extended probe, e.g. the first cDNA strand), the complement of the positional domain will be incorporated in the second strand of the DNA, e.g. cDNA molecules.

In embodiments where the second strand of DNA, e.g. cDNA, is generated in a single reaction, the second strand synthesis may be achieved by any suitable means. For instance, the first strand cDNA (i.e. extended probe) may be incubated with random primers, e.g. hexamer primers, and a DNA polymerase, preferably a strand displacement polymerase, e.g. klenow (exo), under conditions sufficient for templated DNA synthesis to occur. This process will yield double stranded cDNA molecules of varying lengths and is unlikely to yield full-length cDNA molecules, i.e. cDNA molecules that correspond to entire mRNA from which they were synthesized. The random primers will hybridise to the first strand cDNA molecules (i.e. extended probes) at a random position, i.e. within the sequence rather than at the end of the sequence.

If it is desirable to generate full-length DNA, e.g. cDNA, molecules, i.e. molecules that correspond to the whole of the captured nucleic acid, e.g. RNA, molecule (if the nucleic acid, e.g. RNA, was partially degraded in the tissue sample then the captured nucleic acid, e.g. RNA, molecules will not be "full-length" transcripts or the same length as the initial fragments of genomic DNA), then the 3' end of the extended probes, e.g. first stand cDNA, molecules may be modified. For example, a linker or adaptor may be ligated to the 3' end of the cDNA molecules. This may be achieved using single stranded ligation enzymes such as T4 RNA ligase or Circligase^{™} (Epicentre Biotechnologies).

Alternatively, a second strand synthesis helper probe (a partially double stranded DNA molecule capable of hybridising to the 3' end of the first strand cDNA molecule), may be ligated to the 3' end of the extended probe, e.g. first strand cDNA, molecule using a double stranded ligation enzyme such as T4 DNA ligase. Other enzymes appropriate for the ligation step are known in the art and include, e.g. Tth DNA ligase, Taq DNA ligase, *Thermococcus* sp. (strain 9°N) DNA ligase (9°N^{™} DNA ligase, New England Biolabs), and Ampligase^{™} (Epicentre Biotechnologies). The second strand synthesis helper probe comprises also a specific sequence from which the second strand DNA, e.g. cDNA, synthesis may be primed using a primer that is complementary to the part of the helper probe that is ligated to the extended probe, e.g. first cDNA strand. A further alternative comprises the use of a terminal transferase active enzyme to incorporate a polynucleotide tail, e.g. a poly-A tail, at the 3' end of the extended probe, e.g. first strand of cDNA, molecules. The second strand synthesis may be primed using a poly-T primer, which may also comprise a specific amplification domain for further amplification. Other methods for generating "full-length" double stranded DNA, e.g. cDNA, molecules (or maximal length second strand synthesis) are well-established in the art.

In some embodiments, second strand synthesis may use a method of template switching, e.g. using the SMART^{™} technology from Clontech^{®}. SMART (Switching Mechanism at 5' End of RNA Template) technology is well established in the art and is based that the discovery that reverse transcriptase enzymes, e.g. Superscript^{®} II (Invitrogen), are capable of adding a few nucleotides at the 3' end of an extended cDNA molecule, i.e. to produce a DNA/RNA hybrid with a single stranded DNA overhang at the 3' end. The DNA overhang may provide a target sequence to which an oligonucleotide probe can hybridise to provide an additional template for further extension of the cDNA molecule. Advantageously, the oligonucleotide probe that hybridises to the cDNA overhang contains an amplification domain sequence, the complement of which is incorporated into the synthesised first strand cDNA product. Primers containing the amplification domain sequence, which will hybridise to the complementary amplification domain sequence incorporated into the cDNA first strand, can be added to the reaction mix to prime second strand synthesis using a suitable polymerase enzyme and the cDNA first strand as a template. This method avoids the need to ligate adaptors to the 3' end of the cDNA first strand. Whilst template switching was originally developed for full-length mRNAs, which have a 5' cap structure, it has since been demonstrated to work equally well with truncated mRNAs without the cap structure. Thus, template switching may be used in the methods of the invention to generate full length and/or partial or truncated cDNA molecules. Thus, in a preferred embodiment of the invention, the second strand synthesis may utilise, or be achieved by, template switching. In a particularly preferred embodiment, the template switching reaction, i.e. the further extension of the cDNA first strand to incorporate the complementary amplification domain, is performed on the solid substrate (i.e. prior to pooling the extended probes). Preferably, the second strand synthesis reaction is also performed in solution on the solid substrate.

In some embodiments it may be advantageous to treat the double stranded extended probes to remove any unextended capture probes prior to amplification and/or analysis, e.g. sequence analysis. This may be achieved by any suitable means known in the art, e.g. using an enzyme to degrade the unextended probes, e.g. an exonuclease enzyme, or purification columns. Thus, in some embodiments, the method further comprises a step of treating the extended probes to removed unextended capture probes. This may be, for example, after the step of pooling the extended probes.

In embodiments where it may be necessary or advantageous to enhance, enrich or amplify the DNA, e.g. cDNA molecules, amplification domains may be incorporated in the DNA, e.g. cDNA molecules. As discussed above, a first amplification domain may be incorporated into the extended probes, e.g. the first strand of the cDNA molecules, when the capture probe comprises an amplification domain. In these embodiments, the second strand synthesis may incorporate a second amplification domain. For example, the primers used to generate the second strand cDNA, e.g. random hexamer primers, poly-T primer, the primer that is complementary to the second strand synthesis helper probe, may comprise at their 5' end an amplification domain, i.e. a nucleotide sequence to which an amplification primer may hybridize. Thus, the resultant double stranded DNA may comprise an amplification domain at or towards each 5' end of the double stranded DNA, e.g. cDNA molecules. These amplification domains may be used as targets for primers used in an amplification reaction, e.g. PCR. Alternatively, the linker or adaptor which is ligated to the 3' end of the extended probes, e.g. first strand cDNA molecules, may comprise a second amplification domain. Similarly, a second amplification domain may be incorporated into the first strand cDNA molecules by template switching.

In embodiments where the capture probe does not comprise an amplification domain, the second strand of the cDNA molecules may be synthesised in accordance with the above description. The resultant double stranded DNA molecules may be modified to incorporate an amplification domain at the 5' end of the first DNA, e.g. cDNA strand (a first amplification domain) and, if not incorporated in the second strand DNA, e.g. cDNA synthesis step, at the 5' end of the second DNA, e.g. cDNA strand (a second amplification domain). Such amplification domains may be incorporated, e.g. by ligating double stranded adaptors to the ends of the DNA, e.g. cDNA molecules. Enzymes appropriate for the ligation step are known in the art and include, e.g. Tth DNA ligase, Taq DNA ligase, *Thermococcus* sp. (strain 9°N) DNA ligase (9°N^{™} DNA ligase, New England Biolabs), Ampligase^{™} (Epicentre Biotechnologies) and T4 DNA ligase. In a preferred embodiment the first and second amplification domains comprise different sequences.

From the above, it is therefore apparent that amplification domains may be added to the extended probes, i.e. DNA molecules, for example to the cDNA molecules, or their complements (e.g. second strand) by various methods and techniques and combinations of such techniques known in the art, e.g. by use of primers which include such a domain, ligation of adaptors, use of terminal transferase enzymes and/or by template switching methods. As is clear from the discussion herein, such domains may be added before or after the extended probes are pooled, i.e. transferred to a separate vessel or container, e.g. PCR tube.

It will be apparent from the above description that all of the DNA, e.g. cDNA molecules from a single solid substrate, e.g. array, that have been synthesized by the methods of the invention may all comprise the same first and second amplification domains. Consequently, a single amplification reaction, e.g. PCR, may be sufficient to amplify all of the DNA, e.g. cDNA molecules. Thus in a preferred embodiment, the method of the invention may comprise a step of amplifying the DNA, e.g. cDNA molecules. In one embodiment the amplification step is performed after the extended probes, e.g. cDNA molecules, have been pooled. In other embodiments amplification may be performed before the extended probes are pooled, i.e. on the solid substrate, e.g. array. It is known in the art that amplification reactions may be carried out on solid substrates, such as arrays, by forming wells on the surface of the substrate to contain the solution or by immersing the substrate in a solution. Indeed, on-chip thermocyclers exist for carrying out such reactions. Thus, in one embodiment arrays which are known in the art as sequencing platforms or for use in any form of sequence analysis (e.g. in or by next generation sequencing technologies) may be used as the basis of the arrays of the present description (e.g. Illumina bead arrays, flow cells etc. as discussed above)

For the synthesis of the second strand of DNA, e.g. cDNA, it is preferable to use a strand displacement polymerase (e.g. Φ29 DNA polymerase, Bst (exo⁻) DNA polymerase, klenow (exo⁻) DNA polymerase) if the extended probes comprise a partially double stranded nucleic acid molecule. For instance, the extended probes may be at least partially double stranded (e.g. DNA:DNA, DNA:RNA or DNA:DNA/RNA hybrid) in embodiments where the capture probe is immobilized indirectly on the substrate of the array via a surface probe. The strand displacement polymerase is necessary to ensure that the second cDNA strand synthesis incorporates the complement of the positional domain (feature identification domain) into the second DNA, e.g. cDNA strand.

As mentioned above, the method of the invention may be seen to comprise a step of pooling, e.g. collecting or recovering, the extended probes, e.g. cDNA molecules (including double stranded cDNA molecules), and/or their amplicons. As noted above, in the context of amplification on the solid substrate, the extended probes may include amplicons of the extended probes, e.g. cDNA.

The extended probes, i.e. DNA, e.g. cDNA molecules, or amplicons, that have been pooled, which may have been modified as discussed above, are analysed to investigate (e.g. determine their sequence, although as noted above actual sequence determination is not required - any method of analysing the sequence may be used). Thus, any method of nucleic acid analysis may be used. The step of sequence analysis may identify the positional domain and hence allow the analysed molecule to be localised to a position in the biological specimen, e.g. tissue sample. Similarly, the nature or identity of the analysed molecule may be determined. In this way the nucleic acid, e.g. RNA, at given position in the array, and hence in the biological specimen, e.g. tissue sample, may be determined. Hence the analysis step may include or use any method which identifies the analysed molecule (and hence the "target" molecule) and its positional domain. Generally such a method will be a sequence-specific method. For example, the method may use sequence-specific primers or probes, particularly primers or probes specific for the positional domain and/or for a specific nucleic acid molecule to be detected or analysed e.g. a DNA molecule corresponding to a nucleic acid, e.g. RNA or cDNA molecule to be detected. Typically in such a method sequence-specific amplification primers, e.g. PCR primers may be used.

In some embodiments it may be desirable to analyse a subset or family of target related molecules, e.g. all of the sequences that encode a particular group of proteins which share sequence similarity and/or conserved domains, e.g. a family of receptors. Hence, the amplification and/or analysis methods described herein may use degenerate or gene family specific primers or probes that hybridise to a subset of the extended probes or nucleic acids derived therefrom, e.g. amplicons. In a particularly preferred embodiment, the amplification and/or analysis methods may utilise a universal primer (i.e. a primer common to all of the captured sequences) in combination with a degenerate or gene family specific primer specific for a subset of target molecules.

Thus in one embodiment, amplification-based, especially PCR-based methods of sequence analysis are used.

However, the steps of modifying and/or amplifying the extended probes, e.g. cDNA molecules, may introduce additional components into the sample, e.g. enzymes, primers, nucleotides etc. Hence, the methods of the invention may further comprise a step of purifying the sample comprising the extended probes, e.g. cDNA molecules, or amplicons thereof prior to the sequence analysis, e.g. to remove oligonucleotide primers, nucleotides, salts etc. that may interfere with the sequencing reactions. Any suitable method of purifying the extended probes, i.e. DNA, e.g. cDNA molecules, may be used.

Sequence analysis of the released DNA molecules may be direct or indirect. Thus the sequence analysis substrate (which may be viewed as the molecule which is subjected to the sequence analysis step or process) may directly be the extended probe or it may be a molecule which is derived therefrom. Thus, for example in the context of sequence analysis step which involves a sequencing reaction, the sequencing template may be the extended probe or it may be a molecule derived therefrom. For example, a first and/or second strand DNA, e.g. cDNA molecule, may be directly subjected to sequence analysis (e.g. sequencing), i.e. may directly take part in the sequence analysis reaction or process (e.g. the sequencing reaction or sequencing process, or be the molecule which is sequenced or otherwise identified). Alternatively, the extended probe may be subjected to a step of second strand synthesis or amplification before sequence analysis (e.g. sequencing or identification by other means). The sequence analysis substrate (e.g. template) may thus be an amplicon or a second strand of an extended probe.

Both strands of a double stranded molecule may be subjected to sequence analysis (e.g. sequenced) but the invention is not limited to this and single stranded molecules (e.g. cDNA) may be analysed (e.g. sequenced). For example various sequencing technologies may be used for single molecule sequencing, e.g. the Helicos or Pacbio technologies, or nanopore sequencing technologies which are being developed. Thus, in one embodiment the extended probe, i.e. the first strand of DNA, e.g. cDNA, may be subjected to sequencing. The first strand DNA, e.g. cDNA may need to be modified at the 3' end to enable single molecule sequencing. This may be done by procedures analogous to those for handling the second DNA, e.g. cDNA, strand. Such procedures are known in the art.

In a preferred aspect of the invention the sequence analysis will identify or reveal at least a portion of the extended probe, including at least part of captured nucleic acid, e.g. RNA, sequence and the sequence of the positional domain. The sequence of the positional domain (or tag) will identify the feature (or group of adjacent features) from which the capture probe was released and hence the location or vicinity on the solid substrate at which the nucleic acid, e.g. mRNA, molecule was captured. The sequence of the captured nucleic acid, e.g. RNA, molecule may be compared with a sequence database of the organism from which the biological specimen, e.g. tissue sample, originated to determine the gene to which it corresponds. By determining which region (e.g. cell) of the biological specimen, e.g. tissue sample, was in contact with the feature, it is possible to determine which region of the biological specimen, e.g. tissue sample, was expressing said gene (or contained the gene, e.g. in the case of spatial genomics). This analysis may be achieved for all of the DNA, e.g. cDNA, molecules generated by the methods of the invention, yielding a spatial transcriptome or genome of the biological specimen, e.g. tissue sample.

By way of a representative example, sequencing data may be analysed to sort the sequences into specific species of capture probe, i.e. according to the sequence of the positional domain. This may be achieved by, e.g. using the FastX toolkit FASTQ Barcode splitter tool to sort the sequences into individual files for the respective capture probe positional domain (tag) sequences. The sequences of each species, i.e. from each feature (or group of adjacent features), may be analyzed to determine the identity of the transcripts. For instance, the sequences may be identified using e.g. Blastn software, to compare the sequences to one or more genome databases, preferably the database for the organism from which the biological specimen, e.g. tissue sample, was obtained. The identity of the database sequence with the greatest similarity to the sequence generated by the methods of the invention will be assigned to said sequence. In general, only hits with a certainty of at least 1e⁻⁶, preferably 1e⁻⁷, 1e⁻⁸, or 1e⁻⁹ will be considered to have been successfully identified.

It will be apparent that any nucleic acid sequencing method may be utilised in the methods of the invention. However, the so-called "next generation sequencing" techniques will find particular utility in the present invention. High-throughput sequencing is particularly useful in the methods of the invention because it enables a large number of nucleic acids to be partially sequenced in a very short period of time. In view of the recent explosion in the number of fully or partially sequenced genomes, it is not essential to sequence the full length of the extended probes, e.g. cDNA molecules, to determine the gene to which each molecule corresponds. For example, the first 100 nucleotides from each end of the DNA, e.g. cDNA, molecules should be sufficient to identify both the feature from which the capture probe was released and hence the location or vicinity on the solid substrate at which the nucleic acid, e.g. mRNA, molecule was captured (i.e. its location on the solid substrate, e.g. array) and the gene expressed. The sequence reaction from the "capture probe end" of the DNA, e.g. cDNA, molecules yields the sequence of the positional domain and at least about 20 bases, preferably 30 or 40 bases of transcript or gene specific sequence data. The sequence reaction from the "non-capture probe end" may yield at least about 70 bases, preferably 80, 90, or 100 bases of transcript or gene specific sequence data.

In some embodiments it may be desirable to sequence the full-length of the extended probes generated by the methods of the invention. As mentioned above, template switching is particularly useful for generating full-length double stranded cDNA molecules and, in some embodiments, it may be desirable or advantageous to sequence the full-length of these molecules. Any suitable sequencing technique may be used to sequence the full-length DNA molecules and representative examples are presented below.

As a representative example, the sequencing reaction may be based on reversible dye-terminators, such as used in the Illumina^{™} technology. For example, DNA molecules are first attached to primers on, e.g. a glass or silicon slide and amplified so that local clonal colonies are formed (bridge amplification), as described above. Four types of ddNTPs are added, and non-incorporated nucleotides are washed away. Unlike pyrosequencing, the DNA can only be extended one nucleotide at a time. A camera takes images of the fluorescently labelled nucleotides then the dye along with the terminal 3' blocker is chemically removed from the DNA, allowing a next cycle. This may be repeated until the required sequence data is obtained. Using this technology, thousands of nucleic acids may be sequenced simultaneously on a single slide.

Other high-throughput sequencing techniques may be equally suitable for the methods of the invention, e.g. pyrosequencing. In this method the DNA is amplified inside water droplets in an oil solution (emulsion PCR), with each droplet containing a single DNA template attached to a single primer-coated bead that then forms a clonal colony. The sequencing machine contains many picolitre-volume wells each containing a single bead and sequencing enzymes. Pyrosequencing uses luciferase to generate light for detection of the individual nucleotides added to the nascent DNA and the combined data are used to generate sequence read-outs.

An example of a technology in development is based on the detection of hydrogen ions that are released during the polymerisation of DNA. A microwell containing a template DNA strand to be sequenced is flooded with a single type of nucleotide. If the introduced nucleotide is complementary to the leading template nucleotide it is incorporated into the growing complementary strand. This causes the release of a hydrogen ion that triggers a hypersensitive ion sensor, which indicates that a reaction has occurred. If homopolymer repeats are present in the template sequence multiple nucleotides will be incorporated in a single cycle. This leads to a corresponding number of released hydrogen ions and a proportionally higher electronic signal.

Thus, it is clear that new sequencing formats are being made available, and with shorter run times as one of the main features of those platforms, it will be evident that other sequencing technologies will be useful in the methods of the invention.

An essential feature of the present invention, as described above, is a step of extending the capture probes to produce a complementary strand of the "captured" nucleic acid molecules (i.e. the molecules hybridised to the capture domain of the capture probe), e.g. reverse transcribing the captured RNA molecules. The reverse transcription reaction is well known in the art and in representative reverse transcription reactions, the reaction mixture includes a reverse transcriptase, dNTPs and a suitable buffer. As mentioned above, in preferred embodiments, the reverse transcription reaction mixture also comprises means for releasing said capture probes from the surface of the solid substrate, e.g. a cleavage enzyme, such as the USER enzyme. The reaction mixture may comprise other components, such as RNase inhibitor(s), inhibitors of DNA-dependent DNA synthesis, e.g. actinomycin D. The primers and template are the capture domain of the capture probe and the captured RNA molecules as described above. In the subject methods, each dNTP will typically be present in an amount ranging from about 10 to 5000 µM, usually from about 20 to 1000 µM. The reaction mixture may be incubated on the solid substrate under conditions suitable for extension of the capture probes and cleavage of said probes from the solid substrate. Typically, the reaction mixture may be incubated on the solid substrate for at least 1 hour, e.g. 1-2 hours or 2, 3, 4 or more hours. However, shorter incubation times may be sufficient, e.g. about 30 minutes, such as 10-30 minutes, 15-30 minutes etc. It will be evident that an equivalent reaction may be performed to generate a complementary strand of a captured DNA molecule, using an enzyme with DNA polymerase activity. Reactions of this type are well known in the art and are described in more detail below.

In some embodiments it may be useful or advantageous to use labelled dNTPs in the extension reaction, e.g. reverse transcription reaction. The labelled dNTPs will be incorporated into the synthesized DNA molecule, e.g. cDNA molecule, thereby labelling the extended probes. In a representative embodiment, the labelled dNTP is a fluorescently labelled dNTP, e.g. Cy3-dCTP.

Thus, in some embodiments, the method further comprises a step of labelling the extended probes generated in step (c). In some embodiments, the labelling step may be contemporaneous with said extending step, i.e. by incorporation of labelled nucleotides into the extended probes. In some embodiments, the labelling step may be subsequent to the extending step, e.g. by using labelled nucleotides in the second strand synthesis and/or amplification steps, thereby incorporating labelled nucleotides into the second strand (i.e. complementary strand of the extended probes) and/or amplicons thereof. Alternatively, labels (detectable molecules) may be incorporated into the synthesized cDNA by binding to the molecules, e.g., via intercalation.

Representative detectable molecules that may find use in such embodiments include fluorescent nucleic acid stains, such as phenanthridinium dyes, including monomers or homo- or heterodimers thereof, that give an enhanced fluorescence when complexed with nucleic acids. Examples of phenanthridinium dyes include ethidium homodimer, ethidium bromide, propidium iodide, and other alkyl-substituted phenanthridinium dyes. In another embodiment of the invention, the nucleic acid stain is or incorporates an acridine dye, or a homo- or heterodimer thereof, such as acridine orange, acridine homodimer, ethidium-acridine heterodimer, or 9-amino-6-chloro-2-methoxyacridine. In yet another embodiment of the invention, the nucleic acid stain is an indole or imidazole dye, such as Hoechst 33258, Hoechst 33342, Hoechst 34580 (BIOPROBES 34, Molecular Probes, Inc. Eugene, Oreg., (May 2000)) DAPI (4',6-diamidino-2-phenylindole) or DIPI (4',6-(diimidazolin-2-yl)-2-phenylindole). Other permitted nucleic acid stains include, but are not limited to, 7-aminoactinomycin D, hydroxystilbamidine, LDS 751, selected psoralens (furocoumarins), styryl dyes, metal complexes such as ruthenium complexes, and transition metal complexes (incorporating Tb³⁺ and Eu³⁺, for example). In certain embodiments of the invention, the nucleic acid stain is a cyanine dye or a homo- or heterodimer of a cyanine dye that gives an enhanced fluorescence when associated with nucleic acids. Any of the dyes described in U.S. Pat. No. 4,883,867 to Lee (1989), U.S. Pat. No. 5,582,977 to Yue et al. (1996), U.S. Pat. No. 5,321,130 to Yue et al. (1994), and U.S. Pat. No. 5,410,030 to Yue et al. (1995) may be used, including nucleic acid stains commercially available under the trademarks TOTO, BOBO, POPO, YOYO, TO-PRO, BO-PRO, PO-PRO and YO-PRO from Molecular Probes, Inc., Eugene, Oreg. Any of the dyes described in U.S. Pat. No. 5,436,134 to Haugland et al. (1995), U.S. Pat. No. 5,658,751 to Yue et al. (1997), and U.S. Pat. No. 5,863,753 to Haugland et al. (1999) may be used, including nucleic acid stains commercially available under the trademarks SYBR Green, SYBR Gold, EvaGreen, SYTO, SYTOX, PICOGREEN, OLIGREEN, and RIBOGREEN from Molecular Probes, Inc., Eugene, Oreg. In yet other embodiments of the invention, the nucleic acid stain is a monomeric, homodimeric or heterodimeric cyanine dye that incorporates an aza- or polyazabenzazolium heterocycle, such as an azabenzoxazole, azabenzimidazole, or azabenzothiazole, that gives an enhanced fluorescence when associated with nucleic acids, including nucleic acid stains commercially available under the trademarks SYTO, SYTOX, JOJO, JO-PRO, LOLO, LO-PRO from Molecular Probes, Inc., Eugene, Oreg. The type of nucleic acid stain may be selected based on its capacity to bind to single or double stranded nucleic acid. In embodiments where the first cDNA strand is labelled, it may be preferable to use nucleic acid stains capable of labelling single stranded nucleic acid molecules as the RNA transcript captured on the substrate and used to template cDNA synthesis may be partially or fully degraded.

Further embodiments of labelling and detecting labelled DNA molecules in the context of spatial transcriptomic and genomic methods are described in WO2014/060483.

The reverse transcriptase reaction may be carried out at any suitable temperature, which will be dependent on the properties of the enzyme. Typically, reverse transcriptase reactions are performed between 37-55°C, although temperatures outside of this range may also be appropriate. The reaction time may be as little as 1, 2, 3, 4 or 5 minutes or as much as 48 hours. Typically the reaction will be carried out for between 5-120 minutes, preferably 5-60, 5-45 or 5-30 minutes or 1-10 or 1-5 minutes according to choice. The reaction time is not critical and any desired reaction time may be used.

As indicated above, certain embodiments of the methods include an amplification step, where the copy number of generated DNA, e.g. cDNA, molecules is increased, e.g., in order to enrich the sample to obtain a better representation of the nucleic acids, e.g. transcripts, captured from the biological specimen, e.g. tissue sample. The amplification may be linear or exponential, as desired, where representative amplification protocols of interest include, but are not limited to: polymerase chain reaction (PCR); isothermal amplification, etc.

The polymerase chain reaction (PCR) is well known in the art, being described in U.S. Pat. Nos.: 4,683,202; 4,683,195; 4,800,159; 4,965,188 and 5,512,462. In representative PCR amplification reactions, the reaction mixture that includes the above extended probes, e.g. cDNA molecules, which may be pooled, which are combined with one or more primers that are employed in the primer extension reaction, e.g., the PCR primers that hybridize to the first and/or second amplification domains (such as forward and reverse primers employed in geometric (or exponential) amplification or a single primer employed in a linear amplification). The oligonucleotide primers with which the extended probes, e.g. cDNA molecules, (hereinafter referred to as template DNA for convenience) is contacted will be of sufficient length to provide for hybridization to complementary template DNA under annealing conditions (described in greater detail below). The length of the primers will depend on the length of the amplification domains, but will generally be at least 10 bp in length, usually at least 15 bp in length and more usually at least 16 bp in length and may be as long as 30 bp in length or longer, where the length of the primers will generally range from 18 to 50 bp in length, usually from about 20 to 35 bp in length. The template DNA may be contacted with a single primer or a set of two primers (forward and reverse primers), depending on whether primer extension, linear or exponential amplification of the template DNA is desired.

In addition to the above components, the reaction mixture produced in the subject methods typically includes a polymerase and deoxyribonucleoside triphosphates (dNTPs). The desired polymerase activity may be provided by one or more distinct polymerase enzymes. In many embodiments, the reaction mixture includes at least a Family A polymerase, where representative Family A polymerases of interest include, but are not limited to: Thermus aquaticus polymerases, including the naturally occurring polymerase (Taq) and derivatives and homologues thereof, such as Klentaq (as described in Barnes et al, Proc. Natl. Acad. Sci USA (1994) 91:2216-2220); Thermus thermophilus polymerases, including the naturally occurring polymerase (Tth) and derivatives and homologues thereof, and the like. In certain embodiments where the amplification reaction that is carried out is a high fidelity reaction, the reaction mixture may further include a polymerase enzyme having 3'-5' exonuclease activity, e.g., as may be provided by a Family B polymerase, where Family B polymerases of interest include, but are not limited to: Thermococcus litoralis DNA polymerase (Vent) as described in Perler et al., Proc. Natl. Acad. Sci. USA (1992) 89:5577-5581; Pyrococcus species GB-D (Deep Vent); Pyrococcus furiosus DNA polymerase (Pfu) as described in Lundberg et al., Gene (1991) 108:1-6, Pyrococcus woesei (Pwo) and the like. Where the reaction mixture includes both a Family A and Family B polymerase, the Family A polymerase may be present in the reaction mixture in an amount greater than the Family B polymerase, where the difference in activity will usually be at least 10-fold, and more usually at least about 100-fold. Usually the reaction mixture will include four different types of dNTPs corresponding to the four naturally occurring bases present, i.e. dATP, dTTP, dCTP and dGTP. In the subject methods, each dNTP will typically be present in an amount ranging from about 10 to 5000 µM, usually from about 20 to 1000 µM.

The reaction mixtures prepared in the reverse transcriptase and/or amplification steps of the subject methods may further include an aqueous buffer medium that includes a source of monovalent ions, a source of divalent cations and a buffering agent. Any convenient source of monovalent ions, such as KCI, K-acetate, NH₄-acetate, K-glutamate, NH₄Cl, ammonium sulphate, and the like may be employed. The divalent cation may be magnesium, manganese, zinc and the like, where the cation will typically be magnesium. Any convenient source of magnesium cation may be employed, including MgCl₂, Mg-acetate, and the like. The amount of Mg²⁺ present in the buffer may range from 0.5 to 10 mM, but will preferably range from about 3 to 6 mM, and will ideally be at about 5 mM. Representative buffering agents or salts that may be present in the buffer include Tris, Tricine, HEPES, MOPS and the like, where the amount of buffering agent will typically range from about 5 to 150 mM, usually from about 10 to 100 mM, and more usually from about 20 to 50 mM, where in certain preferred embodiments the buffering agent will be present in an amount sufficient to provide a pH ranging from about 6.0 to 9.5, where most preferred is pH 7.3 at 72 °C. Other agents which may be present in the buffer medium include chelating agents, such as EDTA, EGTA and the like.

In preparing the reverse transcriptase, DNA extension or amplification reaction mixture of the steps of the subject methods, the various constituent components may be combined in any convenient order. For example, in the amplification reaction the buffer may be combined with primer, polymerase and then template DNA, or all of the various constituent components may be combined at the same time to produce the reaction mixture.

As discussed above, a preferred embodiment of the invention the extended probes, e.g. cDNA molecules, may be modified by the addition of amplification domains to the ends of the nucleic acid molecules, which may involve a ligation reaction.

As is known in the art, ligases catalyze the formation of a phosphodiester bond between juxtaposed 3'-hydroxyl and 5'-phosphate termini of two immediately adjacent nucleic acids. Any convenient ligase may be employed, where representative ligases of interest include, but are not limited to: Temperature sensitive and thermostable ligases. Temperature sensitive ligases include, but are not limited to, bacteriophage T4 DNA ligase, bacteriophage T7 ligase, and E. coli ligase. Thermostable ligases include, but are not limited to, Taq ligase, Tth ligase, and Pfu ligase. Thermostable ligase may be obtained from thermophilic or hyperthermophilic organisms, including but not limited to, prokaryotic, eukaryotic, or archael organisms. Certain RNA ligases may also be employed in the methods of the invention.

In this ligation step, a suitable ligase and any reagents that are necessary and/or desirable are combined with the reaction mixture and maintained under conditions sufficient for ligation of the relevant oligonucleotides to occur. Ligation reaction conditions are well known to those of skill in the art. During ligation, the reaction mixture in certain embodiments may be maintained at a temperature ranging from about 4°C to about 50°C, such as from about 20°C to about 37°C for a period of time ranging from about 5 seconds to about 16 hours, such as from about 1 minute to about 1 hour. In yet other embodiments, the reaction mixture may be maintained at a temperature ranging from about 35°C to about 45°C, such as from about 37°C to about 42°C, e.g., at or about 38°C, 39°C, 40°C or 41°C, for a period of time ranging from about 5 seconds to about 16 hours, such as from about 1 minute to about 1 hour, including from about 2 minutes to about 8 hours. In a representative embodiment, the ligation reaction mixture includes 50 mM Tris pH7.5, 10 mM MgCl₂, 10 mM DTT, 1 mM ATP, 25 mg/ml BSA, 0.25 units/ml RNase inhibitor, and T4 DNA ligase at 0.125 units/ml. In yet another representative embodiment, 2.125 mM magnesium ion, 0.2 units/ml RNase inhibitor; and 0.125 units/ml DNA ligase are employed. The amount of adaptor in the reaction will be dependent on the concentration of the DNA, e.g. cDNA in the sample and will generally be present at between 10-100 times the molar amount of DNA, e.g. cDNA.

By way of a representative example the method of the description may comprise the following steps:
(a) providing a solid substrate on which multiple species of capture probes are immobilized such that each species occupies a distinct position on the solid substrate, wherein said probes are for a reverse transcription primer extension reaction (preferably where said probes are oriented on the solid substrate to have a free 3' end) and wherein each species of said capture probe comprises a nucleic acid molecule comprising:
   (i) a cleavage domain for releasing the capture probe from the surface of the solid substrate,
   (ii) a positional domain that corresponds to the position of the capture probe on the solid substrate, and
   (iii) a capture domain;
(b) contacting said solid substrate with a biological specimen;
(c) imaging the biological specimen on the solid substrate;
(d) releasing said capture probes from the surface of the solid substrate under conditions that allow RNA of the biological specimen to hybridise to the capture domain in said capture probes and simultaneously and/or subsequently extending said capture probes using the RNA hybridised to the capture probes as extension templates to produce extended probes (cDNA molecules) thereby spatially tagging the RNA of the biological specimen;
(e) performing second strand cDNA synthesis on the extended probes;
   and
(f) analysing the extended probes, e.g. the sequence of the extended probes (cDNA molecules).

By way of an alternative representative example the method of the description may comprise the following steps:
(a) providing a solid substrate on which multiple species of capture probes are immobilized such that each species occupies a distinct position on the solid substrate, wherein said probes are for a reverse transcription primer extension reaction (preferably where said probes are oriented on the solid substrate to have a free 3' end) and wherein each species of said capture probe comprises a nucleic acid molecule comprising:
   (i) a cleavage domain for releasing the capture probe from the surface of the solid substrate,
   (ii) a positional domain that corresponds to the position of the capture probe on the solid substrate, and
   (iii) a capture domain;
(b) contacting said solid substrate with a biological specimen;
(c) optionally rehydrating the tissue sample;
(d) releasing said capture probes from the surface of the solid substrate under conditions that allow RNA of the biological specimen to hybridise to the capture domain in said capture probes and simultaneously and/or subsequently extending said capture probes using the RNA hybridised to the capture probes as extension templates to produce extended probes (cDNA molecules) thereby spatially tagging the RNA of the biological specimen.
(e) imaging the biological specimen on the solid substrate;
(f) amplifying the extended probes; and
(g) analysing the amplified extended probes, e.g. the sequence of the amplified extended probes (cDNA molecules).

By way of yet a further representative example the method of the description may comprise the following steps:
(a) providing a solid substrate on which multiple species of capture probes are immobilized such that each species occupies a distinct position on the solid substrate, wherein said probes are for a reverse transcription primer extension reaction (preferably where said probes are oriented on the solid substrate to have a free 3' end) and wherein each species of said capture probe comprises a nucleic acid molecule comprising:
   (i) a cleavage domain for releasing the capture probe from the surface of the solid substrate,
   (ii) a positional domain that corresponds to the position of the capture probe on the solid substrate, and
   (iii) a capture domain;
(b) contacting said solid substrate with a biological specimen;
(c) optionally rehydrating the tissue sample;
(d) releasing said capture probes from the surface of the solid substrate under conditions that allow RNA of the biological specimen to hybridise to the capture domain in said capture probes and simultaneously and/or subsequently extending said capture probes using the RNA hybridised to the capture probes as extension templates to produce extended probes (cDNA molecules) thereby spatially tagging the RNA of the biological specimen;
(e) optionally imaging the biological specimen on the solid substrate;
(f) performing second strand cDNA synthesis on the extended probes;
(g) optionally pooling the double stranded extended probes from (f);
(h) amplifying the double stranded extended probes from (f) or (g); and
(i) analysing the amplified extended probes, e.g. the sequence of the amplified extended probes (cDNA molecules).

The present invention includes any suitable combination of the steps in the above described methods. It will be understood that the invention also encompasses variations of these methods, for example where amplification is performed on the solid substrate. Also encompassed are methods which omit the imaging step and methods that comprise a step of correlating sequence analysis information obtain from analysing the extended probes (including double stranded extended probes and/or amplicons thereof) with an image of said biological specimen.

As mentioned above, although the invention is described primarily with reference to detection or analysis of RNA, and transcriptome analysis or detection, it will be appreciated that the principles described can be applied analogously to the detection or analysis of DNA in cells and to genomic studies. Thus, more broadly viewed, the invention can be seen as being generally applicable to the detection of nucleic acids in general and in a further more particular aspect, as providing methods for the analysis or detection of DNA. Spatial information may be valuable also in a genomics context, i.e. detection and/or analysis of a DNA molecule with spatial resolution. This may be achieved by genomic tagging according to the present invention. Such spatial tagging, e.g. localised or spatial detection, methods may be useful for example in the context of studying genomic variations in different cells or regions of a tissue, for example comparing normal and diseased cells or tissues (e.g. normal vs. tumour cells or tissues) or in studying genomic changes in disease progression etc. For example, tumour tissues may comprise a heterogeneous population of cells which may differ in the genomic variants they contain (e.g. mutations and/or other genetic aberrations, for example chromosomal rearrangements, chromosomal amplifications/deletions/insertions etc.). The detection of genomic variations, or different genomic loci, in different cells in a localised way may be useful in such a context, e.g. to study the spatial distribution of genomic variations. A principal utility of such a method would be in tumour analysis. In the context of the present invention, a solid substrate, e.g. array, may be prepared which is designed, for example, to capture the genome of an entire cell on the capture probes from one feature. Different cells in the biological specimen, e.g. tissue sample, may thus be compared. Of course the invention is not limited to such a design and other variations may be possible, wherein the DNA is detected in a localised way and the position of the DNA captured by the capture probes released from the solid substrate, e.g. array, is correlated to a position or location in the biological specimen, e.g. tissue sample.

The sequence analysis (e.g. sequencing) information obtained in the step of analysing the extended probes (including double stranded extended probes and/or amplicons thereof) may be used to obtain spatial information as to the nucleic acid in the sample. In other words the sequence analysis information may provide information as to the location of the nucleic acid in the sample. This spatial information may be derived from the nature of the sequence analysis information obtained, e.g. from a sequence determined or identified, for example it may reveal the presence of a particular nucleic acid molecule which may itself be spatially informative in the context of the tissue sample used, and/or the spatial information (e.g. spatial localisation) may be derived from the position of the tissue sample on the array, coupled with the sequence analysis information. However, as described above, spatial information may conveniently be obtained by correlating the sequence analysis data to an image of the biological specimen, e.g. tissue sample, and this represents one preferred embodiment of the invention.

The extension reaction referred to in step (c) may be defined as a polymerase-catalysed extension reaction and acts to acquire a complementary strand of the "captured nucleic acid molecule", i.e. the nucleic acid molecule that is hybridised to the capture domain of the capture probe, i.e. by synthesising the complementary strand utilising the capture probe as a primer and the captured nucleic acid as a template. In other words it may be any primer extension reaction carried out by any polymerase enzyme. Thus, when the captured nucleic acid molecule nucleic acid is DNA, e.g. genomic DNA, the polymerase will be a DNA polymerase.

Thus, in some embodiments the description provides the method comprises:
(a) providing a solid substrate on which multiple species of capture probes are immobilized such that each species occupies a distinct position on the solid substrate, wherein said probes are for a primer extension reaction (preferably where said probes are oriented on the solid substrate to have a free 3' end) and wherein each species of said capture probe comprises a nucleic acid molecule comprising:
   (i) a cleavage domain for releasing the capture probe from the surface of the solid substrate,
   (ii) a positional domain that corresponds to the position of the capture probe on the solid substrate, and
   (iii) a capture domain;
(b) contacting said solid substrate with a biological specimen; and
(c) fragmenting DNA in said biological specimen tissue sample, wherein said fragmentation is carried out before, during or after contacting the solid substrate with the biological specimen in step (b);
(d) releasing said capture probes from the surface of the solid substrate under conditions that allow DNA of the biological specimen to hybridise to the capture domain in said capture probes and simultaneously and/or subsequently extending said capture probes using the DNA hybridised to the capture probes as extension templates to produce extended probes thereby spatially tagging the DNA of the biological specimen;
(e) optionally generating a complementary strand of said tagged DNA and/or optionally amplifying said tagged DNA;
(f) optionally pooling the extended probes (tagged DNA) from (d) or pooling the double stranded extended probes (tagged DNA) and/or amplicons from step (e); and
(g) analysing the extended probes (tagged DNA) from (d) or (f) and/or the double stranded extended probes (tagged DNA) and/or amplicons from step (e) or (f), e.g. the sequence of the extended probes (tagged DNA) and/or double stranded extended probes (tagged DNA) and/or amplicons .

The method may further include a step of:
(h) correlating said sequence analysis information with an image of said biological specimen, wherein the biological specimen is imaged before or after step (d), preferably before step (d).

In the context of spatial genomics, where the target nucleic acid is DNA the inclusion of imaging and image correlation steps may in some circumstances be preferred.

In embodiments in which DNA is captured, the DNA may be any DNA molecule which may occur in a cell. Thus it may be genomic, i.e. nuclear, DNA, mitochondrial DNA or plastid DNA, e.g. chloroplast DNA. In a preferred embodiment, the DNA is genomic DNA.

It will be understood that where fragmentation is carried out after the contacting in step (b), i.e. after the biological specimen is placed on the solid substrate, e.g. array, fragmentation occurs before the DNA is hybridised to the capture domain. In other words the DNA fragments are hybridised (or more particularly, allowed to hybridise) to the capture domain in said capture probes.

As noted above, in some embodiments it is preferred that the (target) nucleic acid molecules (particularly RNA) in the biological specimen are not treated or modified to enhance or facilitate their interaction with the capture probes prior to contacting the biological specimen with the solid substrate, e.g. contacted with or hybridized to nucleic acids that function as an intermediary between the target nucleic acid of the biological specimen and the capture probes, i.e. that function to indirectly attach or link the target nucleic acid of the biological specimen to the capture probe. However, advantageously, but not necessarily, in a particular embodiment of this aspect of the invention, the DNA fragments of the biological specimen, e.g. tissue sample, may be provided with a binding domain to enable or facilitate their capture by the capture probes. Accordingly, the binding domain is capable of hybridising to the capture domain of the capture probe. Such a binding domain may thus be regarded as a complement of the capture domain (i.e. it may be viewed as a complementary capture domain), although absolute complementarity between the capture and binding domains is not required, merely that the binding domain is sufficiently complementary to allow a productive hybridisation to take place, i.e. that the DNA fragments in the biological specimen, e.g. tissue sample, are able to hybridise to the capture domain of the capture probes. Provision of such a binding domain may ensure that DNA in the sample does not bind to the capture probes until after the fragmentation step. The binding domain may be provided to the DNA fragments by procedures well known in the art, for example by ligation of adaptor or linker sequences which may contain the binding domain. For example a linker sequence with a protruding end may be used. The binding domain may be present in the single-stranded portion of such a linker, such that following ligation of the linker to the DNA fragments, the single-stranded portion containing the binding domain is available for hybridisation to the capture domain of the capture probes. Alternatively and in a preferred embodiment, the binding domain may be introduced by using a terminal transferase enzyme to introduce a polynucleotide tail, e.g. a homopolymeric tail such as a poly-A domain. This may be carried out using a procedure analogous to that described above for introducing a second amplification domain in the context of the RNA methods. Thus, in advantageous embodiments a common binding domain may be introduced. In other words, a binding domain which is common to all the DNA fragments and which may be used to achieve the capture of the fragments on the solid substrate, e.g. array. As discussed below, in some embodiments, the common binding domain may be introduced to the DNA during (i.e. simultaneously with) the step of fragmenting the DNA, e.g. where the DNA is fragmented using a transposase (e.g. Tn5) the common binding domain may be simultaneously ligated to the DNA fragments.

Where a tailing reaction is carried out to introduce a (common) binding domain, the capture probes on the solid substrate, e.g. array, may be protected from the tailing reaction, i.e. the capture probes may be blocked or masked as described above. This may be achieved for example by hybridising a blocking oligonucleotide to the capture probe, e.g. to the protruding end (e.g. single stranded portion) of the capture probe. Where the capture domain comprises a poly-T sequence for example, such a blocking oligonucleotide may be a poly-A oligonucleotide. The blocking oligonucleotide may have a blocked 3' end (i.e. an end incapable of being extended, or tailed). The capture probes may also be protected, i.e. blocked, by chemical and/or enzymatic modifications, as described in detail above.

Thus, in some embodiments of the invention, the method may comprise a step of providing the DNA fragments with a binding domain which is capable of hybridising to the capture domain. This step may be performed after (or at the same time as) the step of fragmenting the DNA but before the step of releasing and extending the capture probes. In preferred embodiments, the step of providing the DNA fragments with a binding domain which is capable of hybridising to the capture domain is performed after the biological specimen if contacted with the substrate (e.g. array), i.e. the step is performed *in situ* on the substrate.

In the methods of DNA detection set out above, it will be evident that the step of extending the capture probe and the optional step of generating a complementary copy of the tagged DNA or of amplifying the tagged DNA, may involve or require the use of a strand displacing polymerase enzyme. For instance, the fragmented DNA hybridised to the capture probe may be partially double stranded DNA and thus the extension of the capture probe may require the displacement of one of the DNA strands. Suitable strand displacing polymerases are discussed above. In the context of producing complementary strand of the extended probe and/or amplicons of the extended probe, the use of strand displacement polymerases is to ensure that the positional domain is copied into the complementary copy or amplicon. This will particularly be the case where the capture probe is immobilized on the solid substrate, e.g. array, by hybridisation to a surface probe.

Thus, in one embodiment, the method of the invention may be used for determining and/or analysing all of the genome of a tissue sample, e.g. the global genome of a tissue sample. However, the method is not limited to this and encompasses determining and/or analysing all or part of the genome. Thus, the method may involve determining and/or analysing a part or subset of the genome, e.g. a partial genome corresponding to a subset or group of genes or of chromosomes, e.g. a set of particular genes or chromosomes or a particular region or part of the genome, for example related to a particular disease or condition, tissue type etc. Thus, the method may be used to detect or analyse genomic sequences or genomic loci from tumour tissue as compared to normal tissue, or even within different types of cell in a tissue sample. The presence or absence, or the distribution or location of different genomic variants or loci in different cells, groups of cells, tissues or parts or types of tissue may be examined.

Viewed from another aspect, the method steps set out above can be seen as providing a method of obtaining spatial information regarding the nucleic acids, e.g. genomic sequences, variants or loci of a biological specimen, e.g. tissue sample. Put another way, the methods of the invention may be used for the labelling (or tagging) of genomes, particularly individual or spatially distributed genomes.

Alternatively viewed, the method of the invention may be seen as a method for spatial detection of DNA in a biological specimen, e.g. tissue sample, or a method for detecting DNA with spatial resolution, or for localised or spatial determination and/or analysis of DNA in a biological specimen, e.g. tissue sample. In particular, the method may be used for the localised or spatial detection or determination and/or analysis of genes or genomic sequences or genomic variants or loci (e.g. distribution of genomic variants or loci) in a biological specimen, e.g. tissue sample. The localised/spatial detection/determination/analysis means that the DNA may be localised to its native position or location within a cell or tissue in the biological specimen, e.g. tissue sample. Thus for example, the DNA may be localised to a cell or group of cells, or type of cells in the sample, or to particular regions of areas within a biological specimen, e.g. tissue sample. The native location or position of the DNA (or in other words, the location or position of the DNA in the biological specimen, e.g. tissue sample), e.g. a genomic variant or locus, may be determined.

The step of fragmenting DNA in a tissue sample may be carried out using any desired procedure known in the art. Thus physical methods of fragmentation may be used, e.g. sonication or ultrasound treatment. Chemical methods are also known. Enzymatic methods of fragmentation may also be used, e.g. with endonucleases, for example restriction enzymes, or transposases, e.g. Tn5. Again methods and enzymes for this are well known in the art. Notably a transposase, such as Tn5, may advantageously result in the simultaneous fragmentation of DNA and ligation of adapters (e.g. comprising common binding domains) to the 5' ends of both strands of DNA fragments, which may facilitate the capture of the fragmented DNA by the capture probes, i.e. the adapters ligated to the 5' ends of the DNA may contain a domain that is capable of interacting with (i.e. hybridizing to) the capture domain of the capture probes. Fragmentation may be done before during or after preparing the biological specimen, e.g. tissue sample, for placing on a solid substrate, e.g. array, e.g. preparing a tissue section. Conveniently, fragmentation may be achieved in the step of fixing tissue. Thus for example, formalin fixation will result in fragmentation of DNA. Other fixatives may produce similar results.

In terms of the detail of preparing and using the solid substrate, e.g. array, in these aspects of the invention, it will understood that the description and detail given above in the context of RNA methods applies analogously to the more DNA detection methods set out herein. Thus, all aspects and details discussed above apply analogously. For example, the discussion of reverse transcriptase primers and reactions etc. may be applied analogously to any aspect of the extension primers, DNA polymerase reactions etc. referred to above. Likewise, references and to first and second strand cDNA synthesis may be applied analogously to the tagged DNA molecule and its complement. Methods of sequence analysis as discussed above may be used.

By way of example, the capture domain may be as described for the capture probes above. A poly-T or poly-T-containing capture domain may be used for example where the DNA fragments are provided with a binding domain comprising a poly-A sequence.

The capture probes/tagged DNA molecules (i.e. the extended probes comprising complements of captured DNA molecules) may be provided with amplification domains as described above.

The invention will be further described with reference to the following nonlimiting Examples with reference to the following drawings in which:
Figure 1 shows a schematic of the experimental design described in Example 2.
Figure 2 shows a fluorescence microscopy image of an array on which cDNA has been synthesised (incorporating nucleotides labelled with Cy3) according to the methods described in Example 2. (A) shows an image following cDNA synthesis (C) and (D) or combined cDNA synthesis and release of the capture probes (E). (B) shows an image following release of the extended capture probes (C) and (D) or after a further incubation period (E).
Figure 3 shows bar charts showing: (A) the total number of unique transcripts identified following separate extension and release steps (ST1.0) and following combined extension and release steps (ST2.0); and (B) the total number of unique genes identified following separate extension and release steps (ST1.0) and following combined extension and release steps (ST2.0).
Figure 4 shows heat maps showing the number transcripts captured at each feature from mouse brain tissue sections using: (A) separate extension and release steps (ST1.0); and (B) combined extension and release steps (ST2.0).
Figure 5 shows an overlay of an image of mouse brain tissue and Penk transcripts captured at features on the array contacted with said tissue using: (A) separate extension and release steps (ST1.0); and (B) combined extension and release steps (ST2.0).

### Example 1

The protocol described below is representative of the "standard" spatial transcriptomic methods in which the steps of cDNA synthesis and release of the extended probes are performed separately, known as "ST1.0".

### Preparation of in-house printed microarray with 5' to 3' oriented probes

The RNA-capture oligonucleotide (Table 1) was printed on glass slides to function as the capture probe. The probe was synthesized with a 5'-terminus amino linker with a C6 spacer. 1007 capture oligonucleotides with 18-mer unique barcodes, a 9-mer semi-randomized UMI and a poly-20TVN capture region were arrayed onto the surface of Codelink Activated Slides according to the manufacturer's (Surmodics) instructions. The oligonucleotides were immobilized in 100µm features with 200µm center-to-center distance (pitch) between the spots. According to the slide manufacturer approximately 200 million oligonucleotides were immobilized in each spot. An area in the top left corner of the array was left blank for the purpose of orientation. Six 6200 x 6600 µm arrays were prepared per glass slide. After printing, surface blocking was performed according to the manufacturer's instructions.

**Table 1**

| Probe 1 |
|---|
| Amino-C6- UUUUUGACTCGTAATACGACTCACTATAGGGACACGACGCTCTTCCGATCT GTCCGATATGATTGCCGCWSNNWSNNVTTTTTTTTTTTTTTTTTTTUVN (SEQ ID NO: 3) |

### Collection and preparation of olfactory bulbs

Adult C57BL/6 mice (>2 months old) were euthanized and olfactory bulbs were immediately isolated and snap-frozen in Isopentane. Tissue was embedded in cold OCT before sectioning. The olfactory bulb was sectioned on a cryostat at a thickness of 10µm. Sections were mounted onto spatially barcoded arrays.

### Permeabilization and reverse transcription

For each well, corresponding to each sub-array with a section, 70µl of 1x Exonuclease I Reaction Buffer (#B0293S, NEB) with 0.19µg/µl BSA (#B9000S, NEB) was added and incubated at 37°C for 30 minutes. Each well was washed with 100µl 0.1x SSC, diluted in deionized water from stock solution (#S6639, Sigma-Aldrich). Next, 70µl of 0.1% pepsin (#P7000-25G, Sigma-Aldrich) dissolved in 0.1M HCl (#318965-1000ML, Sigma-Aldrich) was added to each well and incubated at 37°C for 10 minutes. Each well was washed as previously described and 70µl of reverse transcription mix was added to each well and incubated overnight (ON).

The reverse transcription mix contained 1x First Strand Buffer (#18080-044, Invitrogen), 5mM DTT (#18080-044, Invitrogen), 500µM of dATP/dGTP/dTTP, 12.5µM of dCTP, 25µM of Cyanine 3-dCTP (#NEL576001EA, PerkinElmer), 0.19µg/µl BSA, 50ng/µl Actinomycin D (#A1410-2MG, Sigma-Aldrich), 1% DMSO (#472301-500ML, Sigma-Aldrich), 20U/µl Superscript III (#18080-04, Invitrogen) and 2U/µl RNaseOUT (#10777-019, Invitrogen). The permeabilization was carried out for 2, 10 or 30 minutes. The cDNA synthesis was carried out for 1 hour, 3 hours, 6 hours or overnight.

To degrade and remove mouse tissue, 70µl Proteinase K (#19131, Qiagen) and PKD Buffer (pH 7.5, according to the manufacturer, #1034963, Qiagen), at a ratio of 1:7, were added to each well and incubated at 56°C for 1 hour. After the incubation, slides washed with 2x SSC with 0.1% SDS at 50°C (#71736-100ML, Sigma-Aldrich) followed by 0.2x SSC and 0.1x SSC at room temperature (RT) and finally spin-dried.

### Imaging

The array was imaged at 532 nm using an Agilent microarray scanner at 100% exposure and 5µm resolution.

### Release of cDNA from the array

The array was attached to an Arraylt slide holder and 16 well mask (Arraylt Corporation). A cleavage mixture (50µl) containing 1x Exo I buffer (New England Biolabs, Ipswich, MA, USA), 1x BSA, RNase/DNase free water, 5U of USER enzyme mix (New England Biolabs) was added to each well. The reactions were covered with a plastic sealer and incubated at 37°C for 1 hour using interval mixing of 3 seconds at 300rpm and 6 seconds rest. After the incubation, 45µl cleavage mixture was collected from each of the used wells and placed into 0.2ml PCR tubes.

### Example 2

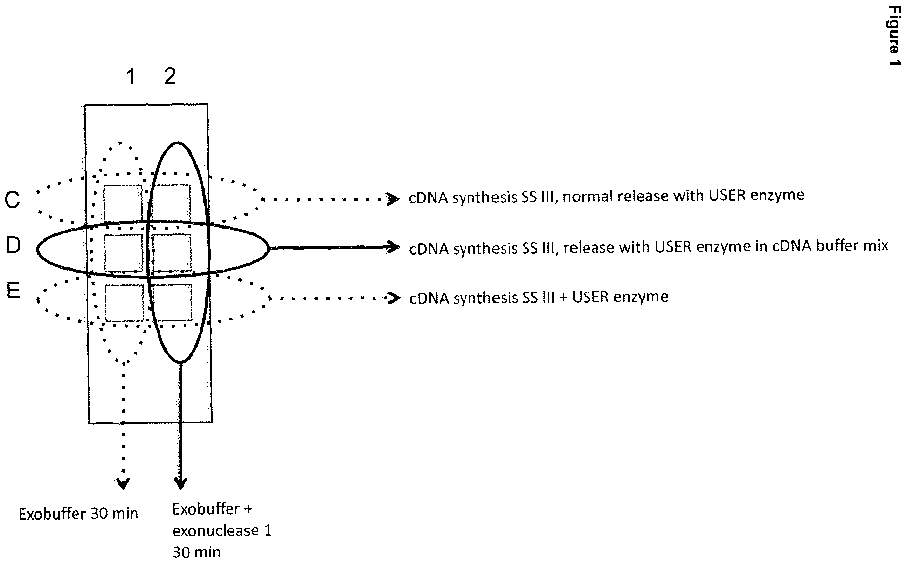

An experiment was designed to determine the effects of performing the extension and release steps simultaneously. A schematic of the experimental design is shown in Figure 1, which depicts six different treatments: C1, C2, D1, D2, E1 and E2.

C1 and C2 relate to the "standard" protocol described in Example 1 above, using the cDNA synthesis mix set out below:

| | |
|---|---|
| a) First strand buffer | 64µL |
| b) DTT | 16µL |
| c) RNase & DNase free water | 133µL |
| d) Actinmycin D | 32µL |
| e) dNTP mix | 16µL |
| f) Cy3 dCTP | 8µL |
| g) BSA | 3µL |
| h) RNase OUT | 16µL |
| i) SuperScript^{®} III | 32µL |

The tissue permeabilization step included treatment with an exonuclease buffer (C1) or exonuclease buffer comprising exonuclease I.

D1 and D2 differ from C1 and C2 only with respect to the cleavage step, wherein the cleavage mixture comprised the USER enzyme in the cDNA buffer mix. The cleavage mix is set out below:

| | |
|---|---|
| a) First strand buffer | 32µL |
| b) DTT | 8µL |
| c) RNAse & DNase free water | 70.5µL |
| d) Actinomycin D | 16µL |
| e) dNTPs | 8µL |
| f) BSA | 1.5µL |
| g) USER enzyme | 16µL |

E1 and E2 differ from C1 and C2 insofar as the cDNA synthesis mix included the cleavage enzyme, i.e. the USER enzyme. The reaction mixture for combined, i.e. simultaneous, cDNA synthesis and probe release is set out below:

| | |
|---|---|
| a) First strand buffer | 32µL |
| b) DTT | 8µL |
| c) RNase & DNase free water | 50.5µL |
| d) Actinmycin D | 16µL |
| e) dNTP mix | 8µL |
| f) Cy3 dCTP | 4µL |
| g) BSA | 1.5µL |
| h) RNase OUT | 8µL |
| i) SuperScript^{®} III | 16µL |
| j) USER enzyme | 16µL |

The protocols E1 and E2 did not include the tissue removal step. The E protocol is also referred to herein as ST2.0.

For each well, the release mixture was added and incubated at 37°C for 1-2 hours.

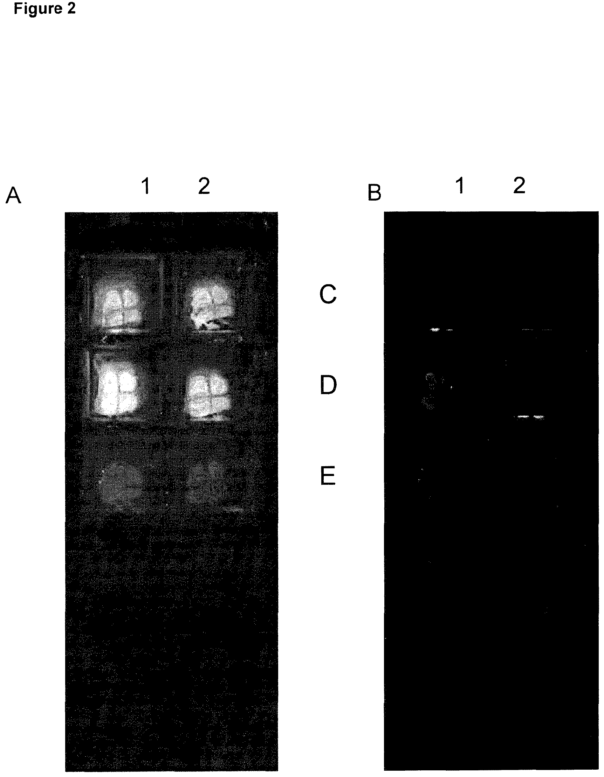

Figure 2 shows the Cy3 signal from the surface of the arrays, i.e. the fluorescent label incorporated into the extended probes, after cDNA synthesis (A) and about 3 hours after the cleavage reaction. Figure 2 demonstrates that protocol C results in cDNA synthesis on the array surface (A) and the subsequent release of the extended probes from the array surface (B), as expected.

Protocol D shows that the cleavage enzyme (USER) is able to function efficiently in the reverse transcriptase buffer, as the extended probes are released from the surface of the array with similar efficacy to the cleavage reaction in protocol C.

Protocol E shows that the capture probes were successfully cleaved simultaneously with cDNA synthesis. In this respect, the extended probes from protocols C and E were pooled and subjected to sequence analysis to confirm that the cDNA was successfully synthesized in protocol E.

### Collection of extended probe mixture

65µl of the extended probe mixture was collected from each of the used wells and placed into 0.2ml PCR tubes.

### cDNA second strand synthesis for preparation of a cDNA library for sequencing

5µl of second strand mix containing 2.7x First Strand Buffer, 3.7U/µl DNA polymerase I (#18010-017, Invitrogen) and 0.18U/µl RNaseH (#18021-014, Invitrogen) was added and samples were incubated at 16°C for 2 hours, 5µl T4 DNA polymerase (#M0203S, NEB) was added and samples were incubated at 16°C for another 20 minutes. 25µl of deionized water or 80mM EDTA (#15575- 038, Invitrogen) was added and purified using Agencourt RNAClean XP beads (#A63987, Beckman Coulter) according to the manufacturer's protocol and eluted into deionized water. 5.6µl of the samples were mixed with 10.4µl In Vitro Transcription mix with a final content of 1x T7 Reaction Buffer (#AM1333, Ambion), 7.5mM of each NTP (#AM1333, Ambion), 1x T7 Enzyme Mix (#AM1333, Ambion) and 1U/µl SUPERaselN (#AM2694, Ambion). Samples were incubated at 37°C for 14 hours.

The samples were purified using Agencourt RNAClean XP beads according to the manufacturer's protocol and eluted into 10µl deionized water. The amount and average fragment length of amplified RNA (aRNA) was determined by using the RNA 6000 Pico Kit (#5067-1513, Agilent) with a 2100 Bioanalyzer (Agilent) according to the manufacturer's protocol. The remaining sample and 2.5µl Ligation adapter was added to a final concentration of 0.71µM. Samples were heated at 70°C for 2 minutes then placed on ice before 4.5µ! ligation mix was added to a final content of 1x T4 RNA Ligase Reaction Buffer (#B0216L, NEB), 20U/µl T4 RNA Ligase2, truncated (#M0242L, NEB), 4U/µl RNase Inhibitor, Murine (#M0314L, NEB) and 0.5µM Ligation Adapter. Samples were incubated at 25°C for 1 hour. The samples were purified using Agencourt RNAClean XP beads according to the protocol previously described and mixed with 1µl RT-primer (IDT) to a final concentration of 1.7µM and 1µl dNTPs and a final concentration 0.83mM of each dNTP. Samples were heated at 65°C for 5 min and then placed on ice, 8µl reverse transcription mix was added to a final content of 1x First Strand Buffer, 0.05M DTT, 500µM of each dNTP, 1mM RT-primer, 10U/µl Superscript III and 2U/µl RNaseOUT. Samples were incubated at 50°C for 1 hour before placed on ice and the samples were subsequently purified using Agencourt RNAClean XP beads according to the protocol previously described. A total reaction volume of 10µl containing 1xKAPA HiFi HotStart ReadyMix (#KK2601, KAPA Biosystems), 1x EVA green (#31000, Biotium), 0.5µM PCR InPE1.0 (Eurofins), 0.01µM PCR InPE2.0 (Eurofins), 0.5µM PCR Index (Eurofins) and 2µl purified cDNA were amplified by qPCR with the following protocol: 98°C for 3 minutes, followed by cycling at 980C for 20 seconds, 60°C for 30 seconds and 72°C for 30 seconds. After determining the amount of cycles needed based on the qPCR, the samples were amplified in a total reaction volume of 25µl. The libraries were purified and samples were eluted in 20µl elution buffer (#19086, Qiagen) and the average fragment length of finished libraries was determined by using the DNA 1000 Kit (#5067-1504, Agilent) with a 2100 Bioanalyzer according to the manufacturer's protocol. The concentration of the finished libraries was determined with Qubit dsDNA HS Assay Kit (#Q32854, Life Technologies) according to the manufacturer's protocol. Finished libraries were diluted to 4nM and sequenced on the Illumina NextSeq platform using paired-end sequencing according to the manufacturer's protocol. Typically, 31 or 51 bases were sequenced on read one, and 121 or 101 bases were sequenced on read two. The oligonucleotides used during library preparation were:
Ligation adapter:
   rApp]AGATCGGAAGAGCACACGTCTGAACTCCAGTCAC[ddC] (SEQ ID NO: 4)
Second reverse transcription primer:
   TGACTGGAGTTCAGACGTGTGCTCTTCCGA (SEQ ID NO: 5)
PCR primer InPE1.0: 
ATGATACGGCGACCACCGAGATCTACACTCTTTCCCTACACGACGCTC
TTCCGATCT (SEQ ID NO: 6)
PCR primer InPE2.0:
   GTGACTGGAGTTCAGACGTGTGCTCTTCCGATCT (SEQ ID NO: 7)
PCR Index primer:

AAGCAGAAGACGGCATACGAGATNNNNNNGTGACTGGAGTTC (SEQ ID
NO: 8)

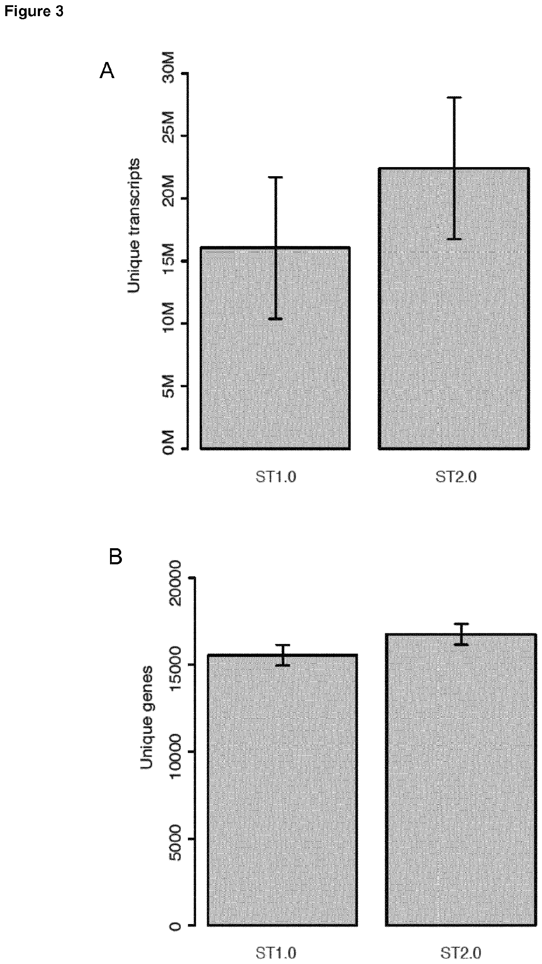

The results in Figure 3 surprisingly demonstrate that the simultaneous cleavage and extension of the capture probes (protocol E, i.e. ST2.0) resulted in an increase in the total number of transcripts (i.e. extended probes) produced (Figure 3A) and also an increase in the number of unique genes detected, i.e. the simultaneous cleavage and extension of the capture probes enhanced the diversity of the nucleic acids captured from the tissue sample.

### Example 3

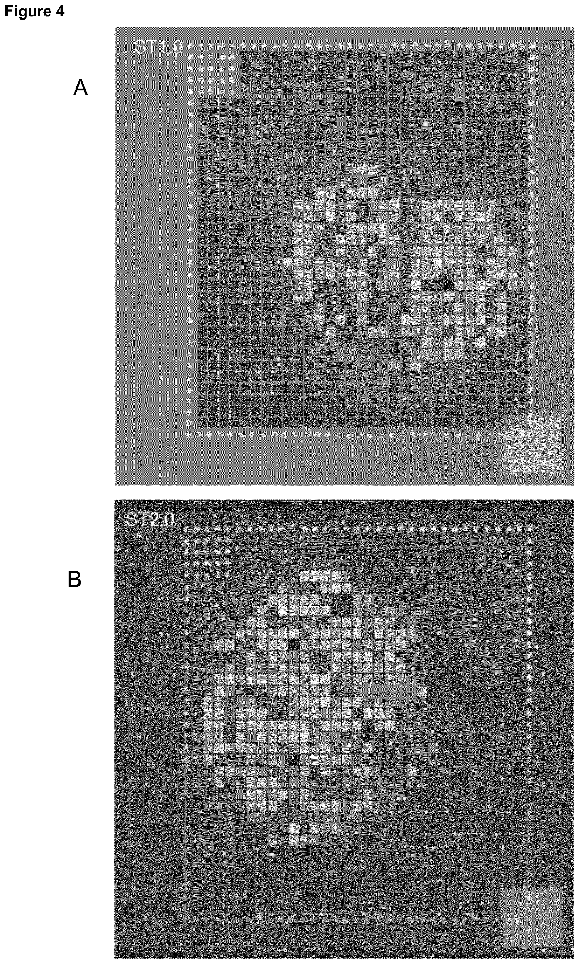

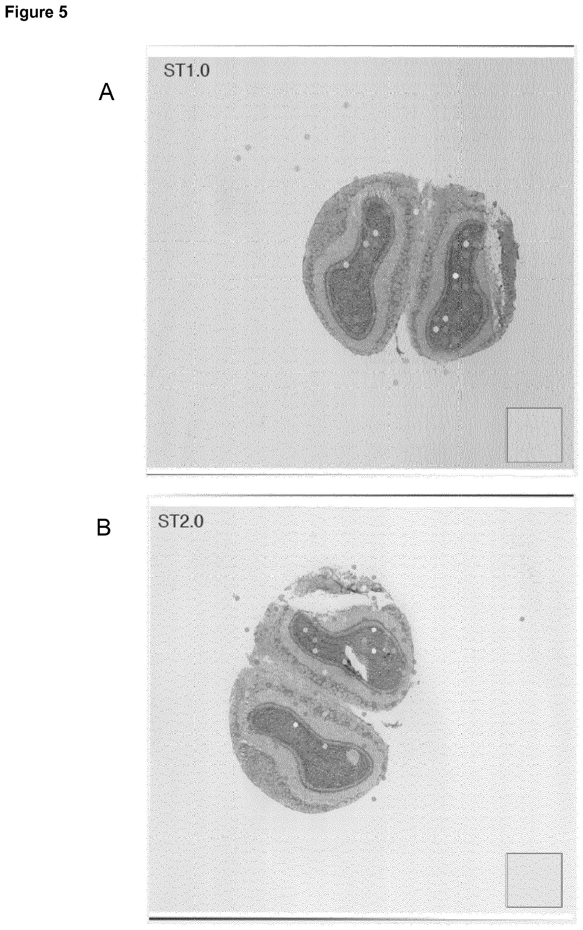

The standard ST1.0 protocol (i.e. separate extension and cleavage reactions) and ST2.0 protocol (i.e. the simultaneous cleavage and extension of the capture probes) were conducted on mouse brain tissue using an array with capture probes containing positional domains. Figure 4 shows heat maps of the sequencing data obtained for each feature on the array using ST1.0 (A) and ST2.0 (B). Figure 5 shows an overlay of the expression data for the proenkephalin (Penk) gene on an image of the mouse brain tissue obtained using ST1.0 (A) and ST2.0 (B).

It is evident from the data in Figure 4 that the majority of the extended probes correlate directly to the part of the array in contact with the tissue sample. Thus the results in Figure 4 indicate that the released primers in the ST2.0 protocol are able to meet with, and hybridise to, transcripts under the tissue. Furthermore, the data in Figure 4 suggests that an increased number of probes are extended using the ST2.0 protocol relative to the ST1.0 protocol.

Figure 5 demonstrates that unique transcripts from the Penk gene can be spatially detected using both the ST 1.0 and ST 2.0 protocols. Both protocols show that the Penk transcripts are located in the central pear shaped structure of the tissue, demonstrating that both protocols facilitate the spatial tagging of nucleic acid molecules from a biological specimen.

## Claims

1. A method for spatially tagging nucleic acids of a biological specimen comprising:
(a) providing a solid substrate on which multiple species of capture probes are immobilized such that each species occupies a distinct position on the solid substrate, wherein each capture probe of the multiple species is a primer for an extension reaction and wherein each capture probe of the multiple species is immobilized at its 3' end and comprises a nucleic acid molecule comprising in a 5' to 3' direction:
(i) a positional domain that corresponds to a position of the capture probe on the solid substrate;
(ii) a capture domain; and
(iii) a cleavage domain for releasing the capture probe from the solid substrate;
(b) contacting the solid substrate with the biological specimen; and
(c) simultaneously releasing the multiple species of capture probes from the solid substrate under conditions that allow a nucleic acid of the biological specimen to hybridise to a capture domain of the capture probe and extending the capture probe using the nucleic acid hybridised to the capture probe as an extension template to produce an extended capture probe, wherein step (c) comprises contacting the solid substrate with an aqueous reaction mixture comprising:
(i) a polymerase capable of extending the capture probe using the nucleic acid hybridised to the capture probe as an extension template to produce the extended capture probe; and
(ii) a means for releasing the multiple species of capture probes from the solid substrate.

2. The method of claim 1, wherein the means for releasing the multiple species of capture probes comprises a cleavage enzyme capable of specifically cleaving the multiple species of capture probes within the cleavage domain, optionally wherein the cleavage domain comprises a poly-U sequence and the cleavage enzyme comprises a mixture of Uracil DNA glycosylase (UDG) and an endonuclease enzyme capable of recognising apurinic/apyrimidinic (AP) sites of dsDNA, preferably a DNA glycosylase-lyase enzyme such as Endonuclease VIII, optionally were the cleavage domain comprises a restriction endonuclease recognition sequence and the cleavage enzyme is a restriction endonuclease.

3. The method of claim 1, wherein the cleavage domain is a cleavable chemical crosslinker.

4. The method of any one of claims 1-3, wherein the polymerase is a reverse transcriptase enzyme, optionally wherein the reverse transcriptase enzyme is selected from the list consisting of M-MLV, MuLV, AMV and HIV reverse transcriptase enzymes and a derivative or a mutant thereof, preferably wherein the derivative is a sequence-modified derivative, optionally wherein the reverse transcriptase enzyme comprises a polypeptide sequence as set forth in SEQ ID NO: 2 or a polypeptide sequence that has at least 80% sequence identity thereto, optionally wherein the reverse transcriptase enzyme is encoded by a nucleotide sequence comprising a sequence as set forth in SEQ ID NO: 1 or a nucleotide sequence that has at least 80% sequence identity thereto.

5. The method of any one of claims 1 to 4, wherein the method further comprises a step of synthesizing a complementary strand of the extended capture probe to produce a double-stranded extended capture probe, optionally wherein the method further comprises a step of amplifying the extended capture probe and/or the double-stranded extended capture probe.

6. The method of any one of claims 1 to 5, wherein the method further comprises a step of analysing the extended capture probe and/or the double-stranded extended capture probe and/or an amplicon thereof, wherein the step of analysing the extended capture probe and/or the double-stranded extended capture probe and/or the amplicon thereof comprises analysing the sequences of the extended capture probe and/or the double-stranded extended capture probe and/or the amplicon thereof.

7. The method of claim 6, wherein the method further comprises a step of correlating the analysis information, e.g. the sequence analysis information, with an image of the biological specimen.

8. The method of any one of claims 1 to 7, wherein the method further comprises a step of labelling the extended capture probe generated in step (c) and/or the complementary strand and/or the amplicon thereof, wherein the labelling step may be performed contemporaneously with, or subsequent to, the extending step.

9. The method of any one of claims 1 to 8, wherein the multiple species of capture probes further comprise an amplification domain.

10. The method of any one of claims 1 to 9, wherein the positional domain of the multiple species of capture probes comprises a barcode sequence.

11. The method of any one of claims 1 to 10, wherein the capture domain comprises a poly-T DNA oligonucleotide comprising at least 10 deoxythymidine residues and/or a random or degenerate oligonucleotide sequence, optionally wherein the capture domain comprises a sequence specific for a particular target gene or group of genes.

12. The method of any one of claims 1 to 11, wherein the solid substrate comprises an array, optionally, wherein the array is a bead array.

13. The method of any one of claims 1 to 12, wherein the multiple species of capture probes are immobilized on the solid substrate in a random arrangement, optionally wherein the method further comprises a step of performing a nucleic acid detection reaction on the solid substrate to determine the positional domain sequences of the multiple species of capture probes on the solid substrate.

14. The method of any one of claims 1 to 13, wherein the biological specimen is a tissue section, optionally wherein the tissue section is prepared using a fixed tissue, e.g., a formalin-fixed paraffin-embedded (FFPE) tissue, or deep frozen tissue.

15. The method of any one of claims 1 to 14, further comprising a step of permeabilizing and/or fixing the biological specimen after contacting the biological specimen with the solid substrate.

## Patentansprüche

1. Verfahren zur räumlichen Markierung von Nukleinsäuren einer biologischen Probe, umfassend:
(a) Bereitstellen eines festen Substrats, auf dem mehrere Arten von Einfangsonden derart immobilisiert sind, dass jede Art eine eindeutige Position auf dem festen Substrat besetzt, wobei jede Einfangsonde der mehreren Arten einen Primer für eine Verlängerungsreaktion darstellt und wobei jede Einfangsonde der mehreren Arten an ihrem 3'-Ende immobilisiert ist und ein Nukleinsäuremolekül umfasst, das in 5'-nach-3'-Richtung umfasst:
(i) eine Positionsdomäne, die einer Position der Einfangsonde auf dem festen Substrat entspricht;
(ii) eine Einfangdomäne; und
(iii) eine Spaltungsdomäne zum Freisetzen der Einfangsonde von dem festen Substrat, und
(b) In-Kontakt-Bringen des festen Substrats mit der biologischen Probe; und
(c) gleichzeitiges Freisetzen der mehreren Arten von Einfangsonden von dem festen Substrat unter Bedingungen, die erlauben, dass eine Nukleinsäure der biologischen Probe an eine Einfangdomäne der Einfangsonde hybridisiert, und Verlängern der Einfangsonde unter Verwendung der an die Einfangsonde hybridisierten Nukleinsäure als Verlängerungsmatrize, um eine verlängerte Einfangsonde zu erzeugen, wobei Schritt (c) das In-Kontakt-Bringen des festen Substrats mit einem wässrigen Reaktionsgemisch umfasst, das umfasst:
(i) eine Polymerase, die in der Lage ist, die Einfangsonde unter Verwendung der an die Einfangsonde hybridisierten Nukleinsäure als Verlängerungsmatrize zu verlängern, um die verlängerte Einfangsonde zu erzeugen; und
(ii) ein Mittel zum Freisetzen der mehreren Arten von Einfangsonden von dem festen Substrat.

2. Verfahren nach Anspruch 1, wobei das Mittel zum Freisetzen der mehreren Arten von Einfangsonden ein Spaltungsenzym umfasst, das in der Lage ist, die mehreren Arten von Einfangsonden innerhalb der Spaltungsdomäne spezifisch zu spalten, gegebenenfalls wobei die Spaltungsdomäne eine Poly-U-Sequenz umfasst und das Spaltungsenzym ein Gemisch aus Uracil-DNA-Glycosylase (UDG) und einem Endonuklease-Enzym, das in der Lage ist, Apurin-/Apyrimidin-(AP-)Stellen von dsDNA zu erkennen, vorzugsweise ein DNA-Glycosylaseenzym wie Endonuklease VIII, umfasst, gegebenenfalls wobei die Spaltungsdomäne eine Restriktionsendonuklease-Erkennungssequenz umfasst und es sich bei dem Spaltungsenzym um eine Restriktionsendonuklease handelt.

3. Verfahren nach Anspruch 1, wobei es sich bei der Spaltungsdomäne um einen spaltbaren chemischen Vernetzer handelt.

4. Verfahren nach einem der Ansprüche 1-3, wobei es sich bei der Polymerase um ein Reverse-Transkriptase-Enzym handelt, wahlweise wobei das Reverse-Transkriptase-Enzym aus der Liste bestehend aus M-MLV-, MuLV-, AMV- und HIV-Reverse-Transkriptase-Enzymen und einem Derivat oder einer Mutante davon ausgewählt ist, vorzugsweise wobei das Derivat ein sequenzmodifiziertes Derivat ist, gegebenenfalls wobei das Reverse-Transkriptase-Enzym eine wie in SEQ ID NO: 2 dargestellte Polypeptidsequenz oder eine Polypeptidsequenz, die wenigstens 80 % Sequenzidentität damit aufweist, umfasst, gegebenenfalls wobei das Reverse-Transkriptase-Enzym durch eine Nukleotidsequenz codiert ist, die eine wie in SEQ ID NO: 1 dargestellte Sequenz oder eine Nukleotidsequenz, die wenigstens 80 % Sequenzidentität damit aufweist, umfasst.

5. Verfahren nach einem der Ansprüche 1 bis 4, wobei das Verfahren ferner einen Schritt des Synthetisierens eines Komplementärstrangs der verlängerten Einfangsonde umfasst, um eine doppelsträngige verlängerte Einfangsonde zu erzeugen, gegebenenfalls wobei das Verfahren ferner einen Schritt des Amplifizierens der verlängerten Einfangsonde und/oder der doppelsträngigen verlängerten Einfangsonde umfasst.

6. Verfahren nach einem der Ansprüche 1 bis 5, wobei das Verfahren ferner einen Schritt des Analysierens der verlängerten Einfangsonde und/oder der doppelsträngigen verlängerten Einfangsonde und/oder eines Amplifikats davon umfasst, wobei der Schritt des Analysierens der verlängerten Einfangsonde und/oder der doppelsträngigen verlängerten Einfangsonde und/oder des Amplifikats davon das Analysieren der Sequenzen der verlängerten Einfangsonde und/oder der doppelsträngigen verlängerten Einfangsonde und/oder des Amplifikats davon umfasst.

7. Verfahren nach Anspruch 6, wobei das Verfahren ferner einen Schritt des Korrelierens der Analyseinformationen, z. B. der Sequenzanalyseinformationen, mit einem Bild der biologischen Probe umfasst.

8. Verfahren nach einem der Ansprüche 1 bis 7, wobei das Verfahren ferner einen Schritt des Markierens der in Schritt (c) erzeugten verlängerten Einfangsonde und/oder des Komplementärstrangs und/oder des Amplifikats davon umfasst, wobei der Markierungsschritt gleichzeitig mit dem oder im Anschluss an den Verlängerungsschritt erfolgen kann.

9. Verfahren nach einem der Ansprüche 1 bis 8, wobei die mehreren Arten von Einfangsonden ferner eine Amplifikationsdomäne umfassen.

10. Verfahren nach einem der Ansprüche 1 bis 9, wobei die Positionsdomäne der mehreren Arten von Einfangsonden eine Strichcodesequenz umfasst.

11. Verfahren nach einem der Ansprüche 1 bis 10, wobei die Einfangdomäne ein Poly-T-DNA-Oligonukleotid, das wenigstens 10 Desoxythymidinreste umfasst, und/oder eine zufällige oder degenerierte Oligonukleotidsequenz umfasst, gegebenenfalls wobei die Einfangdomäne eine für ein bestimmtes Zielgen bzw. eine bestimmte Gruppe von Genen spezifische Sequenz umfasst.

12. Verfahren nach einem der Ansprüche 1 bis 11, wobei das feste Substrat ein Array umfasst, gegebenenfalls, wobei es sich bei dem Array um ein Bead-Array handelt.

13. Verfahren nach einem der Ansprüche 1 bis 12, wobei die mehreren Arten von Einfangsonden an dem festen Substrat in einer Zufallsanordnung immobilisiert sind, gegebenenfalls wobei das Verfahren ferner einen Schritt der Durchführung einer Nukleinsäurenachweisreaktion an dem festen Substrat umfasst, um die Positionsdomänensequenzen der mehreren Arten von Einfangsonden an dem festen Substrat zu bestimmen.

14. Verfahren nach einem der Ansprüche 1 bis 13, wobei es sich bei der biologischen Probe um einen Gewebeschnitt handelt, gegebenenfalls wobei der Gewebeschnitt unter Verwendung eines fixierten Gewebes, z. B. eines formalinfixierten paraffineingebetteten (FFPE-) Gewebes, oder tiefgefrorenen Gewebes präpariert wird.

15. Verfahren nach einem der Ansprüche 1 bis 14, ferner umfassend einen Schritt des Permeabilisierens und/oder Fixierens der biologischen Probe nach In-Kontakt-Bringen der Probe mit dem festen Substrat.

## Revendications

1. Procédé pour l'étiquetage spatial d'acides nucléiques d'un échantillon biologique comprenant :
(a) la mise à disposition d'un substrat solide sur lequel plusieurs espèces de sondes de capture sont immobilisées de sorte que chaque espèce occupe une position distincte sur le substrat solide, chaque sonde de capture des plusieurs espèces étant une amorce pour une réaction d'extension et chaque sonde de capture des plusieurs espèces étant immobilisée au niveau de sa terminaison 3' et comprenant une molécule d'acide nucléique comprenant dans une direction de 5' à 3':
(i) un domaine positionnel qui correspond à une position de la sonde de capture sur le substrat solide ;
(ii) un domaine de capture ; et
(iii) un domaine de clivage pour la libération de la sonde de capture du substrat solide ;
(b) la mise en contact du substrat solide avec l'échantillon biologique ; et
(c) la libération simultanée des plusieurs espèces de sondes de capture du substrat solide dans des conditions qui permettent à un acide nucléique de l'échantillon biologique de s'hybrider à un domaine de capture de la sonde de capture et l'extension de la sonde de capture en utilisant l'acide nucléique hybridé à la sonde de capture comme une matrice d'extension pour produire une sonde de capture étendue, l'étape (c) comprenant la mise en contact du substrat solide avec un milieu de réaction aqueux comprenant :
(i) une polymérase capable d'étendre la sonde de capture en utilisant l'acide nucléique hybridé à la sonde de capture comme une matrice d'extension pour produire la sonde de capture étendue ; et
(ii) un moyen de libération des plusieurs espèces de sondes de capture depuis le substrat solide.

2. Procédé selon la revendication 1, le moyen de libération des plusieurs espèces de sondes de capture comprenant une enzyme de clivage capable de cliver spécifiquement les plusieurs espèces de sondes de capture dans le domaine de clivage, éventuellement, le domaine de clivage comprenant une séquence poly-U et l'enzyme de clivage comprenant un mélange d'uracile ADN glycosylase (UDG) et d'une enzyme de type endonucléase capable de reconnaître des sites apuriniques/apyrimidiniques (AP) d'ADNds, préférablement une enzyme de type ADN glycosylase-lyase telle que l'Endonucléase VIII, éventuellement le domaine de clivage comprenant une séquence de reconnaissance d'endonucléase de restriction et l'enzyme de clivage étant une endonucléase de restriction.

3. Procédé selon la revendication 1, le domaine de clivage étant un agent de réticulation chimique clivable.

4. Procédé selon l'une quelconque des revendications 1 à 3, la polymérase étant une enzyme de type transcriptase inverse, éventuellement, l'enzyme de type transcriptase inverse étant choisie dans la liste constituée par les enzymes de type transcriptase inverse de M-MLV, MuLV, AMV et HIV et un dérivé ou un mutant correspondant, de préférence le dérivé étant un dérivé à séquence modifiée, éventuellement l'enzyme de type transcriptase inverse comprenant une séquence polypeptidique telle que présentée dans la SEQ ID NO : 2 ou une séquence polypeptidique qui présente une identité de séquence d'au moins 80 % avec celle-ci, éventuellement l'enzyme transcriptase inverse étant codée par une séquence nucléotidique comprenant une séquence telle que présentée dans la SEQ ID NO : 1 ou une séquence nucléotidique qui présente une identité de séquence d'au moins 80 % avec celle-ci.

5. Procédé selon l'une quelconque des revendications 1 à 4, le procédé comprenant en outre une étape de synthèse d'un brin complémentaire de la sonde de capture étendue pour produire une sonde de capture étendue à double brin, éventuellement, le procédé comprenant en outre une étape d'amplification de la sonde de capture étendue et/ou de la sonde de capture étendue à double brin.

6. Procédé selon l'une quelconque des revendications 1 à 5, le procédé comprenant en outre une étape d'analyse de la sonde de capture étendue et/ou de la sonde de capture étendue à double brin et/ou d'un amplicon correspondant, l'étape d'analyse de la sonde de capture étendue et/ou de la sonde de capture étendue à double brin et/ou de l'amplicon correspondant comprenant l'analyse des séquences de la sonde de capture étendue et/ou de la sonde de capture étendue à double brin et/ou de l'amplicon correspondant.

7. Procédé selon la revendication 6, le procédé comprenant en outre une étape de corrélation des informations d'analyse, par ex. des informations d'analyse séquence, avec une image de l'échantillon biologique.

8. Procédé selon l'une quelconque des revendications 1 à 7, le procédé comprenant en outre une étape de marquage de la sonde de capture étendue générée dans l'étape (c) et/ou du brin complémentaire et/ou de l'amplicon correspondant, l'étape de marquage pouvant être réalisée de manière simultanée avec, ou subséquemment à, l'étape d'extension.

9. Procédé selon l'une quelconque des revendications 1 à 8, les plusieurs espèces de sondes de capture comprenant en outre un domaine d'amplification.

10. Procédé selon l'une quelconque des revendications 1 à 9, le domaine positionnel des plusieurs espèces de sondes de capture comprenant une séquence à code-barre.

11. Procédé selon l'une quelconque des revendications 1 à 10, le domaine de capture comprenant un oligonucléotide d'ADN poly-T comprenant au moins 10 résidus de désoxythymidine et/ou une séquence oligonucléotidique aléatoire ou dégénérée, éventuellement, le domaine de capture comprenant une séquence spécifique d'un gène ou d'un groupe de gènes cible particulier.

12. Procédé selon l'une quelconque des revendications 1 à 11, le substrat solide comprenant un réseau, éventuellement, le réseau étant un réseau de billes.

13. Procédé selon l'une quelconque des revendications 1 à 12, les plusieurs espèces de sondes de capture étant immobilisées sur le substrat solide en un agencement aléatoire, éventuellement, le procédé comprenant en outre une étape de réalisation d'une réaction de détection d'acide nucléique sur le substrat solide pour déterminer les séquences de domaine positionnel des plusieurs espèces de sondes de capture sur le substrat solide.

14. Procédé selon l'une quelconque des revendications 1 à 13, l'échantillon biologique étant une section de tissu, éventuellement la section de tissu étant préparée en utilisant un tissu fixé, par ex., un tissu incorporé dans de la paraffine fixé au formol (FFPE), ou un tissu congelé.

15. Procédé selon l'une quelconque des revendications 1 à 14, comprenant en outre une étape de perméabilisation et/ou de fixation de l'échantillon biologique après mise en contact de l'échantillon biologique avec le substrat solide.
